# EUROPEAN PATENT APPLICATION

(11) **EP 4 825 976 A2**
(43) Date of publication of application: **21.10.2026**
(21) Application number: 26188842.4
(22) Date of filing: 09.11.2017
(51) Int. Cl.: C12P 21/02

(54) **ACTIVIN RECEPTOR TYPE IIA VARIANTS AND METHODS OF USE THEREOF**

(30) Priority: 10.11.2016 US 201662420476 P; 13.07.2017 US 201762531943 P
(62) Document number(s) of the earlier application(s) in accordance with Art. 76 EPC: 17870560.4 / 3 538 128
(71) Applicant: Keros Therapeutics, Inc., Lexington, MA 02421 (US)
(72) Inventor: SEEHRA, Jasbir S., Lexington, MA 02421 (US); LACHEY, Jennifer, Lincoln, MA 01773 (US)
(74) Representative: Carpmaels & Ransford LLP

(57) **Abstract**

The invention features polypeptides that include an extracellular ActRlla variant. In some embodiments, a polypeptide of the invention includes an extracellular ActRlla variant fused to an Fc domain monomer or moiety. The invention also features pharmaceutical compositions and methods of using the polypeptides to treat diseases and conditions involving weakness and atrophy of muscles, e.g., Duchenne muscular dystrophy, facioscapulohumeral muscular dystrophy, inclusion body myositis, amyotrophic lateral sclerosis, sarcopenia; or cancer cachexia; or metabolic diseases, e.g., obesity, Type-1 diabetes, or Type-2 diabetes.

## Description

### BACKGROUND OF THE INVENTION

Duchenne muscular dystrophy (DMD), facioscapulohumeral muscular dystrophy (FSHD), inclusion body myositis (IBM), and amyotrophic lateral sclerosis (ALS) are examples of muscle diseases that involve weakness and atrophy of muscles and/or motor neurons that control voluntary muscle movements. DMD is caused by mutations in the X-linked *dystrophin* gene and characterized by progressive muscle degeneration and weakness in all skeletal muscles. FSHD particularly affects skeletal muscles of the face, shoulders, upper arms, and lower legs. IBM is an inflammatory muscle disease that mainly affects muscles of the thighs and muscles of the arms that control finger and wrist flexion. ALS is a motor neuron disease characterized by stiff muscles, muscle twitching, and muscle atrophy throughout the body due to the degeneration of the motor neurons. Efforts to improve treatment and survival of subjects having these devastating muscle diseases have not been successful.

Excess body weight is an increasing problem in the United States, with a prevalence of approximately 25% of the population. Increased visceral and subcutaneous fact causes dysfunction of various organs. Excessive body weight is a risk factor for an array of complications, including obesity, diabetes (e.g., Type-1 and Type-2 diabetes), cardiovascular disease, and several forms of cancer. Insulin resistance is also associated with obesity and occurs when pancreatic tissues require an elevated amount of insulin. Once pancreatic β cells can no longer produce sufficient insulin to meet the demand, hyperglycemia occurs and Type-2 diabetes develops. Adipocytes, which are increased in obesity, are believed to play a role in this process. Despite the prevalence of obesity and metabolic diseases such as diabetes (e.g., Type-1 and Type-2 diabetes) and insulin resistance, few therapeutic options are available.

There exists a need for novel treatments for these muscular and metabolic diseases.

### SUMMARY OF THE INVENTION

The present invention features polypeptides that include an extracellular activin receptor type Ila (ActRlla) variant. In some embodiments, a polypeptide of the invention includes an extracellular ActRlla variant fused to the N- or C-terminus of an Fc domain monomer or moiety. Such moieties may be attached by amino acid or other covalent bonds and may increase stability of the polypeptide. A polypeptide including an extracellular ActRlla variant fused to an Fc domain monomer may also form a dimer (e.g., a homodimer or heterodimer) through the interaction between two Fc domain monomers. The polypeptides of the invention may be used to increase muscle mass and strength in a subject having a disease or condition involving weakness and atrophy of muscles, e.g., Duchenne muscular dystrophy (DMD), facioscapulohumeral muscular dystrophy (FSHD), inclusion body myositis (IBM), amyotrophic lateral sclerosis (ALS), sarcopenia, or cancer cachexia. The polypeptides of the invention may also be used to reduce body weight, reduce body fat, increase glucose clearance, increase insulin sensitivity, or reduce fasting insulin levels in a subject having or at risk of developing a metabolic disease, e.g., obesity, Type-1 diabetes, or Type-2 diabetes. Further, the polypeptides of the invention may also be used to affect myostatin, activin, and/or bone morphogenetic protein 9 (BMP9) signaling in a subject having a risk of developing or having a disease or condition involving weakness and atrophy of muscles or a metabolic disease.

In one aspect, the invention features a polypeptide including an extracellular activin receptor type IIa (ActRlla) variant, the variant having a sequence of GAILGRSETQECLX₁X₂NANWX₃X₄X₅X₆ TNQTGVEX₇CX₈GX₉X₁₀X₁₁X₁₂X₁₃X₁₄HCX₁₅ATWX₁₆NISGSIEIV X₁₇X₁₈GCX₁₉X₂₀X₂₁DX₂₂NCYDRTDCVEX₂₃X₂₄X₂₅X₂₆PX₂₇VYFCCCEGNMCNEKFSYFPEMEVTQPTS (SEQ ID NO: 1), wherein X₁ is F or Y; X₂ is F or Y; X₃ is E or A; X₄ is K or L; X₅ is D or E; X₆ is R or A; X₇ is P or R; X₈ is Y or E; X₉ is D or E; X₁₀ is K or Q; X₁₁ is D or A; X₁₂ is K or A; X₁₃ is R or A; X₁₄ is R or L; X₁₅ is F or Y; X₁₆ is K, R, or A; X₁₇ is K, A, Y, F, or I; X₁₈ is Q or K; X₁₉ is W or A; X₂₀ is L or A; X₂₁ is D, K, R, A, F, G, M, N, or I; X₂₂ is I, F, or A; X₂₃ is K or T; X₂₄ is K or E; X₂₅ is D or E; X₂₆ is S or N; and X₂₇ is E or Q, and wherein the variant has at least one amino acid substitution relative to a wild-type extracellular ActRlla having the sequence of SEQ ID NO: 73 or an extracellular ActRlla having any one of the sequences of SEQ ID NOs: 76-96.

In some embodiments, the variant has a sequence of GAILGRSETQECLFX₂NANWX₃X₄X₅X₆TNQTGVEX₇CX₈GX₉KX₁₁X₁₂X₁₃X₁₄HCX₁₅ATWX₁₆NISGSIEIVX₁₇ X₁₈GCX₁₉X₂₀X₂₁DX₂₂NCYDRTDCVEX₂₃X₂₄X₂₅X₂₆PX₂₇VYFCCCEGNMCNEKFSYFPEMEVTQPTS (SEQ ID NO: 2), wherein X₂, X₃, X₄, X₅, X₆, X₇, X₈, X₉, X₁₁, X₁₂, X₁₃, X₁₄, X₁₅, X₁₆, X₁₇, X₁₈, X₁₉, X₂₀, X₂₁, X₂₂, X₂₃, X₂₄, X₂₅, X₂₆, and X₂₇ are defined as above.

In some embodiments, the variant has a sequence of 
GAILGRSETQECLFX2NANWEX4X5RTNQTGVEX7CX8GX9KDKRX14HCX15ATWX16NISGSIEIVKX18GCWL
DDX22NCYDRTDCVEX23X24X25X26PX27VYFCCCEGNMCNEKFSYFPEMEVTQPTS (SEQ ID NO: 3),
 wherein X₂, X₄, X₅, X₇, X₈, X₉, X₁₄, X₁₅, X₁₆, X₁₈, X₂₂, X₂₃, X₂₄, X₂₅, X₂₆, and X₂₇ are defined as above.

In some embodiments, the variant has a sequence of 
GAILGRSETQECLFX2NANWEX4DRTNQTGVEX7CX8GX9KDKRX14HCX15ATWX16NISGSIEIVKX18GCWL
DDX22NCYDRTDCVEX23KX25X26PX27VYFCCCEGNMCNEKFSYFPEMEVTQPTS (SEQ ID NO: 4),
 wherein X₂, X₄, X₇, X₈, X₉, X₁₄, X₁₅, X¹⁶, X₁₈, X₂₂, X₂₃, X₂₅, X₂₆, and X₂₇ are defined as above.

In some embodiments, the variant has a sequence of GAILGRSETQECLFX₂NANWEX₄DRTNQTGVEPCX₈GX₉KDKRX₁₄HCFATWKNISGSIEIVKX₁₈GCWLDDI NCYDRTDCVEX₂₃KX₂₅X₂₆PX₂₇VYFCCCEGNMCNEKFSYFPEMEVTQPTS (SEQ ID NO: 5), wherein X₂, X₄, X₈, X₉, X₁₄, X₁₈, X₂₃, X₂₅, X₂₆, and X₂₇ are defined as above.

In any of the aforementioned embodiments, X₁ is F or Y. In any of the aforementioned embodiments, X₂ is F or Y. In any of the aforementioned embodiments, X₃ is E or A. In any of the aforementioned embodiments, X₄ is K or L. In any of the aforementioned embodiments, X₅ is D or E. In any of the aforementioned embodiments, X₆ is R or A. In any of the aforementioned embodiments, X₇ is P or R. In any of the aforementioned embodiments, X₈ is Y or E. In any of the aforementioned embodiments, X₉ is D or E. In any of the aforementioned embodiments, X₁₀ is K or Q. In any of the aforementioned embodiments, X₁₁ is D or A. In any of the aforementioned embodiments, X₁₂ is K or A. In any of the aforementioned embodiments, X₁₃ is R or A. In any of the aforementioned embodiments, X₁₄ is R or L. In any of the aforementioned embodiments, X₁₅ is F or Y. In any of the aforementioned embodiments, X₁₆ is K, R, or A. In any of the aforementioned embodiments, X₁₇ is K, A, Y, F, or I. In any of the aforementioned embodiments, X₁₈ is Q or K. In any of the aforementioned embodiments, X₁₉ is W or A. In any of the aforementioned embodiments, X₂₀ is L or A. In any of the aforementioned embodiments, X₂₁ is D, K, R, A, F, G, M, N, or I. In any of the aforementioned embodiments, X₂₂ is I, F, or A. In any of the aforementioned embodiments, X₂₃ is K or T. In any of the aforementioned embodiments, X₂₄ is K or E. In any of the aforementioned embodiments, X₂₅ is D or E. In any of the aforementioned embodiments, X₂₆ is S or N. In any of the aforementioned embodiments, X₂₇ is E or Q. In any of the aforementioned embodiments, X₂₃ is T, X₂₄ is E, X₂₅ is E, and X₂₆ is N. In any of the aforementioned embodiments, X₂₃ is T, X₂₄ is K, X₂₅ is E, and X₂₆ is N. In any of the aforementioned embodiments, X₁₇ is K.

In any of the aforementioned embodiments, the variant has the sequence of any one of SEQ ID NOs: 6-72.

In any of the aforementioned embodiments, the amino acid at position X₂₄ may be replaced with the amino acid K.

In any of the aforementioned embodiments, the amino acid at position X₂₄ may be replaced with the amino acid E.

In any of the aforementioned embodiments, a polypeptide described herein may further include a C-terminal extension of one or more amino acids (e.g., 1, 2, 3, 4, 5, 6, or more amino acids). In some embodiments, the C-terminal extension is amino acid sequence NP. In some embodiments, the C-terminal extension is amino acid sequence NPVTPK (SEQ ID NO: 155).

In any of the aforementioned embodiments, a polypeptide described herein may further include a moiety fused or covalently linked to the C-terminus of the polypeptide. In some embodiments, the moiety increases stability or improves the pharmacokinetics of the polypeptide. In some embodiments, the moiety is an Fc domain, an albumin-binding peptide, a fibronectin domain, or human serum albumin.

In any of the aforementioned embodiments, a polypeptide described herein may further include an Fc domain monomer fused to the C-terminus of the polypeptide by way of a linker. In some embodiments, the polypeptide that includes an extracellular ActRlla variant described herein fused to an Fc domain monomer may form a dimer (e.g., a homodimer or heterodimer) through the interaction between two Fc domain monomers. In some embodiments, the Fc domain monomer has the sequence of SEQ ID NO: 97

In any of the aforementioned embodiments, a polypeptide described herein may further include an Fc domain fused to the C-terminus of the polypeptide by way of a linker. In some embodiments, the Fc domain is a wild-type Fc domain. In some embodiments, the wild-type Fc domain has the sequence of SEQ ID NO: 151. In some embodiments, the Fc domain contains one or more amino acid substitutions. In some embodiments, the Fc domain containing one or more amino acid substitutions does not form a dimer.

In any of the aforementioned embodiments, a polypeptide described herein may further include an albumin-binding peptide fused to the C-terminus of the polypeptide by way of a linker. In some embodiments, the albumin-binding peptide has the sequence of SEQ ID NO: 152.

In any of the aforementioned embodiments, a polypeptide described herein may further include a fibronectin domain fused to the C-terminus of the polypeptide by way of a linker. In some embodiments, the fibronectin domain peptide has the sequence of SEQ ID NO: 153.

In any of the aforementioned embodiments, a polypeptide described herein may further include a human serum albumin fused to the C-terminus of the polypeptide by way of a linker. In some embodiments, the human serum albumin has the sequence of SEQ ID NO: 154.

In some embodiments, the linker is an amino acid spacer. In some embodiments, the amino acid spacer is GGG, GGGA (SEQ ID NO: 98), GGGG (SEQ ID NO: 100), GGGAG (SEQ ID NO: 130), GGGAGG (SEQ ID NO: 131), or GGGAGGG (SEQ ID NO: 132).

In some embodiments, the amino acid spacer is GGGS (SEQ ID NO: 99), GGGGA (SEQ ID NO: 101), GGGGS (SEQ ID NO: 102), GGGGG (SEQ ID NO: 103), GGAG (SEQ ID NO: 104), GGSG (SEQ ID NO: 105), AGGG (SEQ ID NO: 106), SGGG (SEQ ID NO: 107), GAGA (SEQ ID NO: 108), GSGS (SEQ ID NO: 109), GAGAGA (SEQ ID NO: 110), GSGSGS (SEQ ID NO: 111), GAGAGAGA (SEQ ID NO: 112), GSGSGSGS (SEQ ID NO: 113), GAGAGAGAGA (SEQ ID NO: 114), GSGSGSGSGS (SEQ ID NO: 115), GAGAGAGAGAGA (SEQ ID NO: 116), and GSGSGSGSGSGS (SEQ ID NO: 117), GGAGGA (SEQ ID NO: 118), GGSGGS (SEQ ID NO: 119), GGAGGAGGA (SEQ ID NO: 120), GGSGGSGGS (SEQ ID NO: 121), GGAGGAGGAGGA (SEQ ID NO: 122), GGSGGSGGSGGS (SEQ ID NO: 123), GGAGGGAG (SEQ ID NO: 124), GGSGGGSG (SEQ ID NO: 125), GGAGGGAGGGAG (SEQ ID NO: 126), and GGSGGGSGGGSG (SEQ ID NO: 127), GGGGAGGGGAGGGGA (SEQ ID NO: 128), GGGGSGGGGSGGGGS (SEQ ID NO: 129), AAAL (SEQ ID NO: 133), AAAK (SEQ ID NO: 134), AAAR (SEQ ID NO: 135), EGKSSGSGSESKST (SEQ ID NO: 136), GSAGSAAGSGEF (SEQ ID NO: 137), AEAAAKEAAAKA (SEQ ID NO: 138), KESGSVSSEQLAQFRSLD (SEQ ID NO: 139), GENLYFQSGG (SEQ ID NO: 140), SACYCELS (SEQ ID NO: 141), RSIAT (SEQ ID NO: 142), RPACKIPNDLKQKVMNH (SEQ ID NO: 143), GGSAGGSGSGSSGGSSGASGTGTAGGTGSGSGTGSG (SEQ ID NO: 144), AAANSSIDLISVPVDSR (SEQ ID NO: 145), GGSGGGSEGGGSEGGGSEGGGSEGGGSEGGGSGGGS (SEQ ID NO: 146), EAAAK (SEQ ID NO: 147), or PAPAP(SEQ ID NO: 148).

In any of the aforementioned embodiments, the polypeptide described herein has a serum half-life of at least 7 days.

In any of the aforementioned embodiments, the polypeptide described herein binds to human bone morphogenetic protein 9 (BMP9) with a K_{D} of 200 pM or higher. In some embodiments, the polypeptide binds to activin and/or myostatin and has reduced (e.g., weak) binding to human BMP9. In some embodiments, the polypeptide does not substantially bind to human BMP9.

In any of the aforementioned embodiments, the polypeptide described herein binds to human activin A with a K_{D} of 800 pM or less.

In any of the aforementioned embodiments, the polypeptide described herein binds to human activin B with a K_{D} of approximately 800 pM or less.

In any of the aforementioned embodiments, the polypeptide described herein binds to human GDF-11 with a K_{D} of approximately 5 pM or higher.

In another aspect, the invention features a nucleic acid molecule encoding a polypeptide described herein (e.g., a polypeptide including an extracellular ActRlla variant having a sequence of any one of SEQ ID NOs: 1-72 (e.g., SEQ ID NOs: 6-72)). In another aspect, the invention also features a vector including the nucleic acid molecule described herein.

In another aspect, the invention features a host cell that expresses a polypeptide described herein, wherein the host cell includes a nucleic acid molecule or a vector described in the previous two aspects, wherein the nucleic acid molecule or vector is expressed in the host cell.

In another aspect, the invention features a method of preparing a polypeptide described herein, wherein the method includes: a) providing a host cell including a nucleic acid molecule or a vector described herein, and b) expressing the nucleic acid molecule or vector in the host cell under conditions that allow for the formation of the polypeptide.

In another aspect, the invention features a pharmaceutical composition including a polypeptide, nucleic acid molecule, or vector described herein and one or more pharmaceutically acceptable carriers or excipients. In some embodiments of the pharmaceutical composition, the polypeptide, nucleic acid molecule, or vector is in a therapeutically effective amount.

In another aspect, the invention also features a construct including two identical polypeptides (e.g., a homodimer) each including an extracellular ActRlla variant having a sequence of any one of SEQ ID NOs: 1-72 (e.g., SEQ ID NOs: 6-72)) fused to the N- or C-terminus of an Fc domain monomer (e.g., the sequence of SEQ ID NO: 97). The two Fc domain monomers in the two polypeptides interact to form an Fc domain in the construct.

In another aspect, the invention also features a construct including two different polypeptides (e.g., a heterodimer) each including an extracellular ActRlla variant having a sequence of any one of SEQ ID NOs: 1-72 (e.g., SEQ ID NOs: 6-72)) fused to the N- or C-terminus of an Fc domain monomer (e.g., the sequence of SEQ ID NO: 97). The two Fc domain monomers in the two polypeptides interact to form an Fc domain in the construct.

In another aspect, the invention features a method of increasing muscle mass in a subject in need thereof. The method includes administering to the subject a therapeutically effective amount of a polypeptide, nucleic acid molecule, or vector described herein or a pharmaceutical composition described herein.

In some embodiments of the method of increasing muscle mass in a subject, the subject has Duchenne muscular dystrophy (DMD), facioscapulohumeral muscular dystrophy (FSHD), inclusion body myositis (IBM), amyotrophic lateral sclerosis (ALS), sarcopenia, or cancer cachexia.

In another aspect, the invention features a method of affecting myostatin, activin, and/or BMP9 signaling (e.g., reducing or inhibiting the binding of myostatin, activin, and/or BMP9 to their receptors) in a subject having a disease or condition involving weakness and atrophy of muscles, wherein method includes administering to the subject a therapeutically effective amount of a polypeptide, nucleic acid molecule, or vector described herein or a pharmaceutical composition described herein. In some embodiments of this aspect, the disease or condition is DMD, FSHD, IBM, ALS, sarcopenia, or cancer cachexia.

In another aspect, the invention features a method of treating a subject having DMD by administering to the subject a therapeutically effective amount of a polypeptide, nucleic acid molecule, or vector described herein or a pharmaceutical composition described herein.

In another aspect, the invention features a method of treating a subject having FSHD by administering to the subject a therapeutically effective amount of a polypeptide, nucleic acid molecule, or vector described herein or a pharmaceutical composition described herein.

In another aspect, the invention features a method of treating a subject having IBM by administering to the subject a therapeutically effective amount of a polypeptide, nucleic acid molecule, or vector described herein or a pharmaceutical composition described herein.

In another aspect, the invention features a method of treating a subject having ALS by administering to the subject a therapeutically effective amount of a polypeptide, nucleic acid molecule, or vector described herein or a pharmaceutical composition described herein.

In another aspect, the invention features a method of reducing body fat in a subject in need thereof by administering to the subject a therapeutically effective amount of a polypeptide, a nucleic acid molecule, or vector described herein or a pharmaceutical composition described herein.

In another aspect, the invention features a method of reducing body weight in a subject in need thereof by administering to the subject a therapeutically effective amount of a polypeptide, a nucleic acid molecule, or vector described herein or a pharmaceutical composition described herein.

In another aspect, the invention features a method of reducing blood glucose in a subject in need thereof by administering to the subject a therapeutically effective amount of a polypeptide, a nucleic acid molecule, or vector described herein or a pharmaceutical composition described herein.

In another aspect, the invention features a method of increasing insulin sensitivity in a subject in need thereof, by administering to the subject a therapeutically effective amount of a polypeptide, a nucleic acid molecule, or vector described herein or a pharmaceutical composition described herein.

In some embodiments of any of the above aspects, the subject has or is at risk of developing a metabolic disease. In some embodiments, the metabolic disease is selected from the group including obesity, Type-1 diabetes, and Type-2 diabetes.

In another aspect, the invention features a method of affecting myostatin, activin, and/or BMP9 signaling (e.g., reducing or inhibiting the binding of myostatin, activin, and/or BMP9 to their receptors) in a subject having or at risk of developing a metabolic disease by administering to the subject a therapeutically effective amount of a polypeptide, a nucleic acid molecule, or vector described herein or a pharmaceutical composition described herein.

In another aspect, the invention features a method of treating and/or preventing a metabolic disease in a subject by administering to the subject a therapeutically effective amount of a polypeptide, a nucleic acid molecule, or vector described herein or a pharmaceutical composition described herein.

In some embodiments of any of the above aspect, the metabolic disease is selected from the group including obesity, Type-1 diabetes, and Type-2 diabetes. In some embodiments of any of the above aspects, the metabolic disease is obesity. In some embodiments of any of the above aspects, the metabolic disease is Type-1 diabetes. In some embodiments of any of the above aspects, the metabolic disease is Type-2 diabetes.

In some embodiments of any of the above aspects, the method reduces body weight and/or percentage of body weight gain of said subject. In some embodiments of any of the above aspects, the method reduces amount of body fat and/or percentage of body fat of said subject. In some embodiments of any of the above aspects, the method does not affect the appetite for food intake of said subject. In some embodiments of any of the above aspects, the method reduces adiposity of said subject. In some embodiments of any of the above aspects, the method reduces the weights of epididymal and perirenal fat pads of said subject. In some embodiments of any of the above aspects, the method reduces the amount of subcutaneous and/or visceral fat of said subject. In some embodiments of any of the above aspects, the method lowers the level of fasting insulin of said subject. In some embodiments of any of the above aspects, the method lowers the level of blood glucose of said subject. In some embodiments of any of the above aspects, the method increases insulin sensitivity of said subject. In some embodiments of any of the above aspects, the method increases the rate of glucose clearance of said subject. In some embodiments of any of the above aspects, the method improves the serum lipid profile of said subject. In some embodiments of any of the above aspects, the method does not reduce lean mass.

In some embodiments of any of the above aspects, the method increases muscle mass.

In some embodiments of any of the above aspects, the method reduces or inhibits the binding of activin and/or myostatin to their receptors.

In some embodiments of any of the above aspects, the polypeptide, nucleic acid, vector, or pharmaceutical composition is administered in an amount sufficient to increase muscle mass and/or strength, affect myostatin, activin, and/or BMP9 signaling in the subject, or reduce or inhibit the binding of activin and/or myostatin to their receptors,

In some embodiments of any of the above aspects, the polypeptide, nucleic acid, vector, or pharmaceutical composition is administered in an amount sufficient to reduce body fat, reduce the amount of subcutaneous fat, reduce the amount of visceral fat, reduce adiposity, reduce the weights of epididymal and perirenal fat pads, reduce body fat percentage, reduce body weight, reduce the percentage of body weight gain, reduce fasting insulin level, reduce blood glucose level, increase insulin sensitivity, affect myostatin, activin, and/or BMP9 signaling in the subject, reduce the proliferation of adipose cells, reduce or inhibit the binding of activin and/or myostatin to their receptors, reduce LDL, reduce triglycerides, improve the serum lipid profile, regulate insulin biosynthesis and/or secretion from β-cells, delay, postpone, or reduce the need for insulin, or increase glucose clearance.

In some embodiments of any of the methods described herein, the method does not cause a vascular complication (e.g., an increase vascular permeability or leakage) in the subject. In some embodiments of any of the methods described herein, the method increases bone mineral density in the subject.

In some embodiments of any of the above aspects, the variant has the sequence of SEQ ID NO: 69. In some embodiments, the variant having the sequence of SEQ ID NO: 69 has the amino acid K at position X₁₇, the amino acid sequence TEEN or TKEN at positions X₂₃, X₂₄, X₂₅, and X₂₆, and/or a C-terminal extension (e.g., 1, 2, 3, 4, 5, 6 or more additional amino acids at the C-terminus, e.g., the amino acids NP or NPVTPK (SEQ ID NO: 155)). In some embodiments of any of the above aspects, the method includes increasing muscle mass or treating a muscle disorder in a subject in need thereof (e.g., a subject having DMD, FSHD, IBM, ALS, sarcopenia, or cancer cachexia), affecting myostatin, activin, and/or BMP9 signaling in a subject (e.g., a subject having or at risk of developing DMD, FSHD, IBM, ALS, sarcopenia, cancer cachexia, obesity, Type-1 diabetes, or Type-2 diabetes), reducing body fat or body weight in a subject (e.g., a subject having obesity, Type-1 diabetes, or Type-2 diabetes), or treating and/or preventing a metabolic disease in a subject (e.g., a subject having or at risk of developing obesity, Type-1 diabetes, or Type-2 diabetes) by administering to the subject a therapeutically effective amount of a variant having the sequence of SEQ ID NO: 69, optionally having the amino acid K at position X₁₇, the amino acid sequence TEEN or TKEN at positions X₂₃, X₂₄, X₂₅, and X₂₆, and/or a C-terminal extension, or a pharmaceutical composition containing said variant.

In some embodiments of any of the above aspects, the variant has the sequence of SEQ ID NO: 58. In some embodiments, the variant having the sequence of SEQ ID NO: 58 has the amino acid K at position X₁₇, the amino acid sequence TEEN or TKEN at positions X₂₃, X₂₄, X₂₅, and X₂₆, and/or a C-terminal extension (e.g., 1, 2, 3, 4, 5, 6 or more additional amino acids at the C-terminus, e.g., the amino acids NP or NPVTPK (SEQ ID NO: 155)). In some embodiments of any of the above aspects, the method includes increasing muscle mass or treating a muscle disorder in a subject in need thereof (e.g., a subject having DMD, FSHD, IBM, ALS, sarcopenia, or cancer cachexia), affecting myostatin, activin, and/or BMP9 signaling in a subject (e.g., a subject having or at risk of developing DMD, FSHD, IBM, ALS, sarcopenia, cancer cachexia, obesity, Type-1 diabetes, or Type-2 diabetes), reducing body fat or body weight in a subject (e.g., a subject having obesity, Type-1 diabetes, or Type-2 diabetes), or treating and/or preventing a metabolic disease in a subject (e.g., a subject having or at risk of developing obesity, Type-1 diabetes, or Type-2 diabetes) by administering to the subject a therapeutically effective amount of a variant having the sequence of SEQ ID NO: 58, optionally having the amino acid K at position X₁₇, the amino acid sequence TEEN or TKEN at positions X₂₃, X₂₄, X₂₅, and X₂₆, and/or a C-terminal extension, or a pharmaceutical composition containing said variant.

In some embodiments of any of the above aspects, the variant has the sequence of SEQ ID NO: 6. In some embodiments, the variant having the sequence of SEQ ID NO: 6 has the amino acid K at position X₁₇, the amino acid sequence TEEN or TKEN at positions X₂₃, X₂₄, X₂₅, and X₂₆, and/or a C-terminal extension (e.g., 1, 2, 3, 4, 5, 6,or more additional amino acids at the C-terminus, e.g., the amino acids NP or NPVTPK (SEQ ID NO: 155)). In some embodiments of any of the above aspects, the method includes increasing muscle mass or treating a muscle disorder in a subject in need thereof (e.g., a subject having DMD, FSHD, IBM, ALS, sarcopenia, or cancer cachexia), affecting myostatin, activin, and/or BMP9 signaling in a subject (e.g., a subject having or at risk of developing DMD, FSHD, IBM, ALS, sarcopenia, cancer cachexia, obesity, Type-1 diabetes, or Type-2 diabetes), reducing body fat or body weight in a subject (e.g., a subject having obesity, Type-1 diabetes, or Type-2 diabetes), or treating and/or preventing a metabolic disease in a subject (e.g., a subject having or at risk of developing obesity, Type-1 diabetes, or Type-2 diabetes) by administering to the subject a therapeutically effective amount of a variant having the sequence of SEQ ID NO: 6, optionally having the amino acid K at position X₁₇, the amino acid sequence TEEN or TKEN at positions X₂₃, X₂₄, X₂₅, and X₂₆, and/or a C-terminal extension, or a pharmaceutical composition containing said variant.

In some embodiments of any of the above aspects, the variant has the sequence of SEQ ID NO: 38. In some embodiments, the variant having the sequence of SEQ ID NO: 38 has the amino acid K at position X₁₇, the amino acid sequence TEEN or TKEN at positions X₂₃, X₂₄, X₂₅, and X₂₆, and/or a C-terminal extension (e.g., 1, 2, 3, 4, 5, 6, or more additional amino acids at the C-terminus, e.g., the amino acids NP or NPVTPK (SEQ ID NO: 155)). In some embodiments of any of the above aspects, the method includes increasing muscle mass or treating a muscle disorder in a subject in need thereof (e.g., a subject having DMD, FSHD, IBM, ALS, sarcopenia, or cancer cachexia), affecting myostatin, activin, and/or BMP9 signaling in a subject (e.g., a subject having or at risk of developing DMD, FSHD, IBM, ALS, sarcopenia, cancer cachexia, obesity, Type-1 diabetes, or Type-2 diabetes), reducing body fat or body weight in a subject (e.g., a subject having obesity, Type-1 diabetes, or Type-2 diabetes), or treating and/or preventing a metabolic disease in a subject (e.g., a subject having or at risk of developing obesity, Type-1 diabetes, or Type-2 diabetes) by administering to the subject a therapeutically effective amount of a variant having the sequence of SEQ ID NO: 38, optionally having the amino acid K at position X₁₇, the amino acid sequence TEEN or TKEN at positions X₂₃, X₂₄, X₂₅, and X₂₆, and/or a C-terminal extension, or a pharmaceutical composition containing said variant.

In some embodiments of any of the above aspects, the variant has the sequence of SEQ ID NO: 41. In some embodiments, the variant having the sequence of SEQ ID NO: 41 has the amino acid K at position X₁₇, the amino acid sequence TEEN or TKEN at positions X₂₃, X₂₄, X₂₅, and X₂₆, and/or a C-terminal extension (e.g., 1, 2, 3, 4, 5, 6, or more additional amino acids at the C-terminus, e.g., the amino acids NP or NPVTPK (SEQ ID NO: 155)). In some embodiments of any of the above aspects, the method includes increasing muscle mass or treating a muscle disorder in a subject in need thereof (e.g., a subject having DMD, FSHD, IBM, ALS, sarcopenia, or cancer cachexia), affecting myostatin, activin, and/or BMP9 signaling in a subject (e.g., a subject having or at risk of developing DMD, FSHD, IBM, ALS, sarcopenia, cancer cachexia, obesity, Type-1 diabetes, or Type-2 diabetes), reducing body fat or body weight in a subject (e.g., a subject having obesity, Type-1 diabetes, or Type-2 diabetes), or treating and/or preventing a metabolic disease in a subject (e.g., a subject having or at risk of developing obesity, Type-1 diabetes, or Type-2 diabetes) by administering to the subject a therapeutically effective amount of a variant having the sequence of SEQ ID NO: 41, optionally having the amino acid K at position X₁₇, the amino acid sequence TEEN or TKEN at positions X₂₃, X₂₄, X₂₅, and X₂₆, and/or a C-terminal extension, or a pharmaceutical composition containing said variant.

In some embodiments of any of the above aspects, the variant has the sequence of SEQ ID NO: 44. In some embodiments, the variant having the sequence of SEQ ID NO: 44 has the amino acid K at position X₁₇, the amino acid sequence TEEN or TKEN at positions X₂₃, X₂₄, X₂₅, and X₂₆, and/or a C-terminal extension (e.g., 1, 2, 3, 4, 5, 6 or more additional amino acids at the C-terminus, e.g., the amino acids NP or NPVTPK (SEQ ID NO: 155)). In some embodiments of any of the above aspects, the method includes increasing muscle mass or treating a muscle disorder in a subject in need thereof (e.g., a subject having DMD, FSHD, IBM, ALS, sarcopenia, or cancer cachexia), affecting myostatin, activin, and/or BMP9 signaling in a subject (e.g., a subject having or at risk of developing DMD, FSHD, IBM, ALS, sarcopenia, cancer cachexia, obesity, Type-1 diabetes, or Type-2 diabetes), reducing body fat or body weight in a subject (e.g., a subject having obesity, Type-1 diabetes, or Type-2 diabetes), or treating and/or preventing a metabolic disease in a subject (e.g., a subject having or at risk of developing obesity, Type-1 diabetes, or Type-2 diabetes) by administering to the subject a therapeutically effective amount of a variant having the sequence of SEQ ID NO: 44, optionally having the amino acid K at position X₁₇, the amino acid sequence TEEN or TKEN at positions X₂₃, X₂₄, X₂₅, and X₂₆, and/or a C-terminal extension, or a pharmaceutical composition containing said variant.

In some embodiments of any of the above aspects, the variant has the sequence of SEQ ID NO: 70. In some embodiments, the variant having the sequence of SEQ ID NO: 70 has the amino acid K at position X₁₇, the amino acid sequence TEEN or TKEN at positions X₂₃, X₂₄, X₂₅, and X₂₆, and/or a C-terminal extension (e.g., 1, 2, 3, 4, 5, 6 or more additional amino acids at the C-terminus, e.g., the amino acids NP or NPVTPK (SEQ ID NO: 155)). In some embodiments of any of the above aspects, the method includes increasing muscle mass or treating a muscle disorder in a subject in need thereof (e.g., a subject having DMD, FSHD, IBM, ALS, sarcopenia, or cancer cachexia), affecting myostatin, activin, and/or BMP9 signaling in a subject (e.g., a subject having or at risk of developing DMD, FSHD, IBM, ALS, sarcopenia, cancer cachexia, obesity, Type-1 diabetes, or Type-2 diabetes), reducing body fat or body weight in a subject (e.g., a subject having obesity, Type-1 diabetes, or Type-2 diabetes), or treating and/or preventing a metabolic disease in a subject (e.g., a subject having or at risk of developing obesity, Type-1 diabetes, or Type-2 diabetes) by administering to the subject a therapeutically effective amount of a variant having the sequence of SEQ ID NO: 70, optionally having the amino acid K at position X₁₇, the amino acid sequence TEEN or TKEN at positions X₂₃, X₂₄, X₂₅, and X₂₆, and/or a C-terminal extension, or a pharmaceutical composition containing said variant.

In some embodiments of any of the above aspects, the variant has the sequence of SEQ ID NO: 71. In some embodiments, the variant having the sequence of SEQ ID NO: 71 has the amino acid K at position X₁₇, the amino acid sequence TEEN or TKEN at positions X₂₃, X₂₄, X₂₅, and X₂₆, and/or a C-terminal extension (e.g., 1, 2, 3, 4, 5, 6 or more additional amino acids at the C-terminus, e.g., the amino acids VTPK). In some embodiments of any of the above aspects, the method includes increasing muscle mass or treating a muscle disorder in a subject in need thereof (e.g., a subject having DMD, FSHD, IBM, ALS, sarcopenia, or cancer cachexia), affecting myostatin, activin, and/or BMP9 signaling in a subject (e.g., a subject having or at risk of developing DMD, FSHD, IBM, ALS, sarcopenia, cancer cachexia, obesity, Type-1 diabetes, or Type-2 diabetes), reducing body fat or body weight in a subject (e.g., a subject having obesity, Type-1 diabetes, or Type-2 diabetes), or treating and/or preventing a metabolic disease in a subject (e.g., a subject having or at risk of developing obesity, Type-1 diabetes, or Type-2 diabetes) by administering to the subject a therapeutically effective amount of a variant having the sequence of SEQ ID NO: 71, optionally having the amino acid K at position X₁₇, the amino acid sequence TEEN or TKEN at positions X₂₃, X₂₄, X₂₅, and X₂₆, and/or a C-terminal extension, or a pharmaceutical composition containing said variant.

In some embodiments of any of the above aspects, the variant has the sequence of SEQ ID NO: 72. In some embodiments, the variant having the sequence of SEQ ID NO: 72 has the amino acid K at position X₁₇ and/or the amino acid sequence TEEN or TKEN at positions X₂₃, X₂₄, X₂₅, and X₂₆. In some embodiments of any of the above aspects, the method includes increasing muscle mass or treating a muscle disorder in a subject in need thereof (e.g., a subject having DMD, FSHD, IBM, ALS, sarcopenia, or cancer cachexia), affecting myostatin, activin, and/or BMP9 signaling in a subject (e.g., a subject having or at risk of developing DMD, FSHD, IBM, ALS, sarcopenia, cancer cachexia, obesity, Type-1 diabetes, or Type-2 diabetes), reducing body fat or body weight in a subject (e.g., a subject having obesity, Type-1 diabetes, or Type-2 diabetes), or treating and/or preventing a metabolic disease in a subject (e.g., a subject having or at risk of developing obesity, Type-1 diabetes, or Type-2 diabetes) by administering to the subject a therapeutically effective amount of a variant having the sequence of SEQ ID NO: 72, optionally having the amino acid K at position X₁₇ and/or the amino acid sequence TEEN or TKEN at positions X₂₃, X₂₄, X₂₅, and X₂₆.

### Definitions

As used herein, the term "extracellular activin receptor type IIa (ActRlla) variant" refers to a peptide including a soluble, extracellular portion of the single transmembrane receptor, ActRlla, that has at least one amino acid substitution relative to a wild-type extracellular ActRlla (e.g., bold portion of the sequence of SEQ ID NO: 75 shown below) or an extracellular ActRlla having any one of the sequences of SEQ ID NOs: 76-96. The sequence of the wild-type, human ActRlla precursor protein is shown below (SEQ ID NO: 75), in which the signal peptide is italicized and the extracellular portion is bold.

Wild-type, human ActRIIa precursor protein (SEQ ID NO: 75):
MGAAAKLAFAVFLISCSSGAILGRSETQECLFFNANWEKDRTNQTGVEPCYGDKDKRRHCFAT
WKNISGSIEIVKQGCWLDDINCYDRTDCVEKKDSPEVYFCCCEGNMCNEKFSYFPEMEVTQPT
SNPVTPKPPYYNILLYSLVPLMLIAGIVICAFWVYRHHKMAYPPVLVPTQDPGPPPPSPLLGLKPL
QLLEVKARGRFGCVWKAQLLNEYVAVKIFPIQDKQSWQNEYEVYSLPGMKHENILQFIGAEKRG
TSVDVDLWLITAFHEKGSLSDFLKANVVSWNELCHIAETMARGLAYLHEDIPGLKDGHKPAISHR
DIKSKNVLLKNNLTACIADFGLALKFEAGKSAGDTHGQVGTRRYMAPEVLEGAINFQRDAFLRID
MYAMGLVLWELASRCTAADGPVDEYMLPFEEEIGQHPSLEDMQEVVVHKKKRPVLRDYWQKH
AGMAMLCETIEECWDHDAEARLSAGCVGERITQMQRLTNIITTEDIVTVVTMVTNVDFPPKESSL

An extracellular ActRlla variant may have a sequence of any one of SEQ ID NOs: 1-72. In particular embodiments, an extracellular ActRlla variant has a sequence of any one of SEQ ID NOs: 6-72 (Table 2). In some embodiments, an extracellular ActRlla variant may have at least 85% (e.g., at least 85%, 87%, 90%, 92%, 95%, 97%, or greater) amino acid sequence identity to the sequence of a wild-type extracellular ActRlla (SEQ ID NO: 73).

As used herein, the term "extracellular ActRllb variant" refers to a peptide including a soluble, extracellular portion of the single transmembrane receptor, ActRllb, that has at least one amino acid substitution relative to a wild-type extracellular ActRllb (e.g., the sequence of SEQ ID NO: 74). An extracellular ActRllb variant may have the sequence of SEQ ID NO: 149 shown below:

extracellular ActRIIb variant (SEQ ID NO: 149):
GRGEAETRECIFYNANWEKDRTNQSGLEPCYGDQDKRRHCFASWKNSSGTIELVKQGCWLDDI
NCYDRQECVAKKDSPEVYFCCCEGNFCNERFTHLPEAGGPEVTYEPPPTAPT

As used herein, the term "linker" refers to a linkage between two elements, e.g., peptides or protein domains. A polypeptide described herein may include an extracellular ActRlla variant (e.g., an extracellular ActRlla variant having a sequence of any one of SEQ ID NOs: 1-72 (e.g., SEQ ID NOs: 6-72)) fused to a moiety. The moiety may increase stability or improve pharmacokinetic properties of the polypeptide. The moiety (e.g., Fc domain monomer, a wild-type Fc domain, an Fc domain with amino acid substitutions (e.g., one or more substitutions that reduce dimerization), an albumin-binding peptide, a fibronectin domain, or a human serum albumin) may be fused to the polypeptide by way of a linker. A linker can be a covalent bond or a spacer. The term "bond" refers to a chemical bond, e.g., an amide bond or a disulfide bond, or any kind of bond created from a chemical reaction, e.g., chemical conjugation. The term "spacer" refers to a moiety (e.g., a polyethylene glycol (PEG) polymer) or an amino acid sequence (e.g., a 1-200 amino acid sequence) occurring between two elements, e.g., peptides or protein domains, to provide space and/or flexibility between the two elements. An amino acid spacer is part of the primary sequence of a polypeptide (e.g., fused to the spaced peptides via the polypeptide backbone). The formation of disulfide bonds, e.g., between two hinge regions that form an Fc domain, is not considered a linker.

As used herein, the term "Fc domain" refers to a dimer of two Fc domain monomers. An Fc domain has at least 80% sequence identity (e.g., at least 85%, 90%, 95%, 97%, or 100% sequence identity) to a human Fc domain that includes at least a C_{H}2 domain and a C_{H}3 domain. An Fc domain monomer includes second and third antibody constant domains (C_{H}2 and C_{H}3). In some embodiments, the Fc domain monomer also includes a hinge domain. An Fc domain does not include any portion of an immunoglobulin that is capable of acting as an antigen-recognition region, e.g., a variable domain or a complementarity determining region (CDR). In the wild-type Fc domain, the two Fc domain monomers dimerize by the interaction between the two C_{H}3 antibody constant domains, as well as one or more disulfide bonds that form between the hinge domains of the two dimerizing Fc domain monomers. In some embodiments, an Fc domain may be mutated to lack effector functions, typical of a "dead Fc domain." In certain embodiments, each of the Fc domain monomers in an Fc domain includes amino acid substitutions in the C_{H}2 antibody constant domain to reduce the interaction or binding between the Fc domain and an Fcγ receptor. In some embodiments, the Fc domain contains one or more amino acid substitutions that reduce or inhibit Fc domain dimerization. An Fc domain can be any immunoglobulin antibody isotype, including IgG, IgE, IgM, IgA, or IgD. Additionally, an Fc domain can be an IgG subtype (e.g., IgG1, IgG2a, IgG2b, IgG3, or IgG4). The Fc domain can also be a non-naturally occurring Fc domain, e.g., a recombinant Fc domain.

As used herein, the term "albumin-binding peptide" refers to an amino acid sequence of 12 to 16 amino acids that has affinity for and functions to bind serum albumin. An albumin-binding peptide can be of different origins, e.g., human, mouse, or rat. In some embodiments, an albumin-binding peptide has the sequence DICLPRWGCLW (SEQ ID NO: 152).

As used herein, the term "fibronectin domain" refers to a high molecular weight glycoprotein of the extracellular matrix, or a fragment thereof, that binds to, e.g., membrane-spanning receptor proteins such as integrins and extracellular matrix components such as collagens and fibrins. In some embodiments, a fibronectin domain is a fibronectin type III domain (SEQ ID NO: 153) having amino acids 610-702 of the sequence of UniProt ID NO: P02751. In other embodiments, a fibronectin domain is an adnectin protein.

As used herein, the term "human serum albumin" refers to the albumin protein present in human blood plasma. Human serum albumin is the most abundant protein in the blood. It constitutes about half of the blood serum protein. In some embodiments, a human serum albumin has the sequence of UniProt ID NO: P02768 (SEQ ID NO: 154).

As used herein, the term "fused" is used to describe the combination or attachment of two or more elements, components, or protein domains, e.g., peptides or polypeptides, by means including chemical conjugation, recombinant means, and chemical bonds, e.g., amide bonds. For example, two single peptides in tandem series can be fused to form one contiguous protein structure, e.g., a polypeptide, through chemical conjugation, a chemical bond, a peptide linker, or any other means of covalent linkage. In some embodiments of a polypeptide described herein, an extracellular ActRlla variant (e.g., an extracellular ActRlla variant having the sequence of any one of SEQ ID NOs: 1-72 (e.g., SEQ ID NOs: 6-72)) may be fused in tandem series to the N- or C-terminus of a moiety (e.g., Fc domain monomer (e.g., the sequence of SEQ ID NO: 97) a wild-type Fc domain (e.g., the sequence of SEQ ID NO: 151), an Fc domain with amino acid substitutions (e.g., one or more substitutions that reduce dimerization), an albumin-binding peptide (e.g., the sequence of SEQ ID NO: 152), a fibronectin domain (e.g., the sequence of SEQ ID NO: 153), or a human serum albumin (e.g., the sequence of SEQ ID NO: 154)) by way of a linker. For example, an extracellular ActRlla variant is fused to a moiety (e.g., an Fc domain monomer, a wild-type Fc domain, an Fc domain with amino acid substitutions (e.g., one or more substitutions that reduce dimerization), an albumin-binding peptide, a fibronectin domain, or a human serum albumin) by way of a peptide linker, in which the N-terminus of the peptide linker is fused to the C-terminus of the extracellular ActRlla variant through a chemical bond, e.g., a peptide bond, and the C-terminus of the peptide linker is fused to the N-terminus of the moiety (e.g., Fc domain monomer, wild-type Fc domain, Fc domain with amino acid substitutions (e.g., one or more substitutions that reduce dimerization), albumin-binding peptide, fibronectin domain, or human serum albumin) through a chemical bond, e.g., a peptide bond.

As used herein, the term "C-terminal extension" refers to the addition of one or more amino acids to the C-terminus of a polypeptide including an extracellular ActRlla variant (e.g., an extracellular ActRlla variant having the sequence of any one of SEQ ID NOs: 1-70 (e.g., SEQ ID NOs: 6-70)). The C-terminal extension can be 1-6 amino acids (e.g., 1, 2, 3, 4, 5, 6 or more amino acids). Exemplary C-terminal extensions are the amino acid sequence NP (a two amino acid C-terminal extension) and the amino acid sequence NPVTPK (SEQ ID NO: 155) (a six amino acid C-terminal extension). Any amino acid sequence that does not disrupt the activity of the polypeptide can be used. SEQ ID NO: 71, which is the sequence of SEQ ID NO: 69 with a C-terminal extension of NP, and SEQ ID NO: 72, which is the sequence of SEQ ID NO: 69 with a C-terminal extension of NPVTPK, represent two of the possible ways that a polypeptide of the invention can be modified to include a C-terminal extension.

As used herein, the term "percent (%) identity" refers to the percentage of amino acid (or nucleic acid) residues of a candidate sequence, e.g., an extracellular ActRlla variant, that are identical to the amino acid (or nucleic acid) residues of a reference sequence, e.g., a wild-type extracellular ActRlla (e.g., SEQ ID NO: 73), after aligning the sequences and introducing gaps, if necessary, to achieve the maximum percent identity (i.e., gaps can be introduced in one or both of the candidate and reference sequences for optimal alignment and non-homologous sequences can be disregarded for comparison purposes). Alignment for purposes of determining percent identity can be achieved in various ways that are within the skill in the art, for instance, using publicly available computer software such as BLAST, ALIGN, or Megalign (DNASTAR) software. Those skilled in the art can determine appropriate parameters for measuring alignment, including any algorithms needed to achieve maximal alignment over the full length of the sequences being compared. In some embodiments, the percent amino acid (or nucleic acid) sequence identity of a given candidate sequence to, with, or against a given reference sequence (which can alternatively be phrased as a given candidate sequence that has or includes a certain percent amino acid (or nucleic acid) sequence identity to, with, or against a given reference sequence) is calculated as follows: $100 \times \left(fraction of A/B\right)$ where A is the number of amino acid (or nucleic acid) residues scored as identical in the alignment of the candidate sequence and the reference sequence, and where B is the total number of amino acid (or nucleic acid) residues in the reference sequence. In some embodiments where the length of the candidate sequence does not equal to the length of the reference sequence, the percent amino acid (or nucleic acid) sequence identity of the candidate sequence to the reference sequence would not equal to the percent amino acid (or nucleic acid) sequence identity of the reference sequence to the candidate sequence.

In particular embodiments, a reference sequence aligned for comparison with a candidate sequence may show that the candidate sequence exhibits from 50% to 100% identity across the full length of the candidate sequence or a selected portion of contiguous amino acid (or nucleic acid) residues of the candidate sequence. The length of the candidate sequence aligned for comparison purpose is at least 30%, e.g., at least 40%, e.g., at least 50%, 60%, 70%, 80%, 90%, or 100% of the length of the reference sequence. When a position in the candidate sequence is occupied by the same amino acid (or nucleic acid) residue as the corresponding position in the reference sequence, then the molecules are identical at that position.

As used herein, the term "serum half-life" refers to, in the context of administering a therapeutic protein to a subject, the time required for plasma concentration of the protein in the subject to be reduced by half. The protein can be redistributed or cleared from the bloodstream, or degraded, e.g., by proteolysis. As described herein, a polypeptide including an extracellular ActRlla variant (e.g., an extracellular ActRlla variant having a sequence of any one of SEQ ID NOs: 1-72 (e.g., SEQ ID NOs: 6-72)) displays a serum half-life of 7 days in humans.

As used herein, the term "metabolic disease" refers to a disease, disorder, or syndrome that is related to a subject's metabolism, such as breaking down carbohydrates, proteins, and fats in food to release energy, and converting chemicals into other substances and transporting them inside cells for energy utilization and/or storage. Some symptoms of a metabolic disease include high serum triglycerides, high low-density cholesterol (LDL), low high-density cholesterol (HDL), and/or high fasting insulin levels, elevated fasting plasma glucose, abdominal (central) obesity, and elevated blood pressure. Metabolic diseases increase the risk of developing other diseases, such as cardiovascular disease. In the present invention, metabolic diseases include, but are not limited to, obesity, Type-1 diabetes, and Type-2 diabetes.

As used herein, the term "percentage of body weight gain" refers to the percentage of gained body weight compared to a prior body weight of a subject at a prior time. The percentage of body weight gain can be calculated as follows:$100 X \left[\left(body weight at a later time-body weight at a prior time\right) / \left(body weight at a prior time\right)\right]$ In the present invention, administration of a polypeptide including an extracellular ActRlla variant (e.g., an extracellular ActRlla variant having the sequence of any one of SEQ ID NOs: 1-72 (e.g., SEQ ID NOs: 6-72)), a nucleic acid molecule encoding a polypeptide including an extracellular ActRlla variant (e.g., an extracellular ActRlla variant having the sequence of any one of SEQ ID NOs: 1-72 (e.g., SEQ ID NOs: 6-72)), or vector containing such a nucleic acid molecule to a subject reduces the percentage of body weight gain of the subject.

As used herein, the term "appetite for food intake" refers to a subject's natural desire or need for food. The appetite for food intake of a subject can be monitored by measuring the amount of food consumed after the polypeptide including an extracellular ActRlla variant (e.g., an extracellular ActRlla variant having the sequence of any one of SEQ ID NOs: 1-72 (e.g., SEQ ID NOs: 6-72)) is administered. In the present invention, administration of a polypeptide including an extracellular ActRlla variant (e.g., an extracellular ActRlla variant having the sequence of any one of SEQ ID NOs: 1-72 (e.g., SEQ ID NOs: 6-72)), a nucleic acid molecule encoding a polypeptide including an extracellular ActRlla variant (e.g., an extracellular ActRlla variant having the sequence of any one of SEQ ID NOs: 1-72 (e.g., SEQ ID NOs: 6-72)), or vector containing such a nucleic acid molecule to a subject does not affect the subject's appetite for food intake.

As used herein, the term "adiposity" refers to the fat stored in the adipose tissue of a subject. In the present invention, administration of a polypeptide including an extracellular ActRlla variant (e.g., an extracellular ActRlla variant having the sequence of any one of SEQ ID NOs: 1-72 (e.g., SEQ ID NOs: 6-72)), a nucleic acid molecule encoding a polypeptide including an extracellular ActRlla variant (e.g., an extracellular ActRlla variant having the sequence of any one of SEQ ID NOs: 1-72 (e.g., SEQ ID NOs: 6-72)), or vector containing such a nucleic acid molecule to a subject reduces the subject's adiposity without affecting lean mass.

As used herein, the term "lean mass" refers to a component of body composition which includes, e.g., lean mass, body fat, and body fluid. Normally lean mass is calculated by subtracting the weights of body fat and body fluid from total body weight. Typically, a subject's lean mass is between 60% and 90% of totally body weight. In the present invention, administration of a polypeptide including an extracellular ActRlla variant (e.g., an extracellular ActRlla variant having the sequence of any one of SEQ ID NOs: 1-72 (e.g., SEQ ID NOs: 6-72)), a nucleic acid molecule encoding a polypeptide including an extracellular ActRlla variant (e.g., an extracellular ActRlla variant having the sequence of any one of SEQ ID NOs: 1-72 (e.g., SEQ ID NOs: 6-72)), or vector containing such a nucleic acid molecule to a subject reduces the subject's adiposity (i.e., fat) without affecting lean mass.

As used herein, the term "epididymal and perirenal fat pads" refers to the tightly packed fat cells in the epididymis and around the kidney. In the present invention, administration of a polypeptide including an extracellular ActRlla variant (e.g., an extracellular ActRlla variant having the sequence of any one of SEQ ID NOs: 1-72 (e.g., SEQ ID NOs: 6-72)), a nucleic acid molecule encoding a polypeptide including an extracellular ActRlla variant (e.g., an extracellular ActRlla variant having the sequence of any one of SEQ ID NOs: 1-72 (e.g., SEQ ID NOs: 6-72)), or vector containing such a nucleic acid molecule to a subject reduces the weights of epididymal and perirenal fat pads of the subject.

As used herein, the term "fasting insulin" refers to a subject's level of insulin while the subject has not had any food intake for a length of time (i.e., 12-24 hours). Fasting insulin level is used in diagnosing metabolic diseases. Fasting insulin level is also used as an indication of whether a subject is at the risk of developing a metabolic disease. Normally, in a subject suffering from Type-1 diabetes, the subject's fasting insulin level is low compared to that of a healthy subject. In a subject suffering from insulin resistance (i.e., Type-2 diabetes), the subject's fasting insulin level is high compared to that of a healthy subject. In the present invention, administration of a polypeptide including an extracellular ActRlla variant (e.g., an extracellular ActRlla variant having the sequence of any one of SEQ ID NOs: 1-72 (e.g., SEQ ID NOs: 6-72)), a nucleic acid molecule encoding a polypeptide including an extracellular ActRlla variant (e.g., an extracellular ActRlla variant having the sequence of any one of SEQ ID NOs: 1-72 (e.g., SEQ ID NOs: 6-72)), or vector containing such a nucleic acid molecule to a subject lowers the subject's fasting insulin level.

As used herein, the term "rate of glucose clearance" refers to the rate at which glucose is being cleared from the blood. The rate of glucose clearance can be measured in a glucose tolerance test (GTT). In a GTT, a subject is given a certain amount of glucose and blood samples are taken afterward to determine how quickly it is cleared from the blood. The rate of glucose clearance can be used as a parameter in diagnosing and/or determining the risk of developing metabolic diseases such as obesity, diabetes, and insulin resistance.

As used herein, the term "serum lipid profile" refers to the measurement of the distribution of different types of lipids and lipoproteins in a subject's serum. Such measurement can be accomplished by a panel of blood tests. The types of lipids and lipoproteins in a subject's serum include, but are not limited to, cholesterol (e.g., high-density lipoprotein (HDL) and low-density lipoprotein (LDL)), triglyceride, and free fatty acid (FFA). The distribution of the different types of lipids and lipoproteins can be used as a parameter in diagnosing and/or determining the risk of developing metabolic diseases such as obesity, diabetes, and insulin resistance. High levels of cholesterol, especially low-density lipoprotein, is generally regarded as an indication or risk factor for developing certain metabolic diseases, or in some severe medical cases, cardiovascular diseases. In the present invention, administration of a polypeptide including an extracellular ActRlla variant (e.g., an extracellular ActRlla variant having the sequence of any one of SEQ ID NOs: 1-72 (e.g., SEQ ID NOs: 6-72)), a nucleic acid molecule encoding a polypeptide including an extracellular ActRlla variant (e.g., an extracellular ActRlla variant having the sequence of any one of SEQ ID NOs: 1-72 (e.g., SEQ ID NOs: 6-72)), or vector containing such a nucleic acid molecule to a subject improves the subject's serum lipid profile such that the levels of cholesterol (especially low-density lipoprotein) and triglyceride are lowered.

As used herein, the term "affinity" or "binding affinity" refers to the strength of the binding interaction between two molecules. Generally, binding affinity refers to the strength of the sum total of non-covalent interactions between a molecule and its binding partner, such as an extracellular ActRlla variant and BMP9 or activin A. Unless indicated otherwise, binding affinity refers to intrinsic binding affinity, which reflects a 1:1 interaction between members of a binding pair. The binding affinity between two molecules is commonly described by the dissociation constant (K_{D}) or the affinity constant (K_{A}). Two molecules that have low binding affinity for each other generally bind slowly, tend to dissociate easily, and exhibit a large K_{D}. Two molecules that have high affinity for each other generally bind readily, tend to remain bound longer, and exhibit a small K_{D}. The K_{D} of two interacting molecules may be determined using methods and techniques well known in the art, e.g., surface plasmon resonance. K_{D} is calculated as the ratio of k_{off}/kₒₙ.

As used herein, the term "muscle mass" refers to a component of body composition. Normally muscle mass is calculated by subtracting the weights of body fat and body fluid from total body weight. The percentage of muscle mass may vary greatly among individuals depending on a subject's genetic makeup, age, race, and health status, etc. Typically, a subject's muscle mass may be between 20% and 50% of totally body weight.

As used herein, the phrase "affecting myostatin, activin, and/or BMP9 signaling" means changing the binding of myostatin, activin, and/or BMP9 to their receptors, e.g., ActRlla, ActRllb, and BMPRII (e.g., ActRlla). In some embodiments, a polypeptide including an extracellular ActRlla variant described herein reduces or inhibits the binding of myostatin, activin, and/or BMP9 to their receptors, e.g., ActRlla, ActRllb, and BMPRII (e.g., ActRlla). As described herein, a polypeptide of the invention including an extracellular ActRlla variant (e.g., an extracellular ActRlla variant having the sequence of any one of SEQ ID NOs: 1-72 (e.g., SEQ ID NOs: 6-72)) may have weak binding affinity to BMP9 (e.g., K_{D} of 200 pM or higher).

As used herein, the term "vascular complication" refers to a vascular disorder or any damage to the blood vessels, such as damage to the blood vessel walls. Damage to the blood vessel walls may cause an increase in vascular permeability or leakage. The term "vascular permeability or leakage" refers to the capacity of the blood vessel walls to allow the flow of small molecules, proteins, and cells in and out of blood vessels. An increase in vascular permeability or leakage may be caused by an increase in the gaps (e.g., an increase in the size and/or number of the gaps) between endothelial cells that line the blood vessel walls and/or thinning of the blood vessel walls.

As used herein, the term "polypeptide" describes a single polymer in which the monomers are amino acid residues which are covalently conjugated together through amide bonds. A polypeptide is intended to encompass any amino acid sequence, either naturally occurring, recombinant, or synthetically produced.

As used herein, the term "homodimer" refers to a molecular construct formed by two identical macromolecules, such as proteins or nucleic acids. The two identical monomers may form a homodimer by covalent bonds or non-covalent bonds. For example, an Fc domain may be a homodimer of two Fc domain monomers if the two Fc domain monomers contain the same sequence. In another example, a polypeptide described herein including an extracellular ActRlla variant fused to an Fc domain monomer may form a homodimer through the interaction of two Fc domain monomers, which form an Fc domain in the homodimer.

As used herein, the term "heterodimer" refers to a molecular construct formed by two different macromolecules, such as proteins or nucleic acids. The two monomers may form a heterodimer by covalent bonds or non-covalent bonds. For example, a polypeptide described herein including an extracellular ActRlla variant fused to an Fc domain monomer may form a heterodimer through the interaction of two Fc domain monomers, each fused to a different ActRlla variant, which form an Fc domain in the heterodimer.

As used herein, the term "host cell" refers to a vehicle that includes the necessary cellular components, e.g., organelles, needed to express proteins from their corresponding nucleic acids. The nucleic acids are typically included in nucleic acid vectors that can be introduced into the host cell by conventional techniques known in the art (transformation, transfection, electroporation, calcium phosphate precipitation, direct microinjection, etc.). A host cell may be a prokaryotic cell, e.g., a bacterial cell, or a eukaryotic cell, e.g., a mammalian cell (e.g., a CHO cell or a HEK293 cell).

As used herein, the term "therapeutically effective amount" refers an amount of a polypeptide, nucleic acid, or vector of the invention or a pharmaceutical composition containing a polypeptide, nucleic acid, or vector of the invention effective in achieving the desired therapeutic effect in treating a patient having a disease, such as a muscle disease, or a condition involving weakness and atrophy of muscles, e.g., Duchenne muscular dystrophy (DMD), facioscapulohumeral muscular dystrophy (FSHD), inclusion body myositis (IBM), amyotrophic lateral sclerosis (ALS), sarcopenia, or cancer cachexia. The term "therapeutically effective amount" also refers an amount of a polypeptide, nucleic acid, or vector of the invention or a pharmaceutical composition containing a polypeptide, nucleic acid, or vector of the invention effective in achieving the desired therapeutic effect in treating a patient having a disease, such as a metabolic disease, or a condition involving excess body weight, excess body fat, high blood glucose, high fasting insulin levels, or insulin resistance, e.g., obesity, Type-1 diabetes, or Type-2 diabetes. In particular, the therapeutically effective amount of the polypeptide, nucleic acid, or vector avoids adverse side effects.

As used herein, the term "pharmaceutical composition" refers to a medicinal or pharmaceutical formulation that includes an active ingredient as well as excipients and diluents to enable the active ingredient suitable for the method of administration. The pharmaceutical composition of the present invention includes pharmaceutically acceptable components that are compatible with the polypeptide, nucleic acid, or vector. The pharmaceutical composition may be in tablet or capsule form for oral administration or in aqueous form for intravenous or subcutaneous administration.

As used herein, the term "pharmaceutically acceptable carrier or excipient" refers to an excipient or diluent in a pharmaceutical composition. The pharmaceutically acceptable carrier must be compatible with the other ingredients of the formulation and not deleterious to the recipient. In the present invention, the pharmaceutically acceptable carrier or excipient must provide adequate pharmaceutical stability to the polypeptide including an extracellular ActRlla variant, the nucleic acid molecule(s) encoding the polypeptide, or a vector containing such nucleic acid molecule(s). The nature of the carrier or excipient differs with the mode of administration. For example, for intravenous administration, an aqueous solution carrier is generally used; for oral administration, a solid carrier is preferred.

As used herein, the term "treating and/or preventing" refers to the treatment and/or prevention of a disease, e.g., a metabolic disease (e.g., obesity, Type1 and Type-2 diabetes) or a muscle disease (e.g., DMD, FSHD, IBM, and ALS), using methods and compositions of the invention. Generally, treating a metabolic or muscle disease occurs after a subject has developed the metabolic or muscle disease and/or is already diagnosed with the metabolic or muscle disease. Preventing a metabolic or muscle disease refers to steps or procedures taken when a subject is at risk of developing the metabolic or muscle disease. The subject may show signs or mild symptoms that are judged by a physician to be indications or risk factors for developing the metabolic or muscle disease or have a family history or genetic predisposition of developing the metabolic or muscle disease, but has not yet developed the disease.

As used herein, the term "subject" refers to a mammal, e.g., preferably a human. Mammals include, but are not limited to, humans and domestic and farm animals, such as monkeys (e.g., a cynomolgus monkey), mice, dogs, cats, horses, and cows, etc.

### DESCRIPTION OF THE DRAWINGS

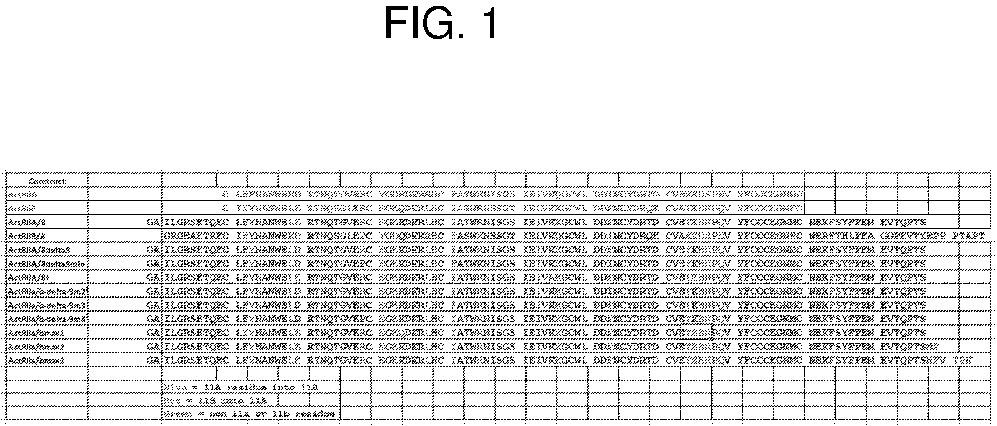

FIG. 1 is a sequence alignment showing the wild-type sequences of extracellular ActRlla and ActRllb and the amino acid substitutions in ActRlla variants.
FIGS. 2A and 2B are scatter plots showing the effects of extracellular ActRlla variants on body weight. Mice received a single hydrodynamic injection of a plasmid construct encoding the indicated ActRlla variant or a control plasmid.
FIGS. 3A and 3B are bar graphs showing the effects of extracellular ActRlla variants on muscle mass.
FIG. 4A is a scatter plot showing the effects of extracellular ActRlla variants on body weight. Mice received an intraperitoneal injection of the indicated purified recombinant ActRlla variant or a vehicle control twice weekly for four weeks.
FIG. 4B is a bar graph showing the effects of extracellular ActRlla variants on individual muscle weights by tissue analysis.
FIG. 5A is a scatter plot showing the effects of extracellular ActRlla variants on body weight during the course of the study. Mice received a single hydrodynamic injection of a plasmid construct encoding the indicated ActRlla variant or a control plasmid.
FIG. 5B is a bar graph showing the effects of extracellular ActRlla variants on body weight at the end of 28 days.
FIGS. 6A and 6B are bar graphs showing the effects of extracellular ActRlla variants on body weight by tissue analysis.
FIGS. 7A and 7B are scatter plots showing the effects of different doses of extracellular ActRlla variants on body weight. Mice received an intraperitoneal injection of the indicated purified recombinant ActRlla variant or a vehicle control twice weekly for four weeks.
FIGS. 8A and 8B are bar graphs showing the effects of different doses of extracellular ActRlla variants on muscle mass (FIG. 8A) and fat mass (FIG. 8B).
FIGS. 9A and 9B are bar graphs showing the effects of different doses of extracellular ActRlla variants on muscle weights by tissue analysis.

### DETAILED DESCRIPTION OF THE INVENTION

The invention features polypeptides that include an extracellular activin receptor type IIa (ActRlla) variant. In some embodiments, a polypeptide of the invention includes an extracellular ActRlla variant fused to a moiety (e.g., Fc domain monomer, a wild-type Fc domain, an Fc domain with amino acid substitutions (e.g., one or more substitutions that reduce dimerization), an albumin-binding peptide, a fibronectin domain, or a human serum albumin). A polypeptide including an extracellular ActRlla variant fused to an Fc domain monomer may also form a dimer (e.g., homodimer or heterodimer) through the interaction between two Fc domain monomers. The ActRlla variants described herein have weak binding affinity or no binding affinity to bone morphogenetic protein 9 (BMP9) compared to activins and myostatin. The invention also includes methods of treating diseases and conditions involving weakness and atrophy of muscles by increasing muscle mass and strength, methods of treating or preventing metabolic diseases, or methods of affecting myostatin, activin, and/or BMP9 signaling in a subject by administering to the subject a polypeptide including an extracellular ActRlla variant described herein.

### I. Extracellular activin receptor type IIa (ActRlla) variants

Activin type II receptors are single transmembrane domain receptors that modulate signals for ligands in the transforming growth factor β (TGF-β) superfamily. Ligands in the TGF-β superfamily are involved in a host of physiological processes, such as muscle growth, vascular growth, cell differentiation, homeostasis, and osteogenesis. Examples of ligands in the TGF-β superfamily include, e.g., activin, inhibin, growth differentiation factors (GDFs) (e.g., GDF8, also known as myostatin), and bone morphogenetic proteins (BMPs) (e.g., BMP9). Myostatin and activins are known to play a role in the regulation of skeletal muscle growth. For example, mice without myostatin show a large increase in skeletal muscle mass.

Activins are also highly expressed in adipose tissue, and increased myostatin levels and activin receptor levels have been observed in subcutaneous and visceral fat of obese mice. Additionally, myostatin has been shown to be elevated in skeletal muscle and plasma of obese and insulin resistant women, and both type I and type II activin receptors have been linked to pancreatic function and diabetes. These data suggest that increased signaling through activin receptors, either due to increased expression of activin ligands (e.g., activin, myostatin) or increased expression of activin receptors themselves, could lead to obesity and metabolic disorders, such as Type-1 and Type-2 diabetes. Methods that reduce or inhibit this signaling could, therefore, be used in the treatment of obesity and metabolic disorders.

There exist two types of activin type II receptors: ActRlla and ActRllb. Studies have shown that BMP9 binds ActRllb with about 300-fold higher binding affinity than ActRlla (see, e.g., Townson et al., J. Biol. Chem. 287:27313, 2012). ActRlla is known to have a longer half-life compared to ActRllb. The present invention describes extracellular ActRlla variants that are constructed by introducing amino acid residues of ActRllb to ActRlla, with the goal of imparting physiological properties conferred by ActRllb, while also maintaining beneficial physiological and pharmacokinetic properties of ActRlla. The optimum peptides confer significant increases in muscle mass, while retaining longer serum half-life and low binding-affinity to BMP9, for example. The preferred ActRlla variants also exhibit improved binding to activins and/or myostatin compared to wild-type ActRlla, which allows them to compete with endogenous activin receptors for ligand binding and reduce or inhibit endogenous activin receptor signaling. These variants can be used to treat disorders in which activin receptor signaling is elevated, such as metabolic disorders, leading to a reduction in body fat, body weight, or insulin resistance (e.g., an increase in insulin sensitivity). In some embodiments, amino acid substitutions may be introduced to an extracellular ActRlla variant to reduce or remove the binding affinity of the variant to BMP9. The wild-type amino acid sequences of the extracellular portions of human ActRlla and ActRllb are shown below.

Human ActRlla, extracellular portion (SEQ ID NO: 73):

GAILGRSETQECLFFNANWEKDRTNQTGVEPCYGDKDKRRHCFATWKNISGSIEIVKQGC
WLDDINCYDRTDCVEKKDSPEVYFCCCEGNMCNEKFSYFPEMEVTQPTS

Human ActRllb, extracellular portion (SEQ ID NO: 74):

GRGEAETRECIYYNANWELERTNQSGLERCEGEQDKRLHCYASWRNSSGTIELVKKGCWL
DDFNCYDRQECVATEENPQVYFCCCEGNFCNERFTHLPEAGGPEVTYEPPPTAPT

Polypeptides described herein include an extracellular ActRlla variant having at least one amino acid substitution relative to the wild-type extracellular ActRlla having the sequence of SEQ ID NO: 73 or the extracellular ActRlla having any one of the sequences of SEQ ID NOs: 76-96. Possible amino acid substitutions at 27 different positions may be introduced to an extracellular ActRlla variant (Table 1). In some embodiments, an extracellular ActRlla variant may have at least 85% (e.g., at least 85%, 87%, 90%, 92%, 95%, 97%, or greater) amino acid sequence identity to the sequence of a wild-type extracellular ActRlla (SEQ ID NO: 73). An extracellular ActRlla variant may have one or more (e.g., 1-27, 1-25, 1-23, 1-21, 1-19, 1-17, 1-15, 1-13, 1-11, 1-9, 1-7, 1-5, 1-3, or 1-2; e.g., 1, 2, 3, 4, 5, 6, 7, 8, 9, 10, 11, 12, 13, 14, 15, 16, 17, 18, 19, 20, 21, 22, 23, 24, 25, 26, or 27) amino acid substitutions relative the sequence of a wild-type extracellular ActRlla (SEQ ID NO: 73). In some embodiments, an extracellular ActRlla variant (e.g., an extracellular ActRlla variant having a sequence of SEQ ID NO: 1) may include amino acid substitutions at all of the 27 positions as listed in Table 1. In some embodiments, an extracellular ActRlla variant may include amino acid substitutions at a number of positions, e.g., at 2, 4, 6, 8, 10, 12, 14, 16, 18, 20, 22, 24, or 26 out of the 27 positions, as listed in Table 1.

Amino acid substitutions can worsen or improve the activity and/or binding affinity of the ActRlla variants of the invention. To maintain polypeptide function, it is important that the lysine (K) at position X₁₇ in the sequences shown in Tables 1 and 2 (SEQ ID NOs: 1-72 (e.g., SEQ ID NOs: 6-72)) be retained. Substitutions at that position can lead to a loss of activity. For example, an ActRlla variant having the sequence GAILGRSETQECLFYNANWELERTNQTGVERCEGEKDKRLHCYATWRNISGSIEIVAKGCWLDDFNCYD RTDCVETEENPQVYFCCCEGNMCNEKFSYFPEMEVTQPTS (SEQ ID NO: 150) has reduced activity in vivo, indicating that the substitution of alanine (A) for lysine (K) at X₁₇ is not tolerated. ActRlla variants of the invention, including variants in Tables 1 and 2 (e.g., SEQ ID NOs: 1-72 (e.g., SEQ ID NOs: 6-72), therefore, retain amino acid K at position X₁₇.

The ActRlla variants of the invention preferably have reduced, weak, or no substantial binding to BMP9. BMP9 binding is reduced in ActRlla variants containing the amino acid sequence TEEN at positions X₂₃, X₂₄, X₂₅, and X₂₆, as well as in variants that maintain the amino acid K at position X24 and have the amino acid sequence TKEN at positions X₂₃, X₂₄, X₂₅, and X₂₆. The sequences TEEN and TKEN can be employed interchangeably in the ActRlla variants (e.g., the variants in Tables 1 and 2, e.g., SEQ ID NOs: 1-72 (e.g., SEQ ID NOs: 6-72)) of the invention to provide reduced BMP9 binding.

The ActRlla variants of the invention may further include a C-terminal extension (e.g., additional amino acids at the C-terminus). The C-terminal extension can add one to six additional amino acids at the C-terminus (e.g., 1, 2, 3, 4, 5, 6 or more additional amino acids) to any of the variants shown in Tables 1 and 2 (e.g., SEQ ID NOs: 1-70 (e.g., SEQ ID NOs: 6-70)). One potential C-terminal extension that can be included in the ActRlla variants of the invention is amino acid sequence NP. For example, the sequence including the C-terminal extension is SEQ ID NO: 71 (e.g., SEQ ID NO: 69 with a C-terminal extension of NP). Another exemplary C-terminal extension that can be included in the ActRlla variants of the invention is amino acid sequence NPVTPK (SEQ ID NO: 155). For example, the sequence including the C-terminal extension is SEQ ID NO: 72 (e.g., SEQ ID NO: 69 with a C-terminal extension of NPVTPK).

**Table 1. Amino acid substitutions in an extracellular ActRlla variant having a sequence of any one of SEQ ID NOs: 1-5**

| | | | |
|---|---|---|---|
| GAILGRSETQECLX1X2NANWX3X4X5X6TNQTGVEX7CX8GX9X10X11X12X13X14HCX15ATWX16NISGSIEIV X17X18GCX19X20X21DX22NCYDRTDCVEX23X24X25X26PX27VYFCCCEGNMCNEKFSYFPEMEVTQPTS (SEQ ID NO: 1) | | | |
| GAILGRSETQECLFX2NANWX3X4X5X6TNQTGVEX7CX8GX9KX11X12X13X14HCX15ATWX16NISGSIEIVX17X1 8GCX19X20X21DX22NCYDRTDCVEX23X24X25X26PX27VYFCCCEGNMCNEKFSYFPEMEVTQPTS (SEQ ID NO: 2) | | | |
| GAILGRSETQECLFX2NANWEX4X5RTNQTGVEX7CX8GX9KDKRX14HCX15ATWX16NISGSIEIVKX18GCWL DDX22NCYDRTDCVEX23X24X25X26PX27VYFCCCEGNMCNEKFSYFPEMEVTQPTS (SEQ ID NO: 3) | | | |
| GAILGRSETQECLFX2NANWEX4DRTNQTGVEX7CX8GX9KDKRX14HCX15ATWX16NISGSIEIVKX18GCWL DDX22NCYDRTDCVEX23KX25X26PX27VYFCCCEGNMCNEKFSYFPEMEVTQPTS (SEQ ID NO: 4) | | | |
| GAILGRSETQECLFX2NANWEX4DRTNQTGVEPCX8GX9KDKRX14HCFATWKNISGSIEIVKX18GCWLDDI NCYDRTDCVEX23KX25X26PX27VYFCCCEGNMCNEKFSYFPEMEVTQPTS (SEQ ID NO: 5) | | | |
| **X₁** | F or Y | **X₁₅** | F or Y |
| **X₂** | F or Y | **X₁₆** | K, R, or A |
| **X₃** | E or A | **X₁₇** | K, A, Y, F, or I |
| **X₄** | K or L | **X₁₈** | Q or K |
| **X₅** | D or E | **X₁₉** | W or A |
| **X₆** | R or A | **X₂₀** | L or A |
| **X₇** | P or R | **X₂₁** | D, K, R, A, F, G, M, N, or I |
| **X₈** | Y or E | **X₂₂** | I, F, or A |
| **X₉** | D or E | **X₂₃** | K or T |
| **X₁₀** | K or Q | **X₂₄** | K or E |
| **X₁₁** | D or A | **X₂₅** | D or E |
| **X₁₂** | K or A | **X₂₆** | S or N |
| **X₁₃** | R or A | **X₂₇** | E or Q |
| **X₁₄** | R or L | | |

In some embodiments of the extracellular ActRlla variant having the sequence of SEQ ID NO: 2, X₃ is E, X₆ is R, X₁₁ is D, X₁₂ is K, X₁₃ is R, X₁₆ is K or R, X₁₇ is K, X₁₉ is W, X₂₀ is L, X₂₁ is D, and X₂₂ is I or F. In some embodiments of the extracellular ActRlla variant having the sequence of SEQ ID NO: 1 or 2, X₁₇ is K. In some embodiments of the extracellular ActRlla variant having the sequence of SEQ ID NOs: 1-3, X₁₇ is K, X₂₃ is T, X₂₄ is E, X₂₅ is E, and X₂₆ is N. In some embodiments of the extracellular ActRlla variant having the sequence of any one of SEQ ID NOs: 1-5, X₁₇ is K, X₂₃ is T, X₂₄ is K, X₂₅ is E, and X₂₆ is N.

In some embodiments, a polypeptide described herein includes an extracellular ActRlla variant having a sequence of any one of SEQ ID NOs: 6-72 (Table 2).

**Table 2. Extracellular ActRlla variants having the sequences of SEQ ID NOs: 6-72**

| **SEQ ID NO** | **Amino Acid Sequence** |
|---|---|
| 6 | GAILGRSETQECLFYNANWELDRTNQTGVEPCEGEKDKRLHCFATWKNISGSIEIV KKGCWLDDINCYDRTDCVETKENPQVYFCCCEGNMCNEKFSYFPEMEVTQPTS |
| 7 | GAILGRSETQECLFYNANWELERTNQTGVEPCEGEKDKRLHCFATWKNISGSIEIV KKGCWLDDINCYDRTDCVETKENPQVYFCCCEGNMCNEKFSYFPEMEVTQPTS |
| 8 | GAILGRSETQECLFYNANWELDRTNQTGVERCEGEKDKRLHCFATWKNISGSIEIV KKGCWLDDINCYDRTDCVETKENPQVYFCCCEGNMCNEKFSYFPEMEVTQPTS |
| 9 | GAILGRSETQECLFYNANWELDRTNQTGVEPCEGEKDKRLHCYATWKNISGSIEIV KKGCWLDDINCYDRTDCVETKENPQVYFCCCEGNMCNEKFSYFPEMEVTQPTS |
| 10 | GAILGRSETQECLFYNANWELDRTNQTGVEPCEGEKDKRLHCFATWRNISGSIEIV KKGCWLDDINCYDRTDCVETKENPQVYFCCCEGNMCNEKFSYFPEMEVTQPTS |
| 11 | GAILGRSETQECLFYNANWELDRTNQTGVEPCEGEKDKRLHCFATWKNISGSIEIV KKGCWLDDFNCYDRTDCVETKENPQVYFCCCEGNMCNEKFSYFPEMEVTQPTS |
| 12 | GAILGRSETQECLFYNANWELDRTNQTGVEPCEGEKDKRLHCFATWKNISGSIEIV KKGCWLDDINCYDRTDCVETEENPQVYFCCCEGNMCNEKFSYFPEMEVTQPTS |
| 13 | GAILGRSETQECLFYNANWELERTNQTGVERCEGEKDKRLHCFATWKNISGSIEIV KKGCWLDDINCYDRTDCVETKENPQVYFCCCEGNMCNEKFSYFPEMEVTQPTS |
| 14 | GAILGRSETQECLFYNANWELERTNQTGVEPCEGEKDKRLHCYATWKNISGSIEIV KKGCWLDDINCYDRTDCVETKENPQVYFCCCEGNMCNEKFSYFPEMEVTQPTS |
| 15 | GAILGRSETQECLFYNANWELERTNQTGVEPCEGEKDKRLHCFATWRNISGSIEIV KKGCWLDDINCYDRTDCVETKENPQVYFCCCEGNMCNEKFSYFPEMEVTQPTS |
| 16 | GAILGRSETQECLFYNANWELERTNQTGVEPCEGEKDKRLHCFATWKNISGSIEIV KKGCWLDDFNCYDRTDCVETKENPQVYFCCCEGNMCNEKFSYFPEMEVTQPTS |
| 17 | GAILGRSETQECLFYNANWELERTNQTGVEPCEGEKDKRLHCFATWKNISGSIEIV KKGCWLDDINCYDRTDCVETEENPQVYFCCCEGNMCNEKFSYFPEMEVTQPTS |
| 18 | GAILGRSETQECLFYNANWELDRTNQTGVERCEGEKDKRLHCYATWKNISGSIEIV KKGCWLDDINCYDRTDCVETKENPQVYFCCCEGNMCNEKFSYFPEMEVTQPTS |
| 19 | GAILGRSETQECLFYNANWELDRTNQTGVERCEGEKDKRLHCFATWRNISGSIEIV KKGCWLDDINCYDRTDCVETKENPQVYFCCCEGNMCNEKFSYFPEMEVTQPTS |
| 20 | GAILGRSETQECLFYNANWELDRTNQTGVERCEGEKDKRLHCFATWKNISGSIEIV KKGCWLDDFNCYDRTDCVETKENPQVYFCCCEGNMCNEKFSYFPEMEVTQPTS |
| 21 | GAILGRSETQECLFYNANWELDRTNQTGVERCEGEKDKRLHCFATWKNISGSIEIV KKGCWLDDINCYDRTDCVETEENPQVYFCCCEGNMCNEKFSYFPEMEVTQPTS |
| 22 | GAILGRSETQECLFYNANWELDRTNQTGVEPCEGEKDKRLHCYATWRNISGSIEIV KKGCWLDDINCYDRTDCVETKENPQVYFCCCEGNMCNEKFSYFPEMEVTQPTS |
| 23 | GAILGRSETQECLFYNANWELDRTNQTGVEPCEGEKDKRLHCYATWKNISGSIEIV KKGCWLDDFNCYDRTDCVETKENPQVYFCCCEGNMCNEKFSYFPEMEVTQPTS |
| 24 | GAILGRSETQECLFYNANWELDRTNQTGVEPCEGEKDKRLHCYATWKNISGSIEIV KKGCWLDDINCYDRTDCVETEENPQVYFCCCEGNMCNEKFSYFPEMEVTQPTS |
| 25 | GAILGRSETQECLFYNANWELDRTNQTGVEPCEGEKDKRLHCFATWRNISGSIEIV KKGCWLDDFNCYDRTDCVETKENPQVYFCCCEGNMCNEKFSYFPEMEVTQPTS |
| 26 | GAILGRSETQECLFYNANWELDRTNQTGVEPCEGEKDKRLHCFATWRNISGSIEIV KKGCWLDDINCYDRTDCVETEENPQVYFCCCEGNMCNEKFSYFPEMEVTQPTS |
| 27 | GAILGRSETQECLFYNANWELDRTNQTGVEPCEGEKDKRLHCFATWKNISGSIEIV KKGCWLDDFNCYDRTDCVETEENPQVYFCCCEGNMCNEKFSYFPEMEVTQPTS |
| 28 | GAILGRSETQECLFYNANWELERTNQTGVERCEGEKDKRLHCYATWKNISGSIEIV KKGCWLDDINCYDRTDCVETKENPQVYFCCCEGNMCNEKFSYFPEMEVTQPTS |
| 29 | GAILGRSETQECLFYNANWELERTNQTGVERCEGEKDKRLHCFATWRNISGSIEIV KKGCWLDDINCYDRTDCVETKENPQVYFCCCEGNMCNEKFSYFPEMEVTQPTS |
| 30 | GAILGRSETQECLFYNANWELERTNQTGVERCEGEKDKRLHCFATWKNISGSIEIV KKGCWLDDFNCYDRTDCVETKENPQVYFCCCEGNMCNEKFSYFPEMEVTQPTS |
| 31 | GAILGRSETQECLFYNANWELERTNQTGVERCEGEKDKRLHCFATWKNISGSIEIV KKGCWLDDINCYDRTDCVETEENPQVYFCCCEGNMCNEKFSYFPEMEVTQPTS |
| 32 | GAILGRSETQECLFYNANWELERTNQTGVEPCEGEKDKRLHCYATWRNISGSIEIV KKGCWLDDINCYDRTDCVETKENPQVYFCCCEGNMCNEKFSYFPEMEVTQPTS |
| 33 | GAILGRSETQECLFYNANWELERTNQTGVEPCEGEKDKRLHCYATWKNISGSIEIV KKGCWLDDFNCYDRTDCVETKENPQVYFCCCEGNMCNEKFSYFPEMEVTQPTS |
| 34 | GAILGRSETQECLFYNANWELERTNQTGVEPCEGEKDKRLHCYATWKNISGSIEIV KKGCWLDDINCYDRTDCVETEENPQVYFCCCEGNMCNEKFSYFPEMEVTQPTS |
| 35 | GAILGRSETQECLFYNANWELERTNQTGVEPCEGEKDKRLHCFATWRNISGSIEIV KKGCWLDDFNCYDRTDCVETKENPQVYFCCCEGNMCNEKFSYFPEMEVTQPTS |
| 36 | GAILGRSETQECLFYNANWELERTNQTGVEPCEGEKDKRLHCFATWRNISGSIEIV KKGCWLDDINCYDRTDCVETEENPQVYFCCCEGNMCNEKFSYFPEMEVTQPTS |
| 37 | GAILGRSETQECLFYNANWELERTNQTGVEPCEGEKDKRLHCFATWKNISGSIEIV KKGCWLDDFNCYDRTDCVETEENPQVYFCCCEGNMCNEKFSYFPEMEVTQPTS |
| 38 | GAILGRSETQECLFYNANWELDRTNQTGVERCEGEKDKRLHCYATWRNISGSIEIV KKGCWLDDINCYDRTDCVETKENPQVYFCCCEGNMCNEKFSYFPEMEVTQPTS |
| 39 | GAILGRSETQECLFYNANWELDRTNQTGVERCEGEKDKRLHCYATWKNISGSIEIV KKGCWLDDFNCYDRTDCVETKENPQVYFCCCEGNMCNEKFSYFPEMEVTQPTS |
| 40 | GAILGRSETQECLFYNANWELDRTNQTGVERCEGEKDKRLHCYATWKNISGSIEIV KKGCWLDDINCYDRTDCVETEENPQVYFCCCEGNMCNEKFSYFPEMEVTQPTS |
| 41 | GAILGRSETQECLFYNANWELDRTNQTGVERCEGEKDKRLHCFATWRNISGSIEIV KKGCWLDDFNCYDRTDCVETKENPQVYFCCCEGNMCNEKFSYFPEMEVTQPTS |
| 42 | GAILGRSETQECLFYNANWELDRTNQTGVERCEGEKDKRLHCFATWRNISGSIEIV KKGCWLDDINCYDRTDCVETEENPQVYFCCCEGNMCNEKFSYFPEMEVTQPTS |
| 43 | GAILGRSETQECLFYNANWELDRTNQTGVERCEGEKDKRLHCFATWKNISGSIEIV KKGC WLDDFNCYDRTDCVETEENPQVYFCCCEGNMCNEKFSYFPEMEVTQPTS |
| 44 | GAILGRSETQECLFYNANWELDRTNQTGVEPCEGEKDKRLHCYATWRNISGSIEIV KKGCWLDDFNCYDRTDCVETKENPQVYFCCCEGNMCNEKFSYFPEMEVTQPTS |
| 45 | GAILGRSETQECLFYNANWELDRTNQTGVEPCEGEKDKRLHCYATWRNISGSIEIV KKGCWLDDINCYDRTDCVETEENPQVYFCCCEGNMCNEKFSYFPEMEVTQPTS |
| 46 | GAILGRSETQECLFYNANWELDRTNQTGVEPCEGEKDKRLHCYATWKNISGSIEIV KKGCWLDDFNCYDRTDCVETEENPQVYFCCCEGNMCNEKFSYFPEMEVTQPTS |
| 47 | GAILGRSETQECLFYNANWELDRTNQTGVEPCEGEKDKRLHCFATWRNISGSIEIV KKGCWLDDFNCYDRTDCVETEENPQVYFCCCEGNMCNEKFSYFPEMEVTQPTS |
| 48 | GAILGRSETQECLFYNANWELERTNQTGVERCEGEKDKRLHCYATWRNISGSIEIV KKGCWLDDINCYDRTDCVETKENPQVYFCCCEGNMCNEKFSYFPEMEVTQPTS |
| 49 | GAILGRSETQECLFYNANWELERTNQTGVERCEGEKDKRLHCYATWKNISGSIEIV KKGCWLDDFNCYDRTDCVETKENPQVYFCCCEGNMCNEKFSYFPEMEVTQPTS |
| 50 | GAILGRSETQECLFYNANWELERTNQTGVERCEGEKDKRLHCYATWKNISGSIEIV KKGCWLDDINCYDRTDCVETEENPQVYFCCCEGNMCNEKFSYFPEMEVTQPTS |
| 51 | GAILGRSETQECLFYNANWELERTNQTGVERCEGEKDKRLHCFATWRNISGSIEIV KKGCWLDDFNCYDRTDCVETKENPQVYFCCCEGNMCNEKFSYFPEMEVTQPTS |
| 52 | GAILGRSETQECLFYNANWELERTNQTGVERCEGEKDKRLHCFATWRNISGSIEIV KKGCWLDDINCYDRTDCVETEENPQVYFCCCEGNMCNEKFSYFPEMEVTQPTS |
| 53 | GAILGRSETQECLFYNANWELERTNQTGVERCEGEKDKRLHCFATWKNISGSIEIV KKGCWLDDFNCYDRTDCVETEENPQVYFCCCEGNMCNEKFSYFPEMEVTQPTS |
| 54 | GAILGRSETQECLFYNANWELERTNQTGVEPCEGEKDKRLHCYATWRNISGSIEIV KKGCWLDDFNCYDRTDCVETKENPQVYFCCCEGNMCNEKFSYFPEMEVTQPTS |
| 55 | GAILGRSETQECLFYNANWELERTNQTGVEPCEGEKDKRLHCYATWRNISGSIEIV KKGCWLDDINCYDRTDCVETEENPQVYFCCCEGNMCNEKFSYFPEMEVTQPTS |
| 56 | GAILGRSETQECLFYNANWELERTNQTGVEPCEGEKDKRLHCYATWKNISGSIEIV KKGCWLDDFNCYDRTDCVETEENPQVYFCCCEGNMCNEKFSYFPEMEVTQPTS |
| 57 | GAILGRSETQECLFYNANWELERTNQTGVEPCEGEKDKRLHCFATWRNISGSIEIV KKGCWLDDFNCYDRTDCVETEENPQVYFCCCEGNMCNEKFSYFPEMEVTQPTS |
| 58 | GAILGRSETQECLFYNANWELDRTNQTGVERCEGEKDKRLHCYATWRNISGSIEIV KKGCWLDDFNCYDRTDCVETKENPQVYFCCCEGNMCNEKFSYFPEMEVTQPTS |
| 59 | GAILGRSETQECLFYNANWELDRTNQTGVERCEGEKDKRLHCYATWRNISGSIEIV KKGCWLDDINCYDRTDCVETEENPQVYFCCCEGNMCNEKFSYFPEMEVTQPTS |
| 60 | GAILGRSETQECLFYNANWELDRTNQTGVERCEGEKDKRLHCYATWKNISGSIEIV KKGCWLDDFNCYDRTDCVETEENPQVYFCCCEGNMCNEKFSYFPEMEVTQPTS |
| 61 | GAILGRSETQECLFYNANWELDRTNQTGVERCEGEKDKRLHCFATWRNISGSIEIV KKGCWLDDFNCYDRTDCVETEENPQVYFCCCEGNMCNEKFSYFPEMEVTQPTS |
| 62 | GAILGRSETQECLFYNANWELDRTNQTGVEPCEGEKDKRLHCYATWRNISGSIEIV KKGCWLDDFNCYDRTDCVETEENPQVYFCCCEGNMCNEKFSYFPEMEVTQPTS |
| 63 | GAILGRSETQECLFYNANWELERTNQTGVERCEGEKDKRLHCYATWRNISGSIEIV KKGCWLDDFNCYDRTDCVETKENPQVYFCCCEGNMCNEKFSYFPEMEVTQPTS |
| 64 | GAILGRSETQECLFYNANWELERTNQTGVERCEGEKDKRLHCYATWRNISGSIEIV KKGCWLDDINCYDRTDCVETEENPQVYFCCCEGNMCNEKFSYFPEMEVTQPTS |
| 65 | GAILGRSETQECLFYNANWELERTNQTGVERCEGEKDKRLHCYATWKNISGSIEIV KKGCWLDDFNCYDRTDCVETEENPQVYFCCCEGNMCNEKFSYFPEMEVTQPTS |
| 66 | GAILGRSETQECLFYNANWELERTNQTGVERCEGEKDKRLHCFATWRNISGSIEIV KKGCWLDDFNCYDRTDCVETEENPQVYFCCCEGNMCNEKFSYFPEMEVTQPTS |
| 67 | GAILGRSETQECLFYNANWELDRTNQTGVERCEGEKDKRLHCYATWRNISGSIEIV KKGCWLDDFNCYDRTDCVETEENPQVYFCCCEGNMCNEKFSYFPEMEVTQPTS |
| 69 | GAILGRSETQECLFYNANWELERTNQTGVEPCEGEKDKRLHCYATWRNISGSIEIV KKGCWLDDFNCYDRTDCVETEENPQVYFCCCEGNMCNEKFSYFPEMEVTQPTS |
| 69 | GAILGRSETQECLFYNANWELERTNQTGVERCEGEKDKRLHCYATWRNISGSIEIV KKGCWLDDFNCYDRTDCVETEENPQVYFCCCEGNMCNEKFSYFPEMEVTQPTS |
| 70 | GAILGRSETQECLYYNANWELERTNQTGVERCEGEQDKRLHCYATWRNISGSIEIV KKGCWLDDFNCYDRTDCVETEENPQVYFCCCEGNMCNEKFSYFPEMEVTQPTS |
| 71 | GAILGRSETQECLFYNANWELERTNQTGVERCEGEKDKRLHCYATWRNISGSIEIV KKGCWLDDFNCYDRTDCVETEENPQVYFCCCEGNMCNEKFSYFPEMEVTQPTSN P |
| 72 | GAILGRSETQECLFYNANWELERTNQTGVERCEGEKDKRLHCYATWRNISGSIEIV KKGCWLDDFNCYDRTDCVETEENPQVYFCCCEGNMCNEKFSYFPEMEVTQPTSN PVTPK |

In some embodiments, a polypeptide of the invention including an extracellular ActRlla variant (e.g., any one of SEQ ID NOs: 1-72 (e.g., SEQ ID NOs: 6-72)) has amino acid K at position X₁₇. Altering the amino acid at position X₁₇ can result in reduced activity. For example, an ActRlla variant having the sequence GAILGRSETQECLFYNANWELERTNQTGVERCEGEKDKRLHCYATWRNISGSIEIVAKGCWLDDFNCYD RTDCVETEENPQVYFCCCEGNMCNEKFSYFPEMEVTQPTS (SEQ ID NO: 150) has reduced activity in vivo, indicating that the substitution of A for K at X₁₇ is not tolerated.

In some embodiments, a polypeptide of the invention including an extracellular ActRlla variant (e.g., any one of SEQ ID NOs: 1-72 (e.g., SEQ ID NOs: 6-72)) with the sequence TEEN at positions X₂₃, X₂₄, X₂₅, and X₂₆ can have a substitution of the amino acid K for the amino acid E at position X₂₄. In some embodiments, a polypeptide of the invention including an extracellular ActRlla variant (e.g., any one of SEQ ID NOs: 1-72 (e.g., SEQ ID NOs: 6-72)) with the sequence TKEN at positions X₂₃, X₂₄, X₂₅, and X₂₆ can have a substitution of the amino acid E for the amino acid K at position X₂₄. Polypeptides having the sequence TEEN or TKEN at positions X₂₃, X₂₄, X₂₅, and X₂₆ have reduced or weak binding to BMP9.

In some embodiments, a polypeptide of the invention including an extracellular ActRlla variant (e.g., any one of SEQ ID NOs: 1-70 (e.g., SEQ ID NOs: 6-70)) may further include a C-terminal extension (e.g., additional amino acids at the C-terminus). In some embodiments, the C-terminal extension is amino acid sequence NP. For example, the sequence including the C-terminal extension is SEQ ID NO: 71 (e.g., SEQ ID NO: 69 with a C-terminal extension of NP). In some embodiments, the C-terminal extension is amino acid sequence NPVTPK (SEQ ID NO: 155). For example, the sequence including the C-terminal extension is SEQ ID NO: 72 (e.g., SEQ ID NO: 69 with a C-terminal extension of NPVTPK). The C-terminal extension can add one to six additional amino acids at the C-terminus (e.g., 1, 2, 3, 4, 5, 6 or more additional amino acids).

In some embodiments, a polypeptide of the invention including an extracellular ActRlla variant may further include a moiety (e.g., Fc domain monomer, a wild-type Fc domain, an Fc domain with amino acid substitutions (e.g., one or more substitutions that reduce dimerization), an albumin-binding peptide, a fibronectin domain, or a human serum albumin), which may be fused to the N- or C-terminus (e.g., C-terminus) of the extracellular ActRlla variant by way of a linker or other covalent bonds. A polypeptide including an extracellular ActRlla variant fused to an Fc domain monomer may form a dimer (e.g., homodimer or heterodimer) through the interaction between two Fc domain monomers, which combine to form an Fc domain in the dimer.

In some embodiments, an extracellular ActRlla variant described herein does not have the sequence of any one of SEQ ID NOs: 76-96 shown in Table 3 below.

**Table 3. Excluded Extracellular ActRlla Variants.**

| **SEQ ID NO** | **Amino Acid Sequence** |
|---|---|
| 76 | GAILGRSETQECLFFNANWEKDRTNQTGVEPCYGDKDKRRHCFATWANISGSIEIV KQGCWLDDINCYDRTDCVEKKDSPEVYFCCCEGNMCNEKFSYFPEMEVTQPTS |
| 77 | GAILGRSETQECLFFNANWAKDRTNQTGVEPCYGDKDKRRHCFATWKNISGSIEIV KQGCWLDDINCYDRTDCVEKKDSPEVYFCCCEGNMCNEKFSYFPEMEVTQPTS |
| 78 | GAILGRSETQECLFFNANWEKDATNQTGVEPCYGDKDRRHCFATWKNISGSIEIV KQGCWLDDINCYDRTDCVEKKDSPEVYFCCCEGNMCNEKFSYFPEMEVTQPTS |
| 79 | GAILGRSETQECLFFNANWEKDRTNQTGVEPCYGDKAKRRHCFATWKNISGSIEIV KQGCWLDDINCYDRTDCVEKKDSPEVYFCCCEGNMCNEKFSYFPEMEVTQPTS |
| 80 | GAILGRSETQECLFFNANWEKDRTNQTGVEPCYGDKDARRHCFATWKNISGSIEIV KQGCWLDDINCYDRTDCVEKKDSPEVYFCCCEGNMCNEKFSYFPEMEVTQPTS |
| 81 | GAILGRSETQECLFFNANWEKDRTNQTGVEPCYGDKDKARHCFATWKNISGSIEIV KQGCWLDDINCYDRTDCVEKKDSPEVYFCCCEGNMCNEKFSYFPEMEVTQPTS |
| 82 | GAILGRSETQECLFFNANWEKDRTNQTGVEPCYGDKDKRRHCFATWKNISGSIEIV AQGCWLDDINCYDRTDCVEKKDSPEVYFCCCEGNMCNEKFSYFPEMEVTQPTS |
| 83 | GAILGRSETQECLFFNANWEKDRTNQTGVEPCYGDKDKRRHCFATWKNISGSIEIV YQGCWLDDINCYDRTDCVEKKDSPEVYFCCCEGNMCNEKFSYFPEMEVTQPTS |
| 84 | GAILGRSETQECLFFNANWEKDRTNQTGVEPCYGDKDKRRHCFATWKNISGSIEIV FQGCWLDDINCYDRTDCVEKKDSPEVYFCCCEGNMCNEKFSYFPEMEVTQPTS |
| 85 | GAILGRSETQECLFFNANWEKDRTNQTGVEPCYGDKDKRRHCFATWKNISGSIEIVI QGCWLDDINCYDRTDCVEKKDSPEVYFCCCEGNMCNEKFSYFPEMEVTQPTS |
| 86 | GAILGRSETQECLFFNANWEKDRTNQTGVEPCYGDKDKRRHCFATWKNISGSIEIV KQGCALDDINCYDRTDCVEKKDSPEVYFCCCEGNMCNEKFSYFPEMEVTQPTS |
| 87 | GAILGRSETQECLFFNANWEKDRTNQTGVEPCYGDKDKRRHCFATWKNISGSIEIV KQGCWADDINCYDRTDCVEKKDSPEVYFCCCEGNMCNEKFSYFPEMEVTQPTS |
| 88 | GAILGRSETQECLFFNANWEKDRTNQTGVEPCYGDKDKRRHCFATWKNISGSIEIV KQGCWLKDINCYDRTDCVEKKDSPEVYFCCCEGNMCNEKFSYFPEMEVTQPTS |
| 89 | GAILGRSETQECLFFNANWEKDRTNQTGVEPCYGDKDKRRHCFATWKNISGSIEIV KQGCWLRDINCYDRTDCVEKKDSPEVYFCCCEGNMCNEKFSYFPEMEVTQPTS |
| 90 | GAILGRSETQECLFFNANWEKDRTNQTGVEPCYGDKDKRRHCFATWKNISGSIEIV KQGCWLADINCYDRTDCVEKKDSPEVYFCCCEGNMCNEKFSYFPEMEVTQPTS |
| 91 | GAILGRSETQECLFFNANWEKDRTNQTGVEPCYGDKDKRRHCFATWKNISGSIEIV KQGCWLFDINCYDRTDCVEKKDSPEVYFCCCEGNMCNEKFSYFPEMEVTQPTS |
| 92 | GAILGRSETQECLFFNANWEKDRTNQTGVEPCYGDKDKRRHCFATWKNISGSIEIV KQGCWLGDINCYDRTDCVEKKDSPEVYFCCCEGNMCNEKFSYFPEMEVTQPTS |
| 93 | GAILGRSETQECLFFNANWEKDRTNQTGVEPCYGDKDKRRHCFATWKNISGSIEIV KQGCWLMDINCYDRTDCVEKKDSPEVYFCCCEGNMCNEKFSYFPEMEVTQPTS |
| 94 | GAILGRSETQECLFFNANWEKDRTNQTGVEPCYGDKDKRRHCFATWKNISGSIEIV KQGCWLNDINCYDRTDCVEKKDSPEVYFCCCEGNMCNEKFSYFPEMEVTQPTS |
| 95 | GAILGRSETQECLFFNANWEKDRTNQTGVEPCYGDKDKRRHCFATWKNISGSIEIV KQGCWLIDINCYDRTDCVEKKDSPEVYFCCCEGNMCNEKFSYFPEMEVTQPTS |
| 96 | GAILGRSETQECLFFNANWEKDRTNQTGVEPCYGDKDKRRHCFATWKNISGSIEIV KQGCWLDDANCYDRTDCVEKKDSPEVYFCCCEGNMCNEKFSYFPEMEVTQPTS |

Furthermore, in some embodiments, a polypeptide described herein has a serum half-life of at least 7 days in humans. The polypeptide may bind to bone morphogenetic protein 9 (BMP9) with a K_{D} of 200 pM or higher. The polypeptide may bind to activin A with a K_{D} of 10 pM or higher. In some embodiments, the polypeptide does not bind to BMP9 or activin A. In some embodiments, the polypeptide binds to activin and/or myostatin and exhibits reduced (e.g., weak) binding to BMP9. In some embodiments, the polypeptide that has reduced or weak binding to BMP9 has the sequence TEEN or TKEN at positions X₂₃, X₂₄, X₂₅, and X₂₆.

Additionally, in some embodiments, the polypeptide may bind to human BMP9 with a K_{D} of about 200 pM or higher (e.g., a K_{D} of about 200, 300, 400, 500, 600, 700, 800, or 900 pM or higher, e.g., a K_{D} of about 1, 2, 3, 4, 5, 6, 7, 8, 9, 10, 20, 30, 40, or 50 nM or higher, e.g., a K_{D} of between about 200 pM and about 50 nM). In some embodiments, the polypeptide does not substantially bind to human BMP9. In some embodiments, the polypeptide may bind to human activin A with a K_{D} of about 800 pM or less (e.g., a K_{D} of about 800, 700, 600, 500, 400, 300, 200, 100, 90, 80, 70, 60, 50, 40, 30, 20, 10, 9, 8, 7, 6, 5, 4, 3, 2, or 1 pM or less, e.g., a K_{D} of between about 800 pM and about 200 pM). In some embodiments, the polypeptide may bind to human activin B with a K_{D} of 800 pM or less (e.g., a K_{D} of about 800, 700, 600, 500, 400, 300, 200, 100, 90, 80, 70, 60, 50, 40, 30, 20, 10, 9, 8, 7, 6, 5, 4, 3, 2, or 1 pM or less, e.g., a K_{D} of between about 800 pM and about 200 pM) The polypeptide may also bind to growth and differentiation factor 11 (GDF-11) with a K_{D} of approximately 5 pM or higher (e.g., a K_{D} of about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200 pM or higher).

### II. Fc domains

In some embodiments, a polypeptide described herein may include an extracellular ActRlla variant fused to an Fc domain monomer of an immunoglobulin or a fragment of an Fc domain to increase the serum half-life of the polypeptide. A polypeptide including an extracellular ActRlla variant fused to an Fc domain monomer may form a dimer (e.g., homodimer or heterodimer) through the interaction between two Fc domain monomers, which form an Fc domain in the dimer. As conventionally known in the art, an Fc domain is the protein structure that is found at the C-terminus of an immunoglobulin. An Fc domain includes two Fc domain monomers that are dimerized by the interaction between the C_{H}3 antibody constant domains. A wild-type Fc domain forms the minimum structure that binds to an Fc receptor, e.g., FcγRI, FcγRIIa, FcγRIIb, FcγRIIIa, FcγRIIIb, FcγRIV. In some embodiments, an Fc domain may be mutated to lack effector functions, typical of a "dead" Fc domain. For example, an Fc domain may include specific amino acid substitutions that are known to minimize the interaction between the Fc domain and an Fcγ receptor. In some embodiments, an Fc domain is from an IgG1 antibody and includes amino acid substitutions L234A, L235A, and G237A. In some embodiments, an Fc domain is from an IgG1 antibody and includes amino acid substitutions D265A, K322A, and N434A. The aforementioned amino acid positions are defined according to Kabat (Sequences of Proteins of Immunological Interest, 5th Ed. Public Health Service, National Institutes of Health, Bethesda, MD. (1991)). The Kabat numbering of amino acid residues may be determined for a given antibody by alignment at regions of homology of the sequence of the antibody with a "standard" Kabat numbered sequence. Furthermore, in some embodiments, an Fc domain does not induce any immune system-related response. For example, the Fc domain in a dimer of a polypeptide including an extracellular ActRlla variant fused to an Fc domain monomer may be modified to reduce the interaction or binding between the Fc domain and an Fcγ receptor. The sequence of an Fc domain monomer that may be fused to an extracellular ActRlla variant is shown below (SEQ ID NO: 97):

THTCPPCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPVPIEKTISKAKGQPREPQVYTL
PPSREEMTKNQVSLTCLVKGFYPSDIAVEWESNGQPENNYKTTPPVLDSDGPFFLYSKLTVDKS
RWQQGNVFSCSVMHEALHNHYTQKSLSLSPGK

In some embodiments, an Fc domain is from an IgG1 antibody and includes amino acid substitutions L12A, L13A, and G15A, relative to the sequence of SEQ ID NO: 97. In some embodiments, an Fc domain is from an IgG1 antibody and includes amino acid substitutions D43A, K100A, and N212A, relative to the sequence of SEQ ID NO: 97. In some embodiments, an extracellular ActRlla variant described herein (e.g., an extracellular ActRlla variant having the sequence of any one of SEQ ID NOs: 1-72 (e.g., SEQ ID NOs: 6-72)) may be fused to the N- or C-terminus of an Fc domain monomer (e.g., SEQ ID NO: 97) through conventional genetic or chemical means, e.g., chemical conjugation. If desired, a linker (e.g., a spacer) can be inserted between the extracellular ActRlla variant and the Fc domain monomer. The Fc domain monomer can be fused to the N- or C-terminus (e.g., C-terminus) of the extracellular ActRlla variant.

In some embodiments, a polypeptide described herein may include an extracellular ActRlla variant fused to an Fc domain. In some embodiments, the Fc domain contains one or more amino acid substitutions that reduce or inhibit Fc domain dimerization. In some embodiments, the Fc domain contains a hinge domain. The Fc domain can be of immunoglobulin antibody isotype IgG, IgE, IgM, IgA, or IgD. Additionally, the Fc domain can be an IgG subtype (e.g., IgG1, IgG2a, IgG2b, IgG3, or IgG4). The Fc domain can also be a non-naturally occurring Fc domain, e.g., a recombinant Fc domain.

Methods of engineering Fc domains that have reduced dimerization are known in the art. In some embodiments, one or more amino acids with large side-chains (e.g., tyrosine or tryptophan) may be introduced to the C_{H}3-C_{H}3 dimer interface to hinder dimer formation due to steric clash. In other embodiments, one or more amino acids with small side-chains (e.g., alanine, valine, or threonine) may be introduced to the C_{H}3-C_{H}3 dimer interface to remove favorable interactions. Methods of introducing amino acids with large or small side-chains in the C_{H}3 domain are described in, e.g., Ying et al. (J Biol Chem. 287:19399-19408, 2012), U.S. Patent Publication No. 2006/0074225, U.S. Patent Nos. 8,216,805 and 5,731,168, Ridgway et al. (Protein Eng. 9:617-612, 1996), Atwell et al. (J Mol Biol. 270:26-35, 1997), and Merchant et al. (Nat Biotechnol. 16:677-681, 1998), all of which are incorporated herein by reference in their entireties.

In yet other embodiments, one or more amino acid residues in the C_{H}3 domain that make up the C_{H}3-C_{H}3 interface between two Fc domains are replaced with positively-charged amino acid residues (e.g., lysine, arginine, or histidine) or negatively-charged amino acid residues (e.g., aspartic acid or glutamic acid) such that the interaction becomes electrostatically unfavorable depending on the specific charged amino acids introduced. Methods of introducing charged amino acids in the C_{H}3 domain to disfavor or prevent dimer formation are described in, e.g., Ying et al. (J Biol Chem. 287:19399-19408, 2012), U.S. Patent Publication Nos. 2006/0074225, 2012/0244578, and 2014/0024111, all of which are incorporated herein by reference in their entireties.

In some embodiments of the invention, an Fc domain includes one or more of the following amino acid substitutions:T366W, T366Y, T394W, F405W, Y349T, Y349E, Y349V, L351T, L351H, L351N, L352K, P353S, S354D, D356K, D356R, D356S, E357K, E357R, E357Q, S364A, T366E, L368T, L368Y, L368E, K370E, K370D, K370Q, K392E, K392D, T394N, P395N, P396T, V397T, V397Q, L398T, D399K, D399R, D399N, F405T, F405H, F405R, Y407T, Y407H, Y407I, K409E, K409D, K409T, and K409I, relative to the sequence of human IgG1. In one particular embodiment, an Fc domain includes the amino acid substitution T366W, relative to the sequence of human IgG1. The sequence of wild-type Fc domain is shown in SEQ ID NO: 151.

### III. Albumin-binding peptide

In some embodiments, a polypeptide described herein may include an extracellular ActRlla variant fused to a serum protein-binding peptide. Binding to serum protein peptides can improve the pharmacokinetics of protein pharmaceuticals.

As one example, albumin-binding peptides that can be used in the methods and compositions described here are generally known in the art. In one embodiment, the albumin binding peptide includes the sequence DICLPRWGCLW (SEQ ID NO: 152).

In the present invention, albumin-binding peptides may be joined to the N- or C-terminus (e.g., C-terminus) of an extracellular ActRlla variant described herein (e.g., an extracellular ActRlla variant having the sequence of any one of SEQ ID NOs: 1-72 (e.g., SEQ ID NOs: 6-72)) to increase the serum half-life of the extracellular ActRlla variant. In some embodiments, an albumin-binding peptide is joined, either directly or through a linker, to the N- or C-terminus of an extracellular ActRlla variant.

In some embodiments, an extracellular ActRlla variant described herein (e.g., an extracellular ActRlla variant having the sequence of any one of SEQ ID NOs: 1-72 (e.g., SEQ ID NOs: 6-72)) may be fused to the N- or C-terminus of albumin-binding peptide (e.g., SEQ ID NO: 152) through conventional genetic or chemical means, e.g., chemical conjugation. If desired, a linker (e.g., a spacer) can be inserted between the extracellular ActRlla variant and the albumin-binding peptide. Without being bound to a theory, it is expected that inclusion of an albumin-binding peptide in an extracellular ActRlla variant described herein may lead to prolonged retention of the therapeutic protein through its binding to serum albumin.

### IV. Fibronectin domain

In some embodiments, a polypeptide described herein may include an extracellular ActRlla variant fused to fibronectin domains. Binding to fibronectin domains can improve the pharmacokinetics of protein pharmaceuticals.

Fibronectin domain is a high molecular weight glycoprotein of the extracellular matrix, or a fragment thereof, that binds to, e.g., membrane-spanning receptor proteins such as integrins and extracellular matrix components such as collagens and fibrins. In some embodiments of the present invention, a fibronectin domain is joined to the N- or C-terminus (e.g., C-terminus) of an extracellular ActRlla variant described herein (e.g., an extracellular ActRlla variant having the sequence of any one of SEQ ID NOs: 1-72 (e.g., SEQ ID NOs: 6-72)) to increase the serum half-life of the extracellular ActRlla variant. A fibronectin domain can be joined, either directly or through a linker, to the N- or C-terminus of an extracellular ActRlla variant.

As one example, fibronectin domains that can be used in the methods and compositions described here are generally known in the art. In one embodiment, the fibronectin domain is a fibronectin type III domain (SEQ ID NO: 153) having amino acids 610-702 of the sequence of UniProt ID NO: P02751. In another embodiment, the fibronectin domain is an adnectin protein.

In some embodiments, an extracellular ActRlla variant described herein (e.g., an extracellular ActRlla variant having the sequence of any one of SEQ ID NOs: 1-72 (e.g., SEQ ID NOs: 6-72)) may be fused to the N- or C-terminus of a fibronectin domain (e.g., SEQ ID NO: 153) through conventional genetic or chemical means, e.g., chemical conjugation. If desired, a linker (e.g., a spacer) can be inserted between the extracellular ActRlla variant and the fibronectin domain. Without being bound to a theory, it is expected that inclusion of a fibronectin domain in an extracellular ActRlla variant described herein may lead to prolonged retention of the therapeutic protein through its binding to integrins and extracellular matrix components such as collagens and fibrins.

### V. Serum albumin

In some embodiments, a polypeptide described herein may include an extracellular ActRlla variant fused to serum albumin. Binding to serum albumins can improve the pharmacokinetics of protein pharmaceuticals.

Serum albumin is a globular protein that is the most abundant blood protein in mammals. Serum albumin is produced in the liver and constitutes about half of the blood serum proteins. It is monomeric and soluble in the blood. Some of the most crucial functions of serum albumin include transporting hormones, fatty acids, and other proteins in the body, buffering pH, and maintaining osmotic pressure needed for proper distribution of bodily fluids between blood vessels and body tissues. In preferred embodiments, serum albumin is human serum albumin. In some embodiments of the present invention, a human serum albumin is joined to the N- or C-terminus (e.g., C-terminus) of an extracellular ActRlla variant described herein (e.g., an extracellular ActRlla variant having the sequence of any one of SEQ ID NOs: 1-72 (e.g., SEQ ID NOs: 6-72)) to increase the serum half-life of the extracellular ActRlla variant. A human serum albumin can be joined, either directly or through a linker, to the N- or C-terminus of an extracellular ActRlla variant.

As one example, serum albumins that can be used in the methods and compositions described herein are generally known in the art. In one embodiment, the serum albumin includes the sequence of UniProt ID NO: P02768 (SEQ ID NO: 154).

In some embodiments, an extracellular ActRlla variant described herein (e.g., an extracellular ActRlla variant having the sequence of any one of SEQ ID NOs: 1-72 (e.g., SEQ ID NOs: 6-72)) may be fused to the N- or C-terminus of a human serum albumin (e.g., SEQ ID NO: 154) through conventional genetic or chemical means, e.g., chemical conjugation. If desired, a linker (e.g., a spacer) can be inserted between the extracellular ActRlla variant and the human serum albumin. Without being bound to a theory, it is expected that inclusion of a human serum albumin in an extracellular ActRlla variant described herein may lead to prolonged retention of the therapeutic protein.

### VI. Linkers

A polypeptide described herein may include an extracellular ActRlla variant (e.g., an extracellular ActRlla variant having a sequence of any one of SEQ ID NOs: 1-72 (e.g., SEQ ID NOs: 6-72)) fused to a moiety by way of a linker. In some embodiments, the moiety increases stability of the polypeptide. Exemplary moieties include an Fc domain monomer, a wild-type Fc domain, an Fc domain with amino acid substitutions (e.g., one or more substitutions that reduce dimerization), an albumin-binding peptide, a fibronectin domain, or a human serum albumin. In the present invention, a linker between a moiety (e.g., an Fc domain monomer (e.g., the sequence of SEQ ID NO: 97), a wild-type Fc domain (e.g., SEQ ID NO: 151), an Fc domain with amino acid substitutions (e.g., one or more substitutions that reduce dimerization), an albumin-binding peptide (e.g., SEQ ID NO: 152), a fibronectin domain (e.g., SEQ ID NO: 153), or a human serum albumin (e.g., SEQ ID NO: 154)) and an extracellular ActRlla variant (e.g., an extracellular ActRlla variant having the sequence of any one of SEQ ID NOs: 1-72 (e.g., SEQ ID NOs: 6-72)), can be an amino acid spacer including 1-200 amino acids. Suitable peptide spacers are known in the art, and include, for example, peptide linkers containing flexible amino acid residues such as glycine, alanine, and serine. In some embodiments, a spacer can contain motifs, e.g., multiple or repeating motifs, of GA, GS, GG, GGA, GGS, GGG, GGGA (SEQ ID NO: 98), GGGS (SEQ ID NO: 99), GGGG (SEQ ID NO: 100), GGGGA (SEQ ID NO: 101), GGGGS (SEQ ID NO: 102), GGGGG (SEQ ID NO: 103), GGAG (SEQ ID NO: 104), GGSG (SEQ ID NO: 105), AGGG (SEQ ID NO: 106), or SGGG (SEQ ID NO: 107). In some embodiments, a spacer can contain 2 to 12 amino acids including motifs of GA or GS, e.g., GA, GS, GAGA (SEQ ID NO: 108), GSGS (SEQ ID NO: 109), GAGAGA (SEQ ID NO: 110), GSGSGS (SEQ ID NO: 111), GAGAGAGA (SEQ ID NO: 112), GSGSGSGS (SEQ ID NO: 113), GAGAGAGAGA (SEQ ID NO: 114), GSGSGSGSGS (SEQ ID NO: 115), GAGAGAGAGAGA (SEQ ID NO: 116), and GSGSGSGSGSGS (SEQ ID NO: 117). In some embodiments, a spacer can contain 3 to 12 amino acids including motifs of GGA or GGS, e.g., GGA, GGS, GGAGGA (SEQ ID NO: 118), GGSGGS (SEQ ID NO: 119), GGAGGAGGA (SEQ ID NO: 120), GGSGGSGGS (SEQ ID NO: 121), GGAGGAGGAGGA (SEQ ID NO: 122), and GGSGGSGGSGGS (SEQ ID NO: 123). In yet some embodiments, a spacer can contain 4 to 12 amino acids including motifs of GGAG (SEQ ID NO: 104), GGSG (SEQ ID NO: 105), e.g., GGAG (SEQ ID NO: 104), GGSG (SEQ ID NO: 105), GGAGGGAG (SEQ ID NO: 124), GGSGGGSG (SEQ ID NO: 125), GGAGGGAGGGAG (SEQ ID NO: 126), and GGSGGGSGGGSG (SEQ ID NO: 127). In some embodiments, a spacer can contain motifs of GGGGA (SEQ ID NO: 101) or GGGGS (SEQ ID NO: 102), e.g., GGGGAGGGGAGGGGA (SEQ ID NO: 128) and GGGGSGGGGSGGGGS (SEQ ID NO: 129). In some embodiments of the invention, an amino acid spacer between a moiety (e.g., an Fc domain monomer, a wild-type Fc domain, an Fc domain with amino acid substitutions (e.g., one or more substitutions that reduce dimerization), an albumin-binding peptide, a fibronectin domain, or a human serum albumin) and an extracellular ActRlla variant (e.g., an extracellular ActRlla variant having the sequence of any one of SEQ ID NOs: 1-72 (e.g., SEQ ID NOs: 6-72)) may be GGG, GGGA (SEQ ID NO: 98), GGGG (SEQ ID NO: 100), GGGAG (SEQ ID NO: 130), GGGAGG (SEQ ID NO: 131), or GGGAGGG (SEQ ID NO: 132).

In some embodiments, a spacer can also contain amino acids other than glycine, alanine, and serine, e.g., AAAL (SEQ ID NO: 133), AAAK (SEQ ID NO: 134), AAAR (SEQ ID NO: 135), EGKSSGSGSESKST (SEQ ID NO: 136), GSAGSAAGSGEF (SEQ ID NO: 137), AEAAAKEAAAKA (SEQ ID NO: 138), KESGSVSSEQLAQFRSLD (SEQ ID NO: 139), GENLYFQSGG (SEQ ID NO: 140), SACYCELS (SEQ ID NO: 141), RSIAT (SEQ ID NO: 142), RPACKIPNDLKQKVMNH (SEQ ID NO: 143), GGSAGGSGSGSSGGSSGASGTGTAGGTGSGSGTGSG (SEQ ID NO: 144), AAANSSIDLISVPVDSR (SEQ ID NO: 145), or GGSGGGSEGGGSEGGGSEGGGSEGGGSEGGGSGGGS (SEQ ID NO: 146). In some embodiments, a spacer can contain motifs, e.g., multiple or repeating motifs, of EAAAK (SEQ ID NO: 147). In some embodiments, a spacer can contain motifs, e.g., multiple or repeating motifs, of proline-rich sequences such as (XP)ₙ, in which X may be any amino acid (e.g., A, K, or E) and n is from 1-5, and PAPAP(SEQ ID NO: 148).

The length of the peptide spacer and the amino acids used can be adjusted depending on the two protein involved and the degree of flexibility desired in the final protein fusion polypeptide. The length of the spacer can be adjusted to ensure proper protein folding and avoid aggregate formation.

### VII. Vectors, host cells, and protein production

The polypeptides of the invention can be produced from a host cell. A host cell refers to a vehicle that includes the necessary cellular components, e.g., organelles, needed to express the polypeptides and fusion polypeptides described herein from their corresponding nucleic acids. The nucleic acids may be included in nucleic acid vectors that can be introduced into the host cell by conventional techniques known in the art (e.g., transformation, transfection, electroporation, calcium phosphate precipitation, direct microinjection, infection, or the like). The choice of nucleic acid vectors depends in part on the host cells to be used. Generally, preferred host cells are of either eukaryotic (e.g., mammalian) or prokaryotic (e.g., bacterial) origin.

### Nucleic acid vector construction and host cells

A nucleic acid sequence encoding the amino acid sequence of a polypeptide of the invention may be prepared by a variety of methods known in the art. These methods include, but are not limited to, oligonucleotide-mediated (or site-directed) mutagenesis and PCR mutagenesis. A nucleic acid molecule encoding a polypeptide of the invention may be obtained using standard techniques, e.g., gene synthesis. Alternatively, a nucleic acid molecule encoding a wild-type extracellular ActRlla may be mutated to include specific amino acid substitutions using standard techniques in the art, e.g., QuikChange^{™} mutagenesis. Nucleic acid molecules can be synthesized using a nucleotide synthesizer or PCR techniques.

A nucleic acid sequence encoding a polypeptide of the invention may be inserted into a vector capable of replicating and expressing the nucleic acid molecule in prokaryotic or eukaryotic host cells. Many vectors are available in the art and can be used for the purpose of the invention. Each vector may include various components that may be adjusted and optimized for compatibility with the particular host cell. For example, the vector components may include, but are not limited to, an origin of replication, a selection marker gene, a promoter, a ribosome binding site, a signal sequence, the nucleic acid sequence encoding protein of interest, and a transcription termination sequence.

In some embodiments, mammalian cells may be used as host cells for the invention. Examples of mammalian cell types include, but are not limited to, human embryonic kidney (HEK) (e.g., HEK293, HEK 293F), Chinese hamster ovary (CHO), HeLa, COS, PC3, Vero, MC3T3, NS0, Sp2/0, VERY, BHK, MDCK, W138, BT483, Hs578T, HTB2, BT20, T47D, NS0 (a murine myeloma cell line that does not endogenously produce any immunoglobulin chains), CRL7O3O, and HsS78Bst cells. In some embodiments, *E. coli* cells may also be used as host cells for the invention. Examples of *E. coli* strains include, but are not limited to, *E. coli 294* (ATCC^{®} 31,446), *E. coli* λ 1776 (ATCC^{®} 31 ,537, *E. coli* BL21 (DE3) (ATCC^{®} BAA-1025), and *E. coli* RV308 (ATCC^{®} 31,608). Different host cells have characteristic and specific mechanisms for the posttranslational processing and modification of protein products (e.g., glycosylation). Appropriate cell lines or host systems may be chosen to ensure the correct modification and processing of the polypeptide expressed. The above-described expression vectors may be introduced into appropriate host cells using conventional techniques in the art, e.g., transformation, transfection, electroporation, calcium phosphate precipitation, and direct microinjection. Once the vectors are introduced into host cells for protein production, host cells are cultured in conventional nutrient media modified as appropriate for inducing promoters, selecting transformants, or amplifying the genes encoding the desired sequences. Methods for expression of therapeutic proteins are known in the art, see, for example, Paulina Balbas, Argelia Lorence (eds.) Recombinant Gene Expression: Reviews and Protocols (Methods in Molecular Biology), Humana Press; 2nd ed. 2004 and Vladimir Voynov and Justin A. Caravella (eds.) Therapeutic Proteins: Methods and Protocols (Methods in Molecular Biology) Humana Press; 2nd ed. 2012.

### Protein production, recovery, and purification

Host cells used to produce the polypeptides of the invention may be grown in media known in the art and suitable for culturing of the selected host cells. Examples of suitable media for mammalian host cells include Minimal Essential Medium (MEM), Dulbecco's Modified Eagle's Medium (DMEM), Expi293^{™} Expression Medium, DMEM with supplemented fetal bovine serum (FBS), and RPMI-1640. Examples of suitable media for bacterial host cells include Luria broth (LB) plus necessary supplements, such as a selection agent, e.g., ampicillin. Host cells are cultured at suitable temperatures, such as from about 20 °C to about 39 °C, e.g., from 25 °C to about 37 °C, preferably 37 °C, and CO₂ levels, such as 5 to 10%. The pH of the medium is generally from about 6.8 to 7.4, e.g., 7.0, depending mainly on the host organism. If an inducible promoter is used in the expression vector of the invention, protein expression is induced under conditions suitable for the activation of the promoter.

In some embodiments, depending on the expression vector and the host cells used, the expressed protein may be secreted from the host cells (e.g., mammalian host cells) into the cell culture media. Protein recovery may involve filtering the cell culture media to remove cell debris. The proteins may be further purified. A polypeptide of the invention may be purified by any method known in the art of protein purification, for example, by chromatography (e.g., ion exchange, affinity, and size-exclusion column chromatography), centrifugation, differential solubility, or by any other standard technique for the purification of proteins. For example, the protein can be isolated and purified by appropriately selecting and combining affinity columns such as Protein A column (e.g., POROS Protein A chromatography) with chromatography columns (e.g., POROS HS-50 cation exchange chromatography), filtration, ultra filtration, salting-out and dialysis procedures.

In other embodiments, host cells may be disrupted, e.g., by osmotic shock, sonication, or lysis, to recover the expressed protein. Once the cells are disrupted, cell debris may be removed by centrifugation or filtration. In some instances, a polypeptide can be conjugated to marker sequences, such as a peptide to facilitate purification. An example of a marker amino acid sequence is a hexa-histidine peptide (His-tag), which binds to nickel-functionalized agarose affinity column with micromolar affinity. Other peptide tags useful for purification include, but are not limited to, the hemagglutinin "HA" tag, which corresponds to an epitope derived from influenza hemagglutinin protein (Wilson et al., Cell 37:767, 1984).

Alternatively, the polypeptides of the invention can be produced by the cells of a subject (e.g., a human), e.g., in the context of gene therapy, by administrating a vector (such as a viral vector (e.g., a retroviral vector, adenoviral vector, poxviral vector (e.g., vaccinia viral vector, such as Modified Vaccinia Ankara (MVA)), adeno-associated viral vector, and alphaviral vector)) containing a nucleic acid molecule encoding the polypeptide of the invention. The vector, once inside a cell of the subject (e.g., by transformation, transfection, electroporation, calcium phosphate precipitation, direct microinjection, infection, etc.) will promote expression of the polypeptide, which is then secreted from the cell. If treatment of a disease or disorder is the desired outcome, no further action may be required. If collection of the protein is desired, blood may be collected from the subject and the protein purified from the blood by methods known in the art.

### VIII. Pharmaceutical compositions and preparations

The invention features pharmaceutical compositions that include the polypeptides described herein (e.g., a polypeptide including an extracellular ActRlla variant (e.g., an extracellular ActRlla variant having the sequence of any one of SEQ ID NOs: 1-72 (e.g., SEQ ID NOs: 6-72)). In some embodiments, a pharmaceutical composition of the invention includes a polypeptide including an extracellular ActRlla variant (e.g., an extracellular ActRlla variant having the sequence of any one of SEQ ID NOs: 1-70 (e.g., SEQ ID NOs: 6-70)) with a C-terminal extension (e.g., 1, 2, 3, 4, 5, 6 or more additional amino acids) as the therapeutic protein. In some embodiments, a pharmaceutical composition of the invention includes a polypeptide including an extracellular ActRlla variant (e.g., an extracellular ActRlla variant having the sequence of any one of SEQ ID NOs: 1-72 (e.g., SEQ ID NOs: 6-72)) fused to a moiety (e.g., Fc domain monomer, or a dimer thereof, a wild-type Fc domain, an Fc domain with amino acid substitutions (e.g., one or more substitutions that reduce dimerization), an albumin-binding peptide, a fibronectin domain, or a human serum albumin) as the therapeutic protein. In some embodiments, a pharmaceutical composition of the invention including a polypeptide of the invention may be used in combination with other agents (e.g., therapeutic biologics and/or small molecules) or compositions in a therapy. In addition to a therapeutically effective amount of the polypeptide, the pharmaceutical composition may include one or more pharmaceutically acceptable carriers or excipients, which can be formulated by methods known to those skilled in the art. In some embodiments, a pharmaceutical composition of the invention includes a nucleic acid molecule (DNA or RNA, e.g., mRNA) encoding a polypeptide of the invention, or a vector containing such a nucleic acid molecule.

Acceptable carriers and excipients in the pharmaceutical compositions are nontoxic to recipients at the dosages and concentrations employed. Acceptable carriers and excipients may include buffers such as phosphate, citrate, HEPES, and TAE, antioxidants such as ascorbic acid and methionine, preservatives such as hexamethonium chloride, octadecyldimethylbenzyl ammonium chloride, resorcinol, and benzalkonium chloride, proteins such as human serum albumin, gelatin, dextran, and immunoglobulins, hydrophilic polymers such as polyvinylpyrrolidone, amino acids such as glycine, glutamine, histidine, and lysine, and carbohydrates such as glucose, mannose, sucrose, and sorbitol. Pharmaceutical compositions of the invention can be administered parenterally in the form of an injectable formulation. Pharmaceutical compositions for injection can be formulated using a sterile solution or any pharmaceutically acceptable liquid as a vehicle. Pharmaceutically acceptable vehicles include, but are not limited to, sterile water, physiological saline, and cell culture media (e.g., Dulbecco's Modified Eagle Medium (DMEM), α-Modified Eagles Medium (α-MEM), F-12 medium). Formulation methods are known in the art, see e.g., Banga (ed.) Therapeutic Peptides and Proteins: Formulation, Processing and Delivery Systems (3rd ed.) Taylor & Francis Group, CRC Press (2015).

The pharmaceutical compositions of the invention may be prepared in microcapsules, such as hydroxylmethylcellulose or gelatin-microcapsule and poly-(methylmethacrylate) microcapsule. The pharmaceutical compositions of the invention may also be prepared in other drug delivery systems such as liposomes, albumin microspheres, microemulsions, nano-particles, and nanocapsules. Such techniques are described in Remington: The Science and Practice of Pharmacy 22^{th} edition (2012). The pharmaceutical compositions to be used for in vivo administration must be sterile. This is readily accomplished by filtration through sterile filtration membranes.

The pharmaceutical compositions of the invention may also be prepared as a sustained-release formulation. Suitable examples of sustained-release preparations include semipermeable matrices of solid hydrophobic polymers containing the polypeptides of the invention. Examples of sustained release matrices include polyesters, hydrogels, polyactides, copolymers of L-glutamic acid and γ ethyl-L-glutamate, non-degradable ethylene-vinyl acetate, degradable lactic acid-glycolic acid copolymers such as LUPRON DEPOT^{™}, and poly-D-(-)-3-hydroxybutyric acid. Some sustained-release formulations enable release of molecules over a few months, e.g., one to six months, while other formulations release pharmaceutical compositions of the invention for shorter time periods, e.g., days to weeks.

The pharmaceutical composition may be formed in a unit dose form as needed. The amount of active component, e.g., a polypeptide of the invention, included in the pharmaceutical preparations is such that a suitable dose within the designated range is provided (e.g., a dose within the range of 0.01-100 mg/kg of body weight).

The pharmaceutical composition for gene therapy can be in an acceptable diluent, or can include a slow release matrix in which the gene delivery vehicle is imbedded. If hydrodynamic injection is used as the delivery method, the pharmaceutical composition containing a nucleic acid molecule encoding a polypeptide described herein or a vector (e.g., a viral vector) containing the nucleic acid molecule is delivered rapidly in a large fluid volume intravenously. Vectors that may be used as in vivo gene delivery vehicle include, but are not limited to, retroviral vectors, adenoviral vectors, poxviral vectors (e.g., vaccinia viral vectors, such as Modified Vaccinia Ankara), adeno-associated viral vectors, and alphaviral vectors.

### IX. Routes, dosage, and administration

Pharmaceutical compositions that include the polypeptides of the invention as the therapeutic proteins may be formulated for, e.g., intravenous administration, parenteral administration, subcutaneous administration, intramuscular administration, intra-arterial administration, intrathecal administration, or intraperitoneal administration. The pharmaceutical composition may also be formulated for, or administered via, oral, nasal, spray, aerosol, rectal, or vaginal administration. For injectable formulations, various effective pharmaceutical carriers are known in the art. See, e.g., ASHP Handbook on Injectable Drugs, Toissel, 18th ed. (2014).

In some embodiments, a pharmaceutical composition that includes a nucleic acid molecule encoding a polypeptide of the invention or a vector containing such nucleic acid molecule may be administered by way of gene delivery. Methods of gene delivery are well-known to one of skill in the art. Vectors that may be used for in vivo gene delivery and expression include, but are not limited to, retroviral vectors, adenoviral vectors, poxviral vectors (e.g., vaccinia viral vectors, such as Modified Vaccinia Ankara (MVA)), adeno-associated viral vectors, and alphaviral vectors. In some embodiments, mRNA molecules encoding polypeptides of the invention may be administered directly to a subject.

In some embodiments of the present invention, nucleic acid molecules encoding a polypeptide described herein or vectors containing such nucleic acid molecules may be administered using a hydrodynamic injection platform. In the hydrodynamic injection method, a nucleic acid molecule encoding a polypeptide described herein is put under the control of a strong promoter in an engineered plasmid (e.g., a viral plasmid). The plasmid is often delivered rapidly in a large fluid volume intravenously. Hydrodynamic injection uses controlled hydrodynamic pressure in veins to enhance cell permeability such that the elevated pressure from the rapid injection of the large fluid volume results in fluid and plasmid extravasation from the vein. The expression of the nucleic acid molecule is driven primarily by the liver. In mice, hydrodynamic injection is often performed by injection of the plasmid into the tail vein. In certain embodiments, mRNA molecules encoding a polypeptide described herein may be administered using hydrodynamic injection.

The dosage of the pharmaceutical compositions of the invention depends on factors including the route of administration, the disease to be treated, and physical characteristics, e.g., age, weight, general health, of the subject. A pharmaceutical composition of the invention may include a dosage of a polypeptide of the invention ranging from 0.01 to 500 mg/kg (e.g., 0.01, 0.1, 0.2, 0.3, 0.4, 0.5, 1, 2, 3, 4, 5, 10, 15, 20, 25, 30, 35, 40, 45, 50, 100, 150, 200, 250, 300, 350, 400, 450, or 500 mg/kg) and, in a more specific embodiment, about 0.1 to about 30 mg/kg and, in a more specific embodiment, about 0.3 to about 30 mg/kg. The dosage may be adapted by the physician in accordance with conventional factors such as the extent of the disease and different parameters of the subject.

The pharmaceutical compositions are administered in a manner compatible with the dosage formulation and in such amount as is therapeutically effective to result in an improvement or remediation of the symptoms. The pharmaceutical compositions are administered in a variety of dosage forms, e.g., intravenous dosage forms, subcutaneous dosage forms, and oral dosage forms (e.g., ingestible solutions, drug release capsules). Generally, therapeutic proteins are dosed at 0.1-100 mg/kg, e.g., 1-50 mg/kg. Pharmaceutical compositions that include a polypeptide of the invention may be administered to a subject in need thereof, for example, one or more times (e.g., 1-10 times or more) daily, weekly, biweekly, monthly, bimonthly, quarterly, biannually, annually, or as medically necessary. In some embodiments, pharmaceutical compositions that include a polypeptide of the invention may be administered to a subject in need thereof weekly, biweekly, monthly, bimonthly, or quarterly. Dosages may be provided in either a single or multiple dosage regimens. The timing between administrations may decrease as the medical condition improves or increase as the health of the patient declines.

### X. Methods of treatment

The invention is based on the discovery that substituting amino acids from the extracellular portion of ActRllb into the extracellular portion ActRlla yields ActRlla variants with improved properties. The ActRlla variants generated by introducing residues from ActRllb into ActRlla retain the beneficial properties of ActRlla, such as longer serum half-life and low binding affinity to BMP9, and gain some of the beneficial properties of ActRllb, such as increased binding to activins A and B (see Table 4) and an ability to increase muscle mass (see Examples 1-3 and 5-6). These ActRlla variant properties make for a useful therapeutic that can compete with endogenous activin receptors for ligand binding. As the ActRlla variants contain the extracellular portion of the receptor, they will be soluble and able to bind to and sequester ligands (e.g., activins A and B, myostatin, GDF11) without activating intracellular signaling pathways. Therefore, the extracellular ActRlla variants can be used to treat diseases or conditions in which elevated activin signaling has been implicated (e.g., associated with increased expression of activin receptors or activin receptor ligands). For example, loss of myostatin has been shown to increase skeletal muscle mass, suggesting that myostatin inhibits skeletal muscle growth. It follows that treatment with a therapeutic agent that binds to myostatin and reduces its interaction with endogenous receptors could be a viable approach for increasing muscle mass. Indeed, extracellular ActRlla variants of the invention increase muscle mass in mice (see Examples 1-3 and 5-6). These data indicate that the extracellular ActRlla variants described herein can be used to increase muscle mass and treat subjects with diseases or conditions that result in muscle weakness or atrophy.

Moreover, these data provide a compelling reason to use the extracellular ActRlla variants of the invention to treat other diseases or conditions associated with elevated expression of activin receptors or activin receptor ligands, such as metabolic diseases (e.g., obesity, Type-1 diabetes, and Type-2 diabetes). Many studies have shown that increasing muscle mass is one way to reduce body fat and/or body weight, indicating that the extracellular ActRlla variants described herein can be used to treat metabolic diseases (e.g., obesity, Type-1 diabetes, and Type-2 diabetes) indirectly by increasing muscle mass. However, as activin receptors and activin receptor ligands have been shown to be increased in in obese mice and humans, the extracellular ActRlla variants described herein can be used to treat obesity by reducing elevated activin receptor signaling (e.g., by binding to and sequestering endogenous activin receptor ligands, e.g., activins and myostatin).

The invention provides compositions and methods of treatment that may be used to increase muscle mass and strength in a subject in need thereof. In some embodiments, the subject may have a disease that results in muscle weakness or atrophy (e.g., skeletal muscle weakness or atrophy). In some embodiments, the methods described herein are directed to affecting myostatin, activin, and/or BMP9 signaling in a subject having a disease or condition involving weakness and atrophy of muscles. In some embodiments, a polypeptide including an extracellular ActRlla variant described herein reduces or inhibits the binding of myostatin, activin, and/or BMP9 to their receptors, e.g., ActRlla, ActRllb, and BMPRII (e.g., ActRlla). In some embodiments, affecting myostatin, activin, and/or BMP9 signaling (e.g., reducing or inhibiting the binding of myostatin, activin, and/or BMP9 to their receptors, e.g., ActRlla, ActRllb, and BMPRII (e.g., ActRlla)) results in an increase in the subject's muscle mass.

In some embodiments, the polypeptides described herein (e.g., a polypeptide including an extracellular ActRlla variant (e.g., an extracellular ActRlla variant having the sequence of any one of SEQ ID NOs: 1-72 (e.g., SEQ ID NOs: 6-72)) may be administered to a subject to increase muscle mass, or to affect myostatin, activin, and/or BMP9 signaling in the subject. In some embodiments, the methods described herein increase bone mineral density of the subject. In some embodiments, the methods described herein do not cause any vascular complications in the subject, such as increased vascular permeability or leakage. In some embodiments of the methods described herein, the subject has a disease or condition involving weakness and atrophy of muscles (e.g., Duchenne muscular dystrophy (DMD), facioscapulohumeral muscular dystrophy (FSHD), inclusion body myositis (IBM), amyotrophic lateral sclerosis (ALS), sarcopenia, or cancer cachexia).

The invention also includes methods of treating a subject having Duchenne muscular dystrophy (DMD), facioscapulohumeral muscular dystrophy (FSHD), inclusion body myositis (IBM), amyotrophic lateral sclerosis (ALS), sarcopenia, or cancer cachexia by administering to the subject a polypeptide described herein (e.g., a polypeptide including an extracellular ActRlla variant (e.g., an extracellular ActRlla variant having the sequence of any one of SEQ ID NOs: 1-72 (e.g., SEQ ID NOs: 6-72)).

The compositions and methods described herein can also be used to treat and/or prevent medical conditions, such as metabolic diseases, e.g., obesity and diabetes (Type-1 and Type-2 diabetes). In some embodiments, the subject may have a disease that results in obesity. In some embodiments, the methods described herein are directed to affecting myostatin, activin, and/or BMP9 signaling in a subject having obesity, diabetes (Type-1 and Type-2 diabetes), or a disease or condition that results in obesity. In some embodiments, a polypeptide including an extracellular ActRlla variant described herein reduces or inhibits the binding of myostatin, activin, and/or BMP9 to their receptors, e.g., ActRlla, ActRllb, and BMPRII (e.g., ActRlla). In some embodiments, affecting myostatin, activin, and/or BMP9 signaling (e.g., reducing or inhibiting the binding of myostatin, activin, and/or BMP9 to their receptors, e.g., ActRlla, ActRllb, and BMPRII (e.g., ActRlla)) results in a reduction in the subject's body fat (e.g., amount of body fat or body fat percentage), a reduction in the subject's body weight or body weight gain, a reduction in fasting insulin levels, an increase in glucose clearance, or an increase in insulin sensitivity (e.g., a reduction in insulin resistance).

In some embodiments, the polypeptides described herein (e.g., a polypeptide including an extracellular ActRlla variant (e.g., an extracellular ActRlla variant having the sequence of any one of SEQ ID NOs: 1-72 (e.g., SEQ ID NOs: 6-72)) may be administered to a subject to prevent the development of obesity (e.g., in patients at risk of developing obesity, e.g., patients who are overweight, who have a family history of obesity, or who have other medical conditions or genetic risk factors linked to increased risk of obesity) and/or to treat patients already diagnosed with obesity. For example, administration of the extracellular ActRlla variant (e.g., an extracellular ActRlla variant having the sequence of any one of SEQ ID NOs: 1-72 (e.g., SEQ ID NOs: 6-72)) to a subject may help to reduce the body weight of the subject by decreasing the amount of fat. In some embodiments, the extracellular ActRlla variant decreases the amount of fat while maintaining or increasing the amount of lean mass.

In some embodiments, the polypeptides described herein (e.g., a polypeptide including an extracellular ActRlla variant (e.g., an extracellular ActRlla variant having the sequence of any one of SEQ ID NOs: 1-72 (e.g., SEQ ID NOs: 6-72)) may be used to prevent the development of diabetes (e.g., Type-1 and Type-2 diabetes) and/or to treat patients already diagnosed with diabetes. Patients who are likely to develop diabetes, e.g., individuals with genetic predisposition, family history of diabetes, prediabetes, association with other autoimmune diseases, or other metabolic diseases, may be administered the polypeptides described herein (e.g., a polypeptide including an extracellular ActRlla variant (e.g., an extracellular ActRlla variant having the sequence of any one of SEQ ID NOs: 1-72 (e.g., SEQ ID NOs: 6-72)) prophylactically, such that the extracellular ActRlla polypeptides may maintain the normal function and health of β-cells and prevent or delay the autoimmune inflammatory damage to β-cells. In other embodiments, the polypeptides described herein (e.g., a polypeptide including an extracellular ActRlla variant (e.g., an extracellular ActRlla variant having the sequence of any one of SEQ ID NOs: 1-72 (e.g., SEQ ID NOs: 6-72)) may be administered to individuals before they would be diagnosed with diabetes (e.g., Type-1 and Type-2 diabetes) or develop clinical symptoms of diabetes, e.g., high blood glucose level, high fasting insulin level, insulin resistance, polyuria, polydipsia, and polyphagia. In some embodiments, the extracellular ActRlla polypeptides may be administered to patients prior to the patients needing insulin. In yet other embodiments, the administration of extracellular ActRlla polypeptides may delay or postpone the need for insulin treatment in diabetic patients. For example, administration of the extracellular ActRlla polypeptides of the invention to a subject may help to increase the rate of glucose clearance from the blood.

In some embodiments, the polypeptides described herein (e.g., a polypeptide including an extracellular ActRlla variant (e.g., an extracellular ActRlla variant having the sequence of any one of SEQ ID NOs: 1-72 (e.g., SEQ ID NOs: 6-72)) may be administered to a subject to prevent the development of and/or treat patients with obesity or diabetes (e.g., Type-1 and Type-2 diabetes), or to affect myostatin, activin, and/or BMP9 signaling in the subject (e.g., to reduce or inhibit the binding of activin, myostatin, and/or BMP9 to their receptors). In some embodiments, the methods described herein reduce body fat (e.g., reduce the amount of subcutaneous and/or visceral fat, reduce adiposity, reduce the weights of epididymal and perirenal fat pads, or reduce body fat percentage). In some embodiments, the methods described herein reduce body weight or reduce body weight gain (e.g., reduce the percentage of body weight gain). In some embodiments, the methods described herein reduce the proliferation of adipose cells. In some embodiments, the methods described herein reduce LDL. In some embodiments, the methods described herein reduce triglycerides. In some embodiments, the methods described herein improve the serum lipid profile of the subject. In some embodiments, the methods described herein reduce body fat without reducing lean mass (e.g., do not affect lean mass or increase lean mass). In some embodiments, the methods described herein reduce body fat and increase muscle mass. In some embodiments, the methods described herein reduce blood glucose levels (e.g., fasting glucose levels) or and/or increase glucose clearance. In some embodiments, the methods described herein reduce fasting insulin levels and/or improve insulin sensitivity (e.g., reduce insulin resistance). In some embodiments, the methods described herein regulate insulin biosynthesis and/or secretion from β-cells. In some embodiments, the methods described herein do not affect the appetite for food intake. In some embodiments, the methods described herein do not cause any vascular complications in the subject, such as increased vascular permeability or leakage.

In some embodiments, the polypeptides described herein (e.g., a polypeptide including an extracellular ActRlla variant (e.g., an extracellular ActRlla variant having the sequence of any one of SEQ ID NOs: 1-72 (e.g., SEQ ID NOs: 6-72)) decrease body fat, decrease body weight, or increase insulin sensitivity and/or glucose clearance by increasing muscle mass.

In any of the methods described herein, a polypeptide including an extracellular ActRlla variant (e.g., an extracellular ActRlla variant having the sequence of any one of SEQ ID NOs: 1-71 (e.g., SEQ ID NOs: 6-71)) that further includes a C-terminal extension of one to six amino acids (e.g., 1, 2, 3, 4, 5, 6 or more amino acids) may be used as the therapeutic protein. In any of the methods described herein, a dimer (e.g., homodimer or heterodimer) of a polypeptide including an extracellular ActRlla variant (e.g., an extracellular ActRlla variant having the sequence of any one of SEQ ID NOs: 1-72 (e.g., SEQ ID NOs: 6-72)) fused to a moiety (e.g., an Fc domain monomer, a wild-type Fc domain, an Fc domain with amino acid substitutions (e.g., one more substitutions that reduce dimerization), an albumin-binding peptide, a fibronectin domain, or a human serum albumin) may be used as the therapeutic protein. Nucleic acids encoding the polypeptides described herein, or vectors containing said nucleic acids can also be administered according to any of the methods described herein. In any of the methods described herein, the polypeptide, nucleic acid, or vector can be administered as part of a pharmaceutical composition.

### EXAMPLES

### Example 1 - Effect of extracellular ActRlla variants on body weight

C57BI/6 mice received a single hydrodynamic injection of a plasmid construct encoding one of the following six polypeptides (n = 10/group):
(1) human Fc (hFc),
(2) extracellular ActRlla (SEQ ID NO: 73) fused to the N-terminus of hFc through a GGG linker;
(3) extracellular ActRllb (SEQ ID NO: 74) fused to the N-terminus of hFc through a GGG linker;
(4) extracellular ActRlla variant (SEQ ID NO: 69) fused to the N-terminus of hFc through a GGG linker; and
(5) extracellular ActRllb variant (SEQ ID NO: 149) fused to the N-terminus of hFc through a GGG linker.

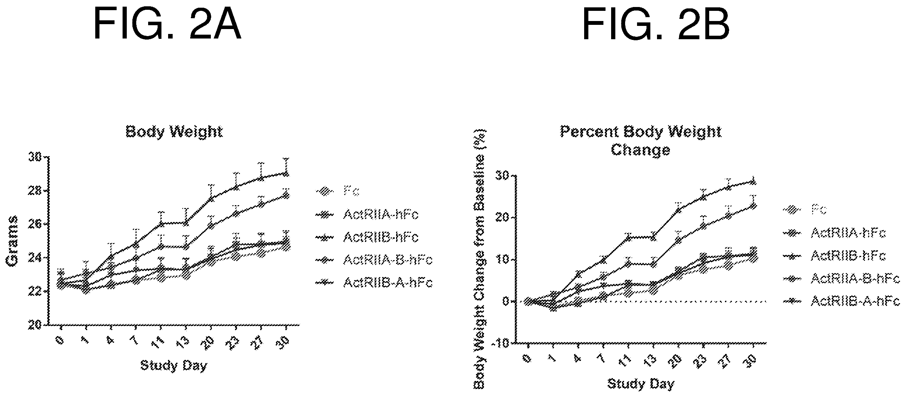

100 µg of plasmid construct was delivered in a volume of 10% body weight over 5-8 seconds. The high volume and short period of injection provides the pressure needed to introduce the plasmid into the liver cells where the plasmid will be expressed, specifically the proteins of interest are expressed under a strong and ubiquitous promoter. The protein of interest is secreted under the endogenous machinery of the liver cells and circulates freely. Mice were weighted twice weekly for 30 days and measurements were recorded as absolute body weight (BW) in grams and as a percent of body weight change from baseline measurements (FIGS. 2A and 2B, respectively).

### Example 2 - Effect of extracellular ActRlla variants on muscle mass

Mice received a single hydrodynamic injection of a plasmid construct encoding one of the following six polypeptides (n = 10/group):
(1) human Fc (hFc),
(2) extracellular ActRlla (SEQ ID NO: 73) fused to the N-terminus of hFc through a GGG linker;
(3) extracellular ActRllb (SEQ ID NO: 74) fused to the N-terminus of hFc through a GGG linker;
(4) extracellular ActRlla variant (SEQ ID NO: 69) fused to the N-terminus of hFc through a GGG linker; and
(5) extracellular ActRllb variant (SEQ ID NO: 149) fused to the N-terminus of hFc through a GGG linker.

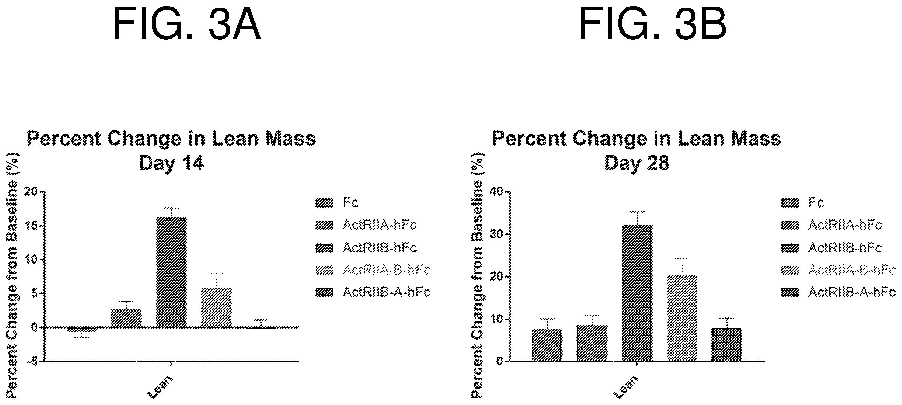

100 µg of plasmid construct was delivered in a volume of 10% body weight over 5-8 seconds. The high volume and short period of injection provides the pressure needed to introduce the plasmid into the liver cells where the plasmid will be expressed, specifically the proteins of interest are expressed under a strong and ubiquitous promoter. The protein of interest is secreted under the endogenous machinery of the liver cells and circulates freely. On study days 0 (baseline), 14, and 28, mice underwent NMR analysis for determination of lean mass using a MiniSpec LF90 NMR analyzer (Bruker, Woodlands, TX). The percent lean mass changes from baseline were recorded on days 14 and 28 (FIGS. 3A and 3B).

### Example 3 - Effect of extracellular ActRlla variants on body weight when administered as purified recombinant protein

Female C57BI/6 mice (Taconic Biosciences, Hudson NY) received an intraperitoneal injection of tris-buffered saline vehicle or one of the following five purified recombinant polypeptides at a dosage of 10 mg/kg twice weekly for four weeks (n = 10/group):
(1) tris-buffered saline vehicle,
(2) extracellular ActRlla (SEQ ID NO: 73) fused to the N-terminus of hFc through a GGG linker;
(3) extracellular ActRllb (SEQ ID NO: 74) fused to the N-terminus of hFc through a GGG linker;
(4) extracellular ActRlla/b variant (SEQ ID NO: 69) fused to the N-terminus of hFc through a GGG linker;
(5) extracellular ActRIIa/bΔ9 variant (SEQ ID NO: 58) fused to the N-terminus of hFc through a GGG linker; and
(6) extracellular ActRII a/bΔ9 min variant (SEQ ID NO: 6) fused to the N-terminus of hFc through a GGG linker.

Purified recombinant protein was made by transient expression in HEK293 cell and purified from the conditioned media using Protein-A Sepharose chromatography.

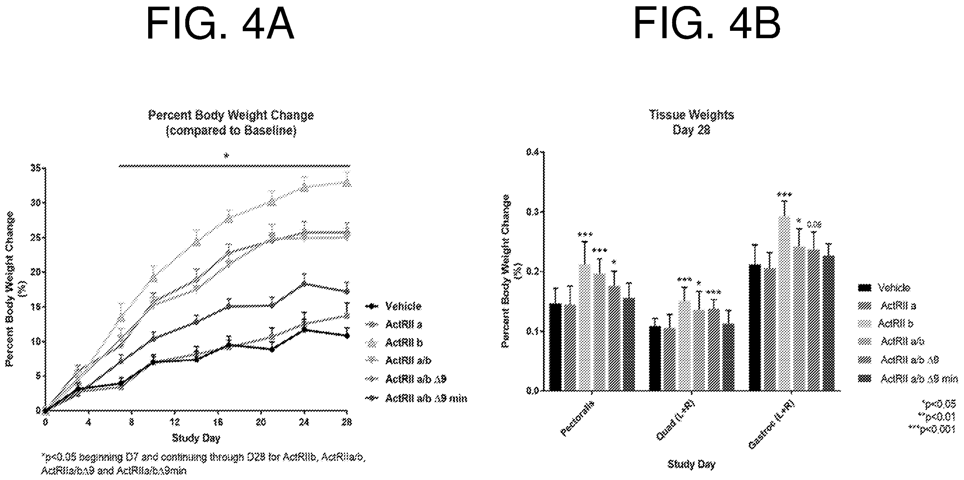

Following four weeks of dosing the mice were humanely sacrificed and necropsy was performed. Necropsy included collection of weights for total body, and the gastrocnemius, pectoralis, and quadriceps muscles. Statistical analysis of muscle/body weight data was performed in GraphPad Prism 7 (GraphPad Software, La Jolla CA) (FIGS. 4A and 4B, respectively).

### Example 4 - Evaluation of ActRlla variants binding affinity by surface plasmon resonance (SPR)

The Biacore 3000 was used to measure the kinetics of the interactions between the ActRlla variants and the ligands Activin A, Activin B, growth differentiation factor 11 (GDF11), and BMP-9. ActRlla variants were expressed and purified according to the methodology described in Example 3. The ActRlla variants were immobilized on the chip (CM4 or CM5) with capture antibodies (anti-mouse from GEGE) in flow cells 2-4 to ensure proper orientation. Flow cell 1 was used as a reference cell to subtract any nonspecific binding and bulk effects. HBS-EP+ buffer from GE Healthcare^{™} was used as a running buffer. Each ligand was run in a concentration series at 40 µl/min to avoid mass transport effects. The data was analyzed using Scrubber2 by BioLogic^{™} Software to calculate the K_{D} of each interaction (Table 4).

**Table 4: Comparison of ActRlla variant binding affinity (K_{D}) to various ligands**

| | **Activin A (K_{D})** | **Activin B (K_{D})** | **GDF-11 (K_{D})** | **BMP-9 (K_{D})** |
|---|---|---|---|---|
| **Vehicle** | **N/A** | **N/A** | **N/A** | **N/A** |
| **ActRIIa (SEQ ID NO: 73)** | 1 nM | 373 pM | 81 pM | 25 nM |
| **ActRIIb (SEQ ID NO: 74)** | 63 pM | 23 pM | 115 pM | 278 pM |
| **ActRlla/b variant (SEQ ID NO: 69)** | 542 pM | 103 pM | 186 pM | 4nM |
| **ActRIIb/a variant (SEQ ID NO: 149)** | No Binding | No Binding | No Binding | No Binding |
| **ActRIIa/bΔ9 variant (SEQ ID NO: 58)** | 213 pM | 12.3 pM | 115 pM | 10 nM |
| **ActRIIa/bΔ9 min variant (SEQ ID NO: 6)** | 310 pM | 88 pM | 114 pM | 17 nM |
| **ActRIIa/b+ variant (SEQ ID NO: 150)** | 242 pM | 282 pM | No dissociation | 26 nM |
| **ActRIIa/bΔ9m2 variant (SEQ ID NO: 38)** | 170 pM | 104 pM | 222 pM | 13-18 nM |
| **ActRIIa/bΔ9m3 variant (SEQ ID NO: 41)** | 71 pM | 72.5 pM | 117 pM | 1.2 nM |
| **ActRIIa/bΔ9m4 variant (SEQ ID NO: 44)** | 375 pM | 254 pM | 394 pM | 14-20 nM |
| **ActRIIa/bmax1 variant (SEQ ID NO: 70)** | 232 pM | 97 pM | 236 pM | 5.6 nM |
| **ActRIIa/bmax2 variant (SEQ ID NO: 71)** | 135 pM | 39 pM | 113 pM | 5 nM |
| **ActRIIa/bmax3 variant (SEQ ID NO: 72)** | 89 pM | 43 pM | 214 pM | 3.3 nM |

| | | | | |
|---|---|---|---|---|
| *Not done in HDI, but recombinant protein demonstrates the BW result is similar to ActRlla/b | | | | |

### Example 5 - Effect of extracellular ActRlla variants on body and muscle weight

C57BI/6 mice received a single hydrodynamic injection of a plasmid construct encoding one of the following twelve polypeptides (n = 10/group):
(1) vehicle;
(2) pLEV113-ActRlla (19-127) (SEQ ID NO: 73) fused to the N-terminus of hFc through a GGG linker;
(3) pLEV113-ActRllb (41-155) (SEQ ID NO: 74) fused to the N-terminus of hFc through a GGG linker;
(4) pLEV113-ActRlla/b (SEQ ID NO: 69) fused to the N-terminus of hFc through a GGG linker;
(5) pLEV113-ActRllb/a (SEQ ID NO: 149) fused to the N-terminus of hFc through a GGG linker;
(6) pLEV113-ActRlla/b+ (SEQ ID NO: 150) fused to the N-terminus of hFc through a GGG linker;
(7) pLEV113-ActRlla/b-delta 9m2 (SEQ ID NO: 38) fused to the N-terminus of hFc through a GGG linker;
(8) pLEV113-ActRlla/b-delta 9m3 (SEQ ID NO: 41) fused to the N-terminus of hFc through a GGG linker;
(9) pLEV113-ActRlla/b-delta 9m4 (SEQ ID NO: 44) fused to the N-terminus of hFc through a GGG linker;
(10) pLEV113-ActRlla/bmax1 (SEQ ID NO: 70) fused to the N-terminus of hFc through a GGG linker;
(11) pLEV113-ActRlla/bmax2 (SEQ ID NO: 71) fused to the N-terminus of hFc through a GGG linker; and
(12) pLEV113-ActRlla/bmax1 (SEQ ID NO: 72) fused to the N-terminus of hFc through a GGG linker.

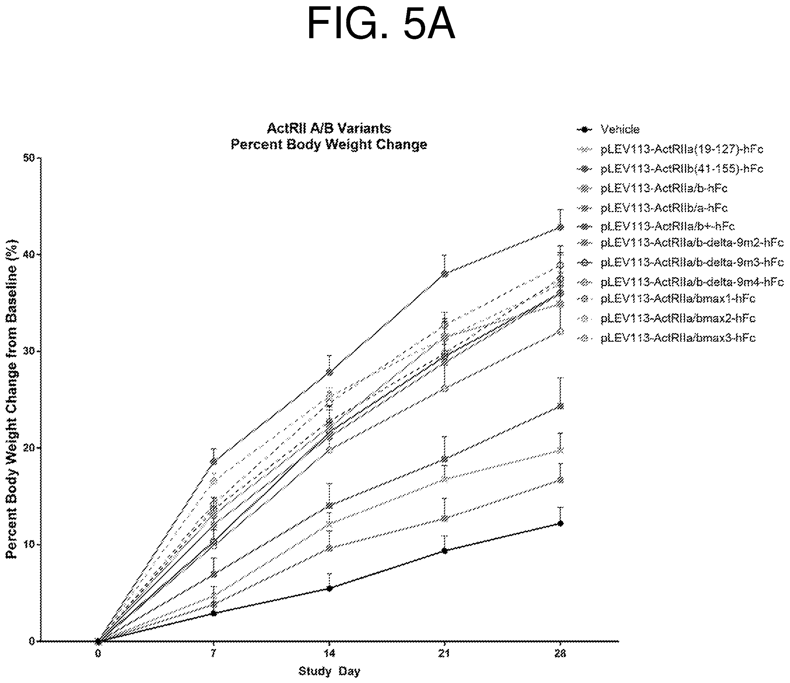

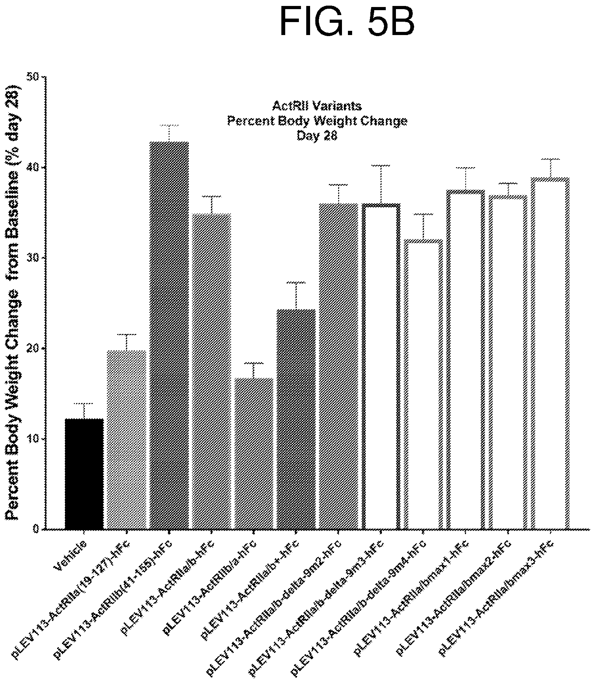

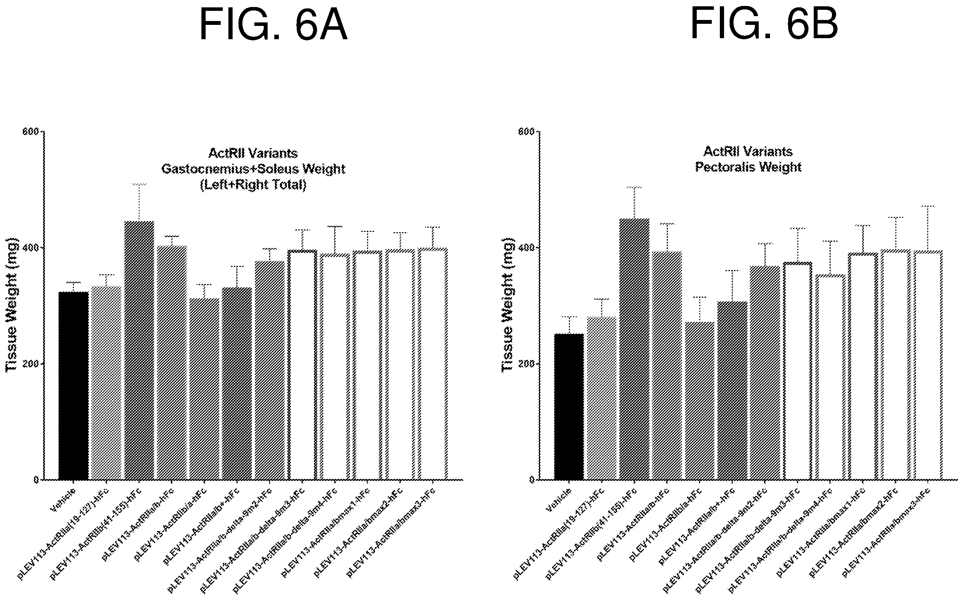

100 µg of plasmid construct was delivered in a volume of 10% body weight over 5-8 seconds. The high volume and short period of injection provides the pressure needed to introduce the plasmid into the liver cells where the plasmid will be expressed, specifically the proteins of interest are expressed under a strong and ubiquitous promoter. The protein of interest is secreted under the endogenous machinery of the liver cells and circulates freely. Mice were weighted twice weekly for 30 days and measurements were recorded as absolute body weight (BW) in grams and as a percent of body weight change from baseline measurements (FIGS. 5A and 5B, respectively). Muscles were also weighed at the end of the study and measurements were recorded in grams (FIGS. 6A and 6B).

### Example 6 - Dose effect of extracellular ActRlla variants on body weight, muscle weight, and muscle mass

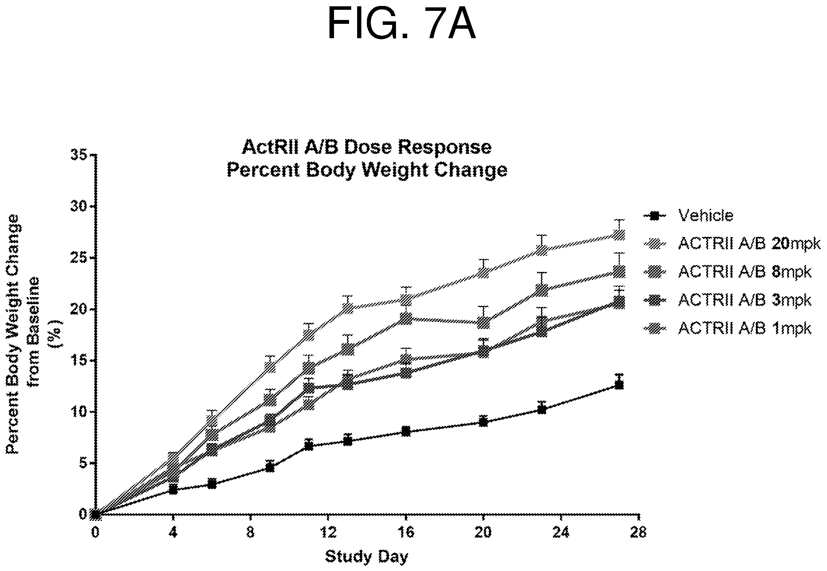

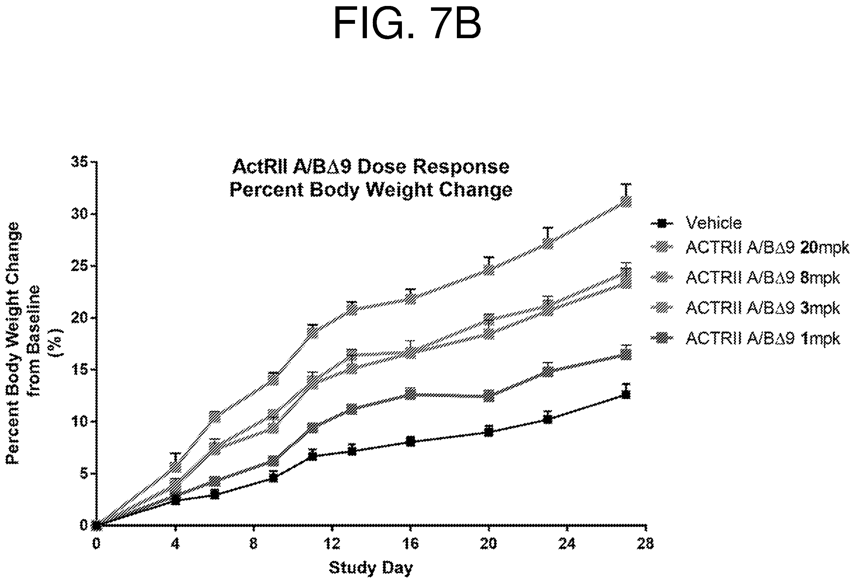

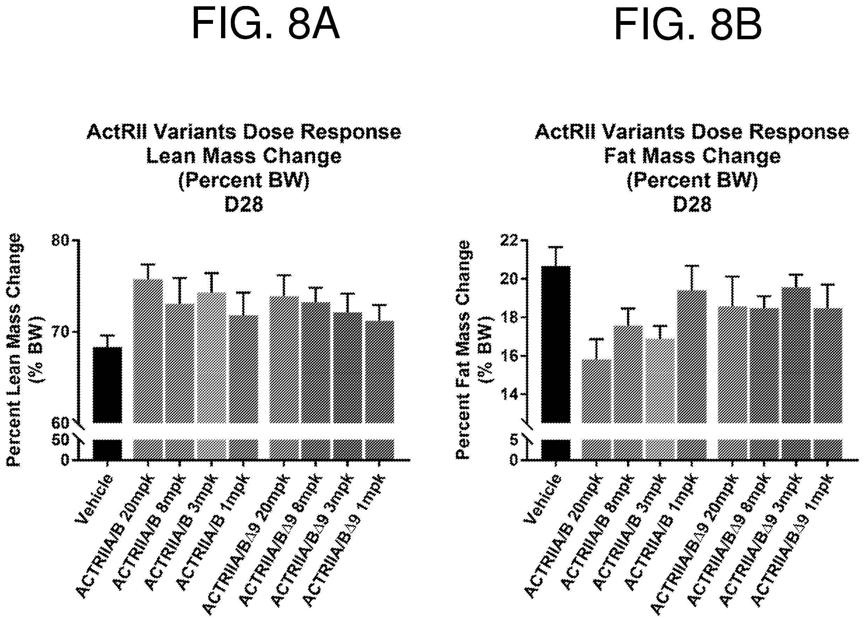

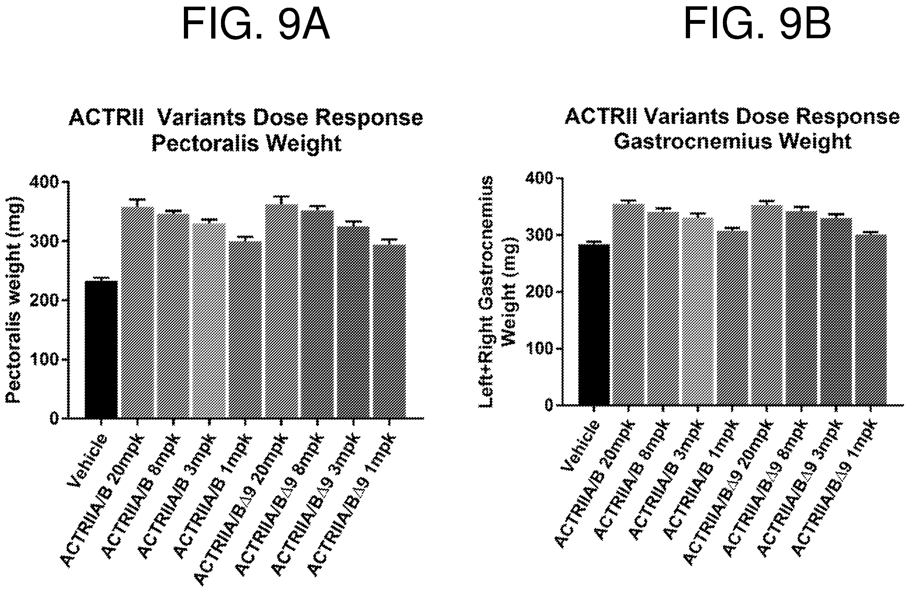

8-week old, male C57BL/6 mice were weight-matched into 9 groups (n=10/group). Groups were dosed with 5 mL/kg of either vehicle (Tris-Buffered Saline, pH 7.4) or one of 4 concentrations of ActRIIA/B-Fc (SEQ ID NO: 69 fused to the N-terminus of hFc through a GGG linker) or ActRIIA/BΔ9-Fc (SEQ ID NO: 58 fused to the N-terminus of hFc through a GGG linker). The doses evaluated were 20 mg/kg, 8 mg/kg, 3 mg/kg and 1 mg/kg. Treatments were administered intraperitoneally (IP) twice a week for 4 weeks (8 doses), and the study was terminated on study day 28. Body weights were recorded on dosing days throughout the study (FIGS. 7A and 7B), and at study termination, groups underwent NMR imaging for lean and fat mass analysis (FIGS. 8A and 8B) and had pectoralis and gastrocnemius muscles weights collected and weighed (FIGS. 9A and 9B).

### Example 7 - Effect of extracellular ActRlla variants on obesity

Adult male C57BL/6 mice are assigned to weight-matched treatment groups (n=10/group). All animals are maintained on either regular chow diet (Chow; Purina LabDiet 5001 ; St. Louis, MO) or high fat diet (HFD; Research Diets D12331; New Brunswick, NJ). Chow- and HFD-fed groups are further divided into groups that are dosed twice weekly with either ActRII variant or vehicle for a period of 60 d. Body weights are measured twice per week at the time of treatment. Body composition is measured using the MiniSpec LF50 at baseline (before administration of treatments and transfer to HFD) and then every other week until the end of the study. At the study termination date, tissues of interest (serum, plasma, muscles and fat depots) are surgically removed and weighed. Serum samples are subsequently evaluated for biomarkers of adiposity and plasma was evaluated for Hba1c levels.

### Other Embodiments

While the invention has been described in connection with specific embodiments thereof, it will be understood that it is capable of further modifications and this application is intended to cover any variations, uses, or adaptations of the invention following, in general, the principles of the invention and including such departures from the present disclosure come within known or customary practice within the art to which the invention pertains and may be applied to the essential features hereinbefore set forth.

All publications, patents, and patent applications are herein incorporated by reference in their entirety to the same extent as if each individual publication, patent or patent application was specifically and individually indicated to be incorporated by reference in its entirety.

Other embodiments are within the following claims.
The invention further provides the following numbered embodiments:
1. A polypeptide comprising an extracellular activin receptor type IIa (ActRlla) variant, the variant having a sequence of 
GAILGRSETQECLX1X2NANWX3X4X5X6TNQTGVEX7CX8GX9X10X11X12X13X14HCX15ATWX16NISGSIEIV
X17X18GCX19X20X21DX22NCYDRTDCVEX23X24X25X26PX27VYFCCCEGNMCNEKFSYFPEMEVTQPTS
(SEQ ID NO: 1),
   wherein X₁ is F or Y; X₂ is For Y; X₃ is E or A; X₄ is K or L; X₅ is D or E; X₆ is R or A; X₇ is P or R; X₈ is Y or E; X₉ is D or E; X₁₀ is K or Q; X₁₁ is D or A; X₁₂ is K or A; X₁₃ is R or A; X₁₄ is R or L; X₁₅ is F or Y; X₁₆ is K, R, or A; X₁₇ is K, A, Y, F, or I; X₁₈ is Q or K; X₁₉ is W or A; X₂₀ is L or A; X₂₁ is D, K, R, A, F, G, M, N, or I; X₂₂ is I, F, or A; X₂₃ is K or T; X₂₄ is K or E; X₂₅ is D or E; X₂₆ is S or N; and X₂₇ is E or Q, and
   wherein the variant has at least one amino acid substitution relative to a wild-type extracellular ActRlla having the sequence of SEQ ID NO: 73 or an extracellular ActRlla having any one of the sequences of SEQ ID NOs: 76-96.
2. The polypeptide of embodiment 1, wherein the variant has a sequence of 
GAILGRSETQECLFX2NANWX3X4X5X6TNQTGVEX7CX8GX9KX11X12X13X14HCX15ATWX16NISGSIEIVX17
X18GCX19X20X21DX22NCYDRTDCVEX23X24X25X26PX27VYFCCCEGNMCNEKFSYFPEMEVTQPTS (SEQ
ID NO: 2),
3. The polypeptide of embodiment 1 or 2, wherein the variant has a sequence of 
GAILGRSETQECLFX2NANWEX4X5RTNQTGVEX7CX8GX9KDKRX14HCX15ATWX16NISGSIEIVKX18GCWL
DDX22NCYDRTDCVEX23X24X25X26PX27VYFCCCEGNMCNEKFSYFPEMEVTQPTS (SEQ ID NO: 3).
4. The polypeptide of any one of embodiments1-3, wherein the variant has a sequence of 
GAILGRSETQECLFX2NANWEX4DRTNQTGVEX7CX8GX9KDKRX14HCX15ATWX16NISGSIEIVKX18GCWL
DDX22NCYDRTDCVEX23KX25X26PX27VYFCCCEGNMCNEKFSYFPEMEVTQPTS (SEQ ID NO: 4).
5. The polypeptide of any one of embodiments 1-4, wherein the variant has a sequence of 
GAILGRSETQECLFX2NANWEX4DRTNQTGVEPCX8GX9KDKRX14HCFATWKNISGSIEIVKX18GCWLDDI
NCYDRTDCVEX23KX25X26PX27VYFCCCEGNMCNEKFSYFPEMEVTQPTS (SEQ ID NO: 5).
6. The polypeptide of embodiment 1, wherein X₁ is F.
7. The polypeptide of embodiment 1, wherein X₁ is Y.
8. The polypeptide of embodiment 1, 6, or 7 wherein X₁₀ is K.
9. The polypeptide of embodiment 1, 6, or 7 wherein X₁₀ is Q.
10. The polypeptide of any one of embodiments 1-9, wherein X₂ is F.
11. The polypeptide of any one of embodiments 1-9, wherein X₂ is or Y.
12. The polypeptide of any one of embodiments 1, 2, and 6-11, wherein X₃ is E.
13. The polypeptide of any one of embodiments 1, 2, and 6-11, wherein X₃ is A.
14. The polypeptide of any one of embodiments 1-13, wherein X₄ is K.
15. The polypeptide of any one of embodiments 1-13, wherein X₄ is L.
16. The polypeptide of any one of embodiments 1, 2, 3, and 6-15, wherein X₅ is D.
17. The polypeptide of any one of embodiments 1, 2, 3, and 6-15, wherein X₅ is E.
18. The polypeptide of any one of embodiments 1, 2 and 6-17, wherein X₆ is R.
19. The polypeptide of any one of embodiments 1, 2 and 6-17, wherein X₆ is A.
20. The polypeptide of any one of embodiments 1-4 and 6-19, wherein X₇ is P.
21. The polypeptide of any one of embodiments 1-4 and 6-19, wherein X₇ is R.
22. The polypeptide of any one of embodiments 1-21, wherein X₈ is Y.
23. The polypeptide of any one of embodiments 1-21, wherein X₈ is E.
24. The polypeptide of any one of embodiments 1-23, wherein X₉ is D.
25. The polypeptide of any one of embodiments 1-23, wherein X₉ is E.
26. The polypeptide of any one of embodiments 1, 2 and 6-25, wherein X₁₁ is D.
27. The polypeptide of any one of embodiments 1, 2 and 6-25, wherein X₁₁ is A.
28. The polypeptide of any one of embodiments 1, 2 and 6-27, wherein X₁₂ is K.
29. The polypeptide of any one of embodiments 1, 2 and 6-27, wherein X₁₂ is A.
30. The polypeptide of any one of embodiments 1, 2 and 6-29, wherein X₁₃ is R.
31. The polypeptide of any one of embodiments 1, 2 and 6-29, wherein X₁₃ is A.
32. The polypeptide of any one of embodiments 1-31, wherein X₁₄ is R.
33. The polypeptide of any one of embodiments 1-31, wherein X₁₄ is L.
34. The polypeptide of any one of embodiments 1-4 and 6-33, wherein X₁₅ is F.
35. The polypeptide of any one of embodiments 1-4 and 6-33, wherein X₁₅ is Y.
36. The polypeptide of any one of embodiments 1-4 and 6-35, wherein X₁₆ is K.
37. The polypeptide of any one of embodiments 1-4 and 6-35, wherein X₁₆ is R.
38. The polypeptide of any one of embodiments 1-4 and 6-35, wherein X₁₆ is A.
39. The polypeptide of any one of embodiments 1, 2 and 6-38, wherein X₁₇ is K.
40. The polypeptide of any one of embodiments 1, 2 and 6-38, wherein X₁₇ is A.
41. The polypeptide of any one of embodiments 1, 2 and 6-38, wherein X₁₇ is Y.
42. The polypeptide of any one of embodiments 1, 2 and 6-38, wherein X₁₇ is F.
43. The polypeptide of any one of embodiments 1, 2 and 6-38, wherein X₁₇ is I.
44. The polypeptide of any one of embodiments 1-43, wherein X₁₈ is Q.
45. The polypeptide of any one of embodiments 1-43, wherein X₁₈ is K.
46. The polypeptide of any one of embodiments 1, 2 and 6-45, wherein X₁₉ is W.
47. The polypeptide of any one of embodiments 1, 2 and 6-45, wherein X₁₉ is A.
48. The polypeptide of any one of embodiments 1, 2 and 6-47, wherein X₂₀ is L.
49. The polypeptide of any one of embodiments 1, 2 and 6-47, wherein X₂₀ is A.
50. The polypeptide of any one of embodiments 1, 2 and 6-49, wherein X₂₁ is D.
51. The polypeptide of any one of embodiments 1, 2 and 6-49, wherein X₂₁ is K.
52. The polypeptide of any one of embodiments 1, 2 and 6-49, wherein X₂₁ is R.
53. The polypeptide of any one of embodiments 1, 2 and 6-49, wherein X₂₁ is A.
54. The polypeptide of any one of embodiments 1, 2 and 6-49, wherein X₂₁ is F.
55. The polypeptide of any one of embodiments 1, 2 and 6-49, wherein X₂₁ is G.
56. The polypeptide of any one of embodiments 1, 2 and 6-49, wherein X₂₁ is M.
57. The polypeptide of any one of embodiments 1, 2 and 6-49, wherein X₂₁ is N.
58. The polypeptide of any one of embodiments 1, 2 and 6-49, wherein X₂₁ is I.
59. The polypeptide of any one of embodiments 1-4 and 6-58, wherein X₂₂ is I.
60. The polypeptide of any one of embodiments 1-4 and 6-58, wherein X₂₂ is F.
61. The polypeptide of any one of embodiments 1-4 and 6-58, wherein X₂₂ is A.
62. The polypeptide of any one of embodiments 1-61, wherein X₂₃ is K.
63. The polypeptide of any one of embodiments 1-61, wherein X₂₃ is T.
64. The polypeptide of any one of embodiments 1, 2, 3, and 6-63, wherein X₂₄ is K.
65. The polypeptide of any one of embodiments 1, 2, 3, and 6-63, wherein X₂₄ is E.
66. The polypeptide of any one of embodiments 1-65, wherein X₂₅ is D.
67. The polypeptide of any one of embodiments 1-65, wherein X₂₅ is E.
68. The polypeptide of any one of embodiments 1-67, wherein X₂₆ is S.
69. The polypeptide of any one of embodiments 1-67, wherein X₂₆ is N.
70. The polypeptide of any one of embodiments 1-69, wherein X₂₇ is E.
71. The polypeptide of any one of embodiments 1-69, wherein X₂₇ is Q.
72. The polypeptide of any one of embodiments 1-71, wherein X₂₃ is T, X₂₄ is E, X₂₅ is E, and X₂₆ is N.
73. The polypeptide of any one of embodiments 1-71, wherein X₂₃ is T, X₂₄ is K, X₂₅ is E, and X₂₆ is N.
74. The polypeptide of any one of embodiments 1-73, wherein X₁₇ is K.
75. The polypeptide of embodiment 1, wherein the variant has the sequence of any one of SEQ ID NOs: 6-72
76. The polypeptide of any one of embodiments 1-75, wherein the amino acid at position X₂₄ is replaced with the amino acid K.
77. The polypeptide of any one of embodiments 1-75, wherein the amino acid at position X₂₄ is replaced with the amino acid E.
78. The polypeptide of any one of embodiments 1-77, further comprising a C-terminal extension of one or more amino acids.
79. The polypeptide of embodiment 78, wherein the C-terminal extension is NP.
80. The polypeptide of embodiment 78, wherein the C-terminal extension is NPVTPK.
81. The polypeptide of any one of embodiments 1-80, further comprising an Fc domain monomer fused to the C-terminus of the polypeptide by way of a linker.
82. The polypeptide of embodiment 81, wherein the Fc domain monomer comprises the sequence of SEQ ID NO: 97.
83. The polypeptide of any one of embodiments 1-80, further comprising a wild-type Fc domain fused to the C-terminus of the polypeptide by way of a linker.
84. The polypeptide of embodiment 83, wherein the wild-type Fc domain comprises the sequence of SEQ ID NO: 151.
85. The polypeptide of any one of embodiments 1-80, further comprising an Fc domain with amino acid substitutions fused to the C-terminus of the polypeptide by way of a linker.
86. The polypeptide of embodiment 85, wherein the Fc domain does not form a dimer.
87. The polypeptide of any one of embodiments 1-80, further comprising an albumin-binding peptide fused to the C-terminus of the polypeptide by way of a linker.
88. The polypeptide of embodiment 87, wherein the albumin-binding peptide comprises the sequence of SEQ **ID** NO: 152.
89. The polypeptide of any one of embodiments 1-80, further comprising a fibronectin domain fused to the C-terminus of the polypeptide by way of a linker.
90. The polypeptide of embodiment 89, wherein the fibronectin domain comprises the sequence of SEQ **ID** NO: 153.
91. The polypeptide of any one of embodiments 1-80, further comprising a human serum albumin fused to the C-terminus of the polypeptide by way of a linker.
92. The polypeptide of embodiment 91, wherein the human serum albumin comprises the sequence of SEQ **ID** NO: 154.
93. The polypeptide of embodiment 81 or 82, wherein the polypeptide forms a dimer.
94. The polypeptide of any one of embodiments 81-93, wherein the linker is an amino acid spacer.
95. The polypeptide of embodiment 94, wherein the amino acid spacer is GGG, GGGA (SEQ **ID** NO: 98), GGGG (SEQ **ID** NO: 100), GGGAG (SEQ **ID** NO: 130), GGGAGG (SEQ **ID** NO: 131), or GGGAGGG (SEQ **ID** NO: 132).
96. The polypeptide of any one of embodiments 1-95, wherein the polypeptide has a serum half-life of at least 7 days.
97. The polypeptide of any one of embodiments 1-96, wherein the polypeptide binds to human bone morphogenetic protein 9 (BMP9) with a K_{D} of 200 pM or higher.
98. The polypeptide of embodiment 97, wherein the polypeptide binds to activin and/or myostatin and has reduced or weak binding to human BMP9.
99. The polypeptide of embodiment 97 or 98, wherein the polypeptide does not substantially bind to human BMP9.
100. The polypeptide of any one of embodiments 1-99, wherein the polypeptide binds to human activin A with a K_{D} of 800 pM or less.
101. The polypeptide of any one of embodiments 1-100, wherein the polypeptide binds to human activin B with a K_{D} of 800 pM or less.
102. The polypeptide of any one of embodiments 1-101, wherein the polypeptide binds to human GDF-11 with a K_{D} of 5 pM or higher.
103. A nucleic acid molecule encoding a polypeptide of any of embodiments 1-102.
104. A vector comprising the nucleic acid molecule of embodiment 103.
105. A host cell that expresses a polypeptide of any one of embodiments 1-102, wherein the host cell comprises a nucleic acid molecule of embodiment 103 or a vector of embodiment 104, wherein the nucleic acid molecule or vector is expressed in the host cell.
106. A method of preparing a polypeptide of any one of embodiments 1-102, wherein the method comprising:
   a) providing a host cell comprising a nucleic acid molecule of embodiment 103 or a vector of embodiment 104, and
   b) expressing the nucleic acid molecule or vector in the host cell under conditions that allow for the formation of the polypeptide.
107. A pharmaceutical composition comprising a polypeptide of any one of embodiments 1-102, a nucleic acid molecule of embodiment 103, or a vector of embodiment 104, and one or more pharmaceutically acceptable carriers or excipients.
108. The pharmaceutical composition of embodiment 107, wherein the polypeptide is in a therapeutically effective amount.
109. A method of increasing muscle mass in a subject in need thereof, comprising administering to the subject a therapeutically effective amount of a polypeptide of any one of embodiments 1-102, a nucleic acid molecule of embodiment 103, a vector of embodiment 104, or a pharmaceutical composition of embodiment 107 or 108.
110. The method of embodiment 109, wherein the subject has Duchenne muscular dystrophy, facioscapulohumeral muscular dystrophy, inclusion body myositis, amyotrophic lateral sclerosis, sarcopenia, or cancer cachexia.
111. A method of affecting myostatin, activin, and/or BMP9 signaling in a subject having a disease or condition involving weakness and atrophy of muscles, comprising administering to the subject a therapeutically effective amount of a polypeptide of any one of embodiments 1-102, a nucleic acid molecule of embodiment 103, a vector of embodiment 104, or a pharmaceutical composition of embodiment 107 or 108.
112. The method of embodiment **111,** wherein the disease or condition is Duchenne muscular dystrophy, facioscapulohumeral muscular dystrophy, inclusion body myositis, amyotrophic lateral sclerosis, sarcopenia, or cancer cachexia.
113. A method of treating a subject having Duchenne muscular dystrophy, comprising administering to the subject a therapeutically effective amount of a polypeptide of any one of embodiments 1-102, a nucleic acid molecule of embodiment 103, a vector of embodiment 104, or a pharmaceutical composition of embodiment 107 or 108.
114. A method of treating a subject having facioscapulohumeral muscular dystrophy, comprising administering to the subject a therapeutically effective amount of a polypeptide of any one of embodiments 1-102, , a nucleic acid molecule of embodiment 103, a vector of embodiment 104, or a pharmaceutical composition of embodiment 107 or 108.
115. A method of treating a subject having inclusion body myositis, comprising administering to the subject a therapeutically effective amount of a polypeptide of any one of embodiments 1-102, a nucleic acid molecule of embodiment 103, a vector of embodiment 104, or a pharmaceutical composition of embodiment 107 or 108.
116. A method of treating a subject having amyotrophic lateral sclerosis, comprising administering to the subject a therapeutically effective amount of a polypeptide of any one of embodiments 1-102, a nucleic acid molecule of embodiment 103, a vector of embodiment 104, or a pharmaceutical composition of embodiment 107 or 108.
117. A method of reducing body fat in a subject in need thereof, comprising administering to the subject a therapeutically effective amount of a polypeptide of any one of embodiments 1-102, a nucleic acid molecule of embodiment 103, a vector of embodiment 104, or a pharmaceutical composition of embodiment 107 or 108.
118. A method of reducing body weight in a subject in need thereof, comprising administering to the subject a therapeutically effective amount of a polypeptide of any one of embodiments 1-102, a nucleic acid molecule of embodiment 103, a vector of embodiment 104, or a pharmaceutical composition of embodiment 107 or 108.
119. A method of reducing blood glucose in a subject in need thereof, comprising administering to the subject a therapeutically effective amount of a polypeptide of any one of embodiments 1-102, a nucleic acid molecule of embodiment 103, a vector of embodiment 104, or a pharmaceutical composition of embodiment 107 or 108.
120. A method of increasing insulin sensitivity in a subject in need thereof, comprising administering to the subject a therapeutically effective amount of a polypeptide of any one of embodiments 1-102, a nucleic acid molecule of embodiment 103, a vector of embodiment 104, or a pharmaceutical composition of embodiment 107 or 108.
121. The method of any one of embodiments 117-120, wherein the subject has or is at risk of developing a metabolic disease.
122. A method of affecting myostatin, activin, and/or BMP9 signaling in a subject having or at risk of developing a metabolic disease, comprising administering to the subject a therapeutically effective amount of a polypeptide of any one of embodiments 1-102, a nucleic acid molecule of embodiment 103, a vector of embodiment 104, or a pharmaceutical composition of embodiment 107 or 108.
123. A method of treating and/or preventing a metabolic disease in a subject, said method comprising administering to said subject a therapeutically effective amount of a polypeptide of any one of embodiments 1-102, a nucleic acid molecule of embodiment 103, a vector of embodiment 104, or a pharmaceutical composition of embodiment 107 or 108.
124. The method of any one of embodiments 121-123, wherein the metabolic disease is selected from the group consisting of obesity, Type-1 diabetes, and Type-2 diabetes.
125. The method of embodiment 124, wherein the metabolic disease is obesity.
126. The method of embodiment 124, wherein the metabolic disease is Type-1 diabetes.
127. The method of embodiment 124, wherein the metabolic disease is Type-2 diabetes.
128. The method of any one of embodiments 117-127, wherein the method reduces body weight and/or percentage of body weight gain of said subject.
129. The method of any one of embodiments 117-128, wherein the method reduces amount of body fat and/or percentage of body fat of said subject.
130. The method of any one of embodiments 117-129, wherein the method does not affect the appetite for food intake of said subject.
131. The method of any one of embodiments 117-130, wherein the method reduces adiposity of said subject.
132. The method of any one of embodiments 117-131, wherein the method reduces the weights of epididymal and perirenal fat pads of said subject.
133. The method of any one of embodiments 117-132, wherein the method reduces the amount of subcutaneous and/or visceral fat of said subject.
134. The method of any one of embodiments 117-133, wherein the method lowers the level of fasting insulin of said subject.
135. The method of any one of embodiments 117-134, wherein the method lowers the level of blood glucose of said subject.
136. The method of any one of embodiments 117-135, wherein the method increases insulin sensitivity of said subject.
137. The method of any one of embodiments 117-136, wherein the method increases the rate of glucose clearance of said subject.
138. The method of any one of embodiments 117-137, wherein the method improves the serum lipid profile of said subject.
139. The method of any one of embodiments 117-138, wherein the method does not reduce lean mass.
140. The method of any one of embodiments 109-139, wherein the method increases muscle mass.
141. The method of any one of embodiments 109-140, wherein the method reduces or inhibits the binding of activin and/or myostatin to their receptors.
142. The method of any one of embodiments 109-116 and 140-141, wherein the polypeptide, nucleic acid, vector, or pharmaceutical composition is administered in an amount sufficient to increase muscle mass and/or strength, affect myostatin, activin, and/or BMP9 signaling in the subject, or reduce or inhibit the binding of activin and/or myostatin to their receptors.
143. The method of any one of embodiments 117-141, wherein the polypeptide, nucleic acid, vector, or pharmaceutical composition is administered in an amount sufficient to reduce body fat, reduce the amount of subcutaneous fat, reduce the amount of visceral fat, reduce adiposity, reduce the weights of epididymal and perirenal fat pads, reduce body fat percentage, reduce body weight, reduce the percentage of body weight gain, reduce fasting insulin level, reduce blood glucose level, increase insulin sensitivity, affect myostatin, activin, and/or BMP9 signaling in the subject, reduce the proliferation of adipose cells, reduce or inhibit the binding of activin and/or myostatin to their receptors, reduce LDL, reduce triglycerides, improve the serum lipid profile, regulate insulin biosynthesis and/or secretion from β-cells, delay, postpone, or reduce the need for insulin, or increase glucose clearance.
144. The method of any one of embodiments 109-143, wherein the method does not cause a vascular complication in the subject.
145. The method of embodiment 144, wherein the method does not increase vascular permeability or leakage.
146. The method of any one of embodiments 109-145, wherein the method increases bone mineral density in the subject.

## Claims

1. A polypeptide comprising an extracellular activin receptor type IIa (ActRIIa) variant, wherein the ActRIIa variant has the sequence of SEQ ID NO: 69, for use in therapy.

2. The polypeptide for use according to claim 1, wherein the use is to increase muscle mass to treat a subject having a disease or condition that results in muscle weakness or atrophy.

3. The polypeptide for use according to claim 1, wherein the use is for treating a muscle disorder in a subject.

4. The polypeptide for use according to claim 1, wherein the use is for treating and/or preventing a metabolic disease, optionally wherein the metabolic disease is obesity, Type-1 diabetes or Type-2 diabetes.

5. The polypeptide for use according to claim 2 or claim 3, wherein the subject has Duchenne muscular dystrophy (DMD), facioscapulohumeral muscular dystrophy (FSHD), inclusion body myositis (IBM), amyotrophic lateral sclerosis (ALS), sarcopenia, or cancer cachexia.

6. The polypeptide for use according to any one of the preceding claims, wherein the polypeptide further comprises a C-terminal extension of one or more amino acids.

7. The polypeptide for use according to claim 6, wherein the C-terminal extension is NP or NPVTPK (SEQ ID NO: 155).

8. The polypeptide for use according to any one of the preceding claims, wherein the polypeptide further comprises a Fc domain monomer, a wild-type Fc domain, an Fc domain with amino acid substitutions, an albumin-binding peptide, a fibronectin domain, or a human serum albumin.

9. The polypeptide for use according to claim 8, wherein the polypeptide further comprises an Fc domain monomer fused to the C-terminus of the ActRIIa variant by way of a linker.

10. The polypeptide for use according to claim 9, wherein the polypeptide is a dimer.

11. The polypeptide for use according to claim 9 or claim 10, wherein the polypeptide is a homodimer.

12. The polypeptide for use according to claim 9 or claim 10, wherein the polypeptide is a heterodimer.

13. The polypeptide for use according to claim 9, wherein the Fc domain is an IgG1 Fc domain.

14. The polypeptide for use according to claim 13, wherein the Fc domain monomer comprises the amino acid sequence of SEQ ID NO: 97.

15. The polypeptide for use according to any one of claims 9-12, wherein the linker is an amino acid spacer, optionally wherein the amino acid spacer is GA, GS, GG, GGA, GGS, GGG, GGGA (SEQ ID NO: 98), GGGS (SEQ ID NO: 99), GGGG (SEQ ID NO: 100), GGGGA (SEQ ID NO: 101), GGGGS (SEQ ID NO: 102), GGGGG (SEQ ID NO: 103), GGAG (SEQ ID NO: 104), GGSG (SEQ ID NO: 105), AGGG (SEQ ID NO: 106), SGGG (SEQ ID NO: 107), GAGA (SEQ ID NO: 108), GSGS (SEQ ID NO: 109), GAGAGA (SEQ ID NO: 110), GSGSGS (SEQ ID NO: 111), GAGAGAGA (SEQ ID NO: 112), GSGSGSGS (SEQ ID NO: 113), GAGAGAGAGA (SEQ ID NO: 114), GSGSGSGSGS (SEQ ID NO: 115), GAGAGAGAGAGA (SEQ ID NO: 116), GSGSGSGSGSGS (SEQ ID NO: 117), GGAGGA (SEQ ID NO: 118), GGSGGS (SEQ ID NO: 119), GGAGGAGGA (SEQ ID NO: 120), GGSGGSGGS (SEQ ID NO: 121), GGAGGAGGAGGA (SEQ ID NO: 122), GGSGGSGGSGGS (SEQ ID NO: 123), GGAGGGAG (SEQ ID NO: 124), GGSGGGSG (SEQ ID NO: 125), GGAGGGAGGGAG (SEQ ID NO: 126), GGSGGGSGGGSG (SEQ ID NO: 127), GGGGAGGGGAGGGGA (SEQ ID NO: 128), GGGGSGGGGSGGGGS (SEQ ID NO: 129), GGGAG (SEQ ID NO: 130), GGGAGG (SEQ ID NO: 131), GGGAGGG (SEQ ID NO: 132), AAAL (SEQ ID NO: 133), AAAK (SEQ ID NO: 134), AAAR (SEQ ID NO: 135), EGKSSGSGSESKST (SEQ ID NO: 136), GSAGSAAGSGEF (SEQ ID NO: 137), AEAAAKEAAAKA (SEQ ID NO: 138), KESGSVSSEQLAQFRSLD (SEQ ID NO: 139), GENLYFQSGG (SEQ ID NO: 140), SACYCELS (SEQ ID NO: 141), RSIAT (SEQ ID NO: 142), RPACKIPNDLKQKVMNH (SEQ ID NO: 143), GGSAGGSGSGSSGGSSGASGTGTAGGTGSGSGTGSG (SEQ ID NO: 144), AAANSSIDLISVPVDSR (SEQ ID NO: 145), GGSGGGSEGGGSEGGGSEGGGSEGGGSEGGGSGGGS (SEQ ID NO: 146), EAAAK (SEQ ID NO: 147), or PAPAP (SEQ ID NO: 148).
